(19) Europäisches Patentamt / European Patent Office / Office européen des brevets

(11) EP 2 601 836 A1

(12) **EUROPEAN PATENT APPLICATION**
published in accordance with Art. 153(4) EPC

(43) Date of publication:
**12.06.2013 Bulletin 2013/24**

(21) Application number: **11814597.8**

(22) Date of filing: **25.07.2011**

(51) Int Cl.:
***A01N 43/80*** (2006.01) ***A01N 43/40*** (2006.01)
***A01N 47/40*** (2006.01) ***A01N 51/00*** (2006.01)
***A01P 7/02*** (2006.01) ***A01P 7/04*** (2006.01)

(86) International application number:
**PCT/JP2011/067565**

(87) International publication number:
**WO 2012/017971 (09.02.2012 Gazette 2012/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.08.2010 JP 2010174935**

(71) Applicant: **Sumitomo Chemical Company Limited**
**Tokyo 104-8260 (JP)**

(72) Inventor: **IKARI, Kaori**
**Takarazuka-shi**
**Hyogo 665-8555 (JP)**

(74) Representative: **Vossius & Partner**
**Siebertstrasse 4**
**81675 München (DE)**

# (54) PEST CONTROL COMPOSITION

(57) A pest control composition comprising a hydrazide compound shown by formula (1) below (in the formula, G, M, $Q^1$, $Q^2$, $Q^3$, $Q^4$, $R^2$, $R^4$, $R^5$, $R^6$ and m have the same meanings described in the specification) and a neonicotinoid compound selected from the group consisting of imidacloprid, thiacloprid, acetamiprid, nitenpyram, clothianidin, and thiamethoxam, and a method for controlling pests whereby an effective dose of the hydrazide compound represented by formula (1) and a neonicotinoid compound selected from the group consisting of imidacloprid, thiacloprid, acetamiprid, nitenpyram, clothianidin, and thiamethoxam is applied to a pest or a pest habitat.

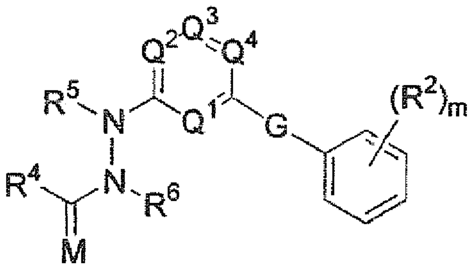
(1)

EP 2 601 836 A1

**Description**

TECHNICAL FIELD

The present invention relates to a pest control composition.

BACKGROUND ART

It is known that a composition containing a neonicotinoid compound such as imidacloprid and clothianidin has a pest control effect against pests in house (for example, see JP-A-2007-518737).

More effective control efficacy may be required according to the object to be controlled.

DISCLOSURE OF THE INVENTION

An object of the present invention is to provide a composition having an excellent control effect on pests suitable for controlling pests.

The present inventors have intensively studied to find a composition having an excellent control effect, and found that a composition comprising a hydrazide compound shown by the following formula (1):

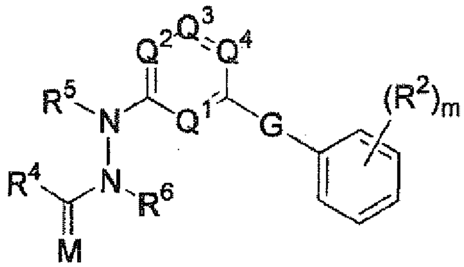

(1)

wherein G represents a group shown by any of the following formulae G-1 to G-3:

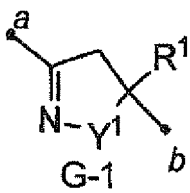

G-1

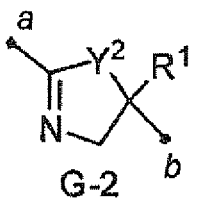

G-2

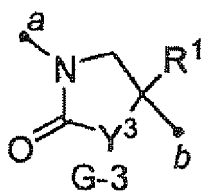

G-3

wherein each of a point (a) and a point (b) represents a bond, and the point (b) represents a bond with a phenyl ring,
$R^1$ represents a C1 to C4 haloalkyl group,
$Y^1$ represents an oxygen atom, a sulfur atom, or an $NR^7$ group,
$Y^2$ represents an oxygen atom, a sulfur atom, an $NR^7$ group, or a methylene group, and
$Y^3$ represents an oxygen atom, a sulfur atom, an $NR^7$ group, or a methylene group,
wherein $R^7$ represents a C1 to C6 alkyl group, a C2 to C6 alkenyl group, a C2 to C6 alkynyl group, a C3 to C6 cycloalkyl group, a C4 to C7 cycloalkylalkyl group, a C2 to C6 alkylcarbonyl group, a C2 to C6 alkoxycarbonyl group, a C2 to C6 alkylaminocarbonyl group, a C3 to C9 dialkylaminocarbonyl group, a phenyl group, a cyano group, a formyl group, or a hydrogen atom,
M represents an oxygen atom or a sulfur atom,
$Q^1$, $Q^2$, $Q^3$, and $Q^4$ independently represent a nitrogen atom or a $CR^3$ group,
wherein $R^3$ represents a C1 to C6 alkyl group optionally substituted by a halogen atom or atoms, a C1 to C6 alkoxy group optionally substituted by a halogen atom or atoms, a nitro group, a cyano group, a halogen atom, or a hydrogen atom,
m represents any integer of 0 to 5,
$R^2$ represents a C1 to C6 alkyl group optionally substituted by a halogen atom or atoms, a C1 to C6 alkoxy group optionally substituted by a halogen atom or atoms, a C1 to C6 alkylthio group, a C1 to C6 alkylsulfinyl group, a C1 to C6 alkylsulfonyl group, a nitro group, a cyano group, or a halogen atom, with the proviso that $R^2$s are the same or different when m is any integer of 2 to 5,

$R^5$ and $R^6$ independently represent a C1 to C12 chain hydrocarbon group optionally substituted by a group or groups selected from Group E1, a benzoyl group optionally substituted by a group or groups selected from Group E2, a C2 to C6 alkylcarbonyl group, a C2 to C6 alkoxycarbonyl group, a C3 to C12 cycloalkyl group, a formyl group, or a hydrogen atom,
$R^4$ represents a C1 to C12 chain hydrocarbon group optionally substituted by a group or groups selected from the Group E1, a C3 to C12 cyclic hydrocarbon group optionally substituted by a group or groups selected from the Group E2, a 5- to 6-membered heterocyclic group optionally substituted by a group or groups selected from the Group E2, an $OR^8$ group, an $N(R^9)R^{10}$ group, or a hydrogen atom,
wherein $R^8$ represents a C1 to C12 chain hydrocarbon group optionally substituted by a group or groups selected from the Group E1, a C3 to C12 cyclic hydrocarbon group optionally substituted by a group or groups selected from the Group E2, or a 5- to 6-membered heterocyclic group optionally substituted by a group or groups selected from the Group E2,
$R^9$ represents a C1 to C12 chain hydrocarbon group optionally substituted by a group or groups selected from the Group E1 or a C3 to C12 cyclic hydrocarbon group optionally substituted by a group or groups selected from the Group E2, and
$R^{10}$ represents a C1 to C12 chain hydrocarbon group optionally substituted by a group or groups selected from the Group E1, or a hydrogen atom, or
$R^9$ and $R^{10}$ are bonded at their ends to represent a C2 to C9 alkanediyl group,
the Group E1 represents a group consisting of a C3 to C12 cyclic hydrocarbon group optionally substituted by a group or groups selected from the Group E2, a 5- to 6-membered heterocyclic group optionally substituted by a group or groups selected from the Group E2, a phenoxy group optionally substituted by a group or groups selected from the Group E2, a phenylamino group optionally substituted by a group or groups selected from the Group E2, a C2 to C6 alkylcarbonyl group, a C2 to C6 alkoxycarbonyl group, a C1 to C6 alkoxy group, a C1 to C6 alkylamino group, a C2 to C12 dialkylamino group, a nitro group, a cyano group, a formyl group, and a halogen atom,
the Group E2 represents a group consisting of a C1 to C6 alkyl group optionally substituted by a halogen atom or atoms, a C1 to C6 alkoxy group optionally substituted by a halogen atom or atoms, a C2 to C6 alkylcarbonyl group, a C2 to C6 alkoxycarbonyl group, a C1 to C6 alkylamino group, a C2 to C12 dialkylamino group, a nitro group, a cyano group, a formyl group, and a halogen atom, and
a neonicotinoid compound selected from the group consisting of imidacloprid, thiacloprid, acetamiprid, nitenpyram, clothianidin, and thiamethoxam,
has an excellent control effect on pests, and thus the present invention has been accomplished.

Specifically, the present invention includes the following inventions:

[Invention 1]

A pest control composition comprising a hydrazide compound shown by the formula (1) and a neonicotinoid compound selected from the group consisting of imidacloprid, thiacloprid, acetamiprid, nitenpyram, clothianidin, and thiamethoxam.

[Invention 2]

The pest control composition as described in Invention 1, wherein in the formula (1), G is a group shown by G-1, $Y^1$ is an oxygen atom, $R^1$ is a trifluoromethyl group, each of $Q^1$, $Q^3$, and $Q^4$ is a CH group, $Q^2$ is a $CR^3$ group, $R^3$ is the group defined above, $(R^2)_m$ is a substituent at 3- and 5-positions, m is 2, and $R^2$ is a chlorine atom.

[Invention 3]

The pest control composition as described in Invention 1 or 2, wherein in the formula (1), $R^6$ is a hydrogen atom.

[Invention 4]

The pest control composition as described in any one of Inventions 1 to 3, wherein in the formula (1), $R^5$ is a hydrogen atom or a methyl group.

[Invention 5]

The pest control composition as described in any one of Inventions 1 to 4, wherein in the formula (1), $R^3$ is a

halogen atom.

[Invention 6]

The pest control composition as described in any one of Inventions 1 to 5, wherein in the formula (1), $R^3$ is a chlorine atom.

[Invention 7]

The pest control composition as described in any one of Inventions 1 to 6, wherein in the formula (1), $R^4$ is a C2 to C6 alkyl group, a C1 to C6 haloalkyl group, a C3 to C6 cycloalkyl group, or a (C1 to C6 alkoxy) C1 to C6 alkyl group.

[Invention 8]

The pest control composition as described in any one of Inventions 1 to 7, wherein in the formula (1), $R^4$ is a C2 to C6 alkyl group.

[Invention 9]

The pest control composition as described in any one of Inventions 1 to 7, wherein in the formula (1), $R^4$ is a C1 to C6 haloalkyl group.

[Invention 10]

The pest control composition as described in any one of Inventions 1] to 7, wherein in the formula (1), $R^4$ is a C3 to C6 cycloalkyl group.

[Invention 11]

The pest control composition as described in any one of Inventions 1 to 7, wherein in the formula (1), $R^4$ is a (C1 to C6 alkoxy) C1 to C6 alkyl group.

[Invention 12]

The pest control composition as described in any one of Inventions 1 to 11, wherein the neonicotinoid compound is a compound selected from the group consisting of clothianidin, nitenpyram, and thiamethoxam.

[Invention 13]

The pest control composition as described in any one of Inventions 1] to 11, wherein the neonicotinoid compound is clothianidin.

[Invention 14]

The pest control composition as described in Invention 1, wherein in the formula (1), G is a group shown by G-1, $Y^1$ is an oxygen atom, $R^1$ is a trifluoromethyl group, each of $Q^1$, $Q^3$, and $Q^4$ is a CH group, $Q^2$ is a $CR^3$ group, $R^3$ is the group defined above, $(R^2)_m$ is a substituent at position 3 and position 5, m is 2, and $R^2$ is a chlorine atom, and the neonicotinoid compound is a compound selected from the group consisting of clothianidin, nitenpyram, and thiamethoxam.

[Invention 15]

The pest control composition as described in Invention 1, wherein in the formula (1), $R^6$ is a hydrogen atom, and the neonicotinoid compound is a compound selected from the group consisting of clothianidin, Nitenpyram, and thiamethoxam.

[Invention 16]

The pest control composition as described in Invention 1, wherein in the formula (1), $R^5$ is a hydrogen atom or a methyl group, and
the neonicotinoid compound is a compound selected from the group consisting of clothianidin, nitenpyram, and thiamethoxam.

[Invention 17]

The pest control composition as described in Invention 1, wherein in the formula (1), $R^3$ is a halogen atom, and the neonicotinoid compound is a compound selected from the group consisting of clothianidin, nitenpyram, and thiamethoxam.

[Invention 18]

The pest control composition as described in Invention 1, wherein in the formula (1), $R^3$ is a chlorine atom, and the neonicotinoid compound is a compound selected from the group consisting of clothianidin, nitenpyram, and thiamethoxam.

[Invention 19]

The pest control composition as described in Invention 1, wherein in the formula (1), $R^4$ is a C2 to C6 alkyl group, a C1 to C6 haloalkyl group, a C3 to C6 cycloalkyl group, or a (C1 to C6 alkoxy) C1 to C6 alkyl group, and
the neonicotinoid compound is a compound selected from the group consisting of clothianidin, nitenpyram, and thiamethoxam.

[Invention 20]

The pest control composition as described in Invention 1, wherein in the formula (1), $R^4$ is a C2 to C6 alkyl group, and the neonicotinoid compound is a compound selected from the group consisting of clothianidin, nitenpyram, and thiamethoxam.

[Invention 21]

The pest control composition as described in Invention 1, wherein in the formula (1), $R^4$ is a C1 to C6 haloalkyl group, and
the neonicotinoid compound is a compound selected from the group consisting of clothianidin, nitenpyram, and thiamethoxam.

[Invention 22]

The pest control composition as described in Invention 1, wherein in the formula (1), $R^4$ is a C3 to C6 cycloalkyl group, and
the neonicotinoid compound is a compound selected from the group consisting of clothianidin, nitenpyram, and thiamethoxam.

[Invention 23]

The pest control composition as described in Invention 1, wherein in the formula (1), $R^4$ is a (C1 to C6 alkoxy) C1 to C6 alkyl group, and
the neonicotinoid compound is a compound selected from the group consisting of clothianidin, nitenpyram, and thiamethoxam.

[Invention 24]

The pest control composition as described in Invention 1, wherein in the formula (1), G is a group shown by G-1, $Y^1$ is an oxygen atom, $R^1$ is a trifluoromethyl group, each of $Q^1$, $Q^3$, and $Q^4$ is a CH group, $Q^2$ is a $CR^3$ group, $R^3$ is the group defined above, $(R^2)_m$ is a substituent at 3- and 5-position, m is 2, and $R^2$ is a chlorine atom, and

the neonicotinoid compound is clothianidin.

[Invention 25]

The pest control composition as described in Invention 1, wherein in the formula (1), $R^6$ is a hydrogen atom, and the neonicotinoid compound is clothianidin.

[Invention 26]

The pest control composition as described in Invention 1, wherein in the formula (1), $R^5$ is a hydrogen atom or a methyl group, and
the neonicotinoid compound is clothianidin.

[Invention 27]

The pest control composition as described in Invention 1, wherein in the formula (1), $R^3$ is a halogen atom, and the neonicotinoid compound is a clothianidin.

[Invention 28]

The pest control composition as described in Invention 1, wherein in the formula (1), $R^3$ is a chlorine atom, and the neonicotinoid compound is a clothianidin.

[Invention 29]

The pest control composition as described in Invention 1, wherein in the formula (1), $R^4$ is a C2 to C6 alkyl group, a C1 to C6 haloalkyl group, a C3 to C6 cycloalkyl group, or a (C1 to C6 alkoxy) C1 to C6 alkyl group, and
the neonicotinoid compound is a clothianidin.

[Invention 30]

The pest control composition as described in Invention 1, wherein in the formula (1), $R^4$ is a C2 to C6 alkyl group, and the neonicotinoid compound is a clothianidin.

[Invention 31]

The pest control composition as described in Invention 1, wherein in the formula (2), $R^4$ is a C1 to C6 haloalkyl group, and
the neonicotinoid compound is a clothianidin.

[Invention 32]

The pest control composition as described in Invention 1, wherein in the formula (1), $R^4$ is a C3 to C6 cycloalkyl group, and
the neonicotinoid compound is a clothianidin.

[Invention 33]

The pest control composition as described in Invention 1, wherein in the formula (1), $R^4$ is a (C1 to C6 alkoxy) C1 to C6 alkyl group, and
the neonicotinoid compound is a clothianidin.

[Invention 34]

A method for controlling pests comprising applying effective amounts of the hydrazide compound shown by the formula (1) of Invention 1 and a neonicotinoid compound selected from the group consisting of imidacloprid, thiacloprid, acetamiprid, nitenpyram, clothianidin, and thiamethoxam to pests or habitats of pests.

[Invention 35]

A use of the combination of the hydrazide compound shown by the formula (1) of Invention 1 and a neonicotinoid compound selected from the group consisting of imidacloprid, thiacloprid, acetamiprid, nitenpyram, clothianidin, and thiamethoxam for controlling pests.

The pest control composition of the present invention has an excellent effect on pest control.

MODE FOR CARRYING OUT THE INVENTION

The weight ratio of the hydrazide compound shown by the formula (1) (hereinafter referred to as the present hydrazide compound.) and a neonicotinoid compound selected from the group consisting of imidacloprid, thiacloprid, acetamiprid, nitenpyram, clothianidin, and thiamethoxam (hereinafter referred to as the present neonicotinoid compound.) in the pest control composition of the present invention is within a range of preferably from 1000:1 to 1:1000, and more preferably from 1:1 to 1:500.

The term phenyl ring in "G represents a group shown by any of the following formulae G-1 to G-3:

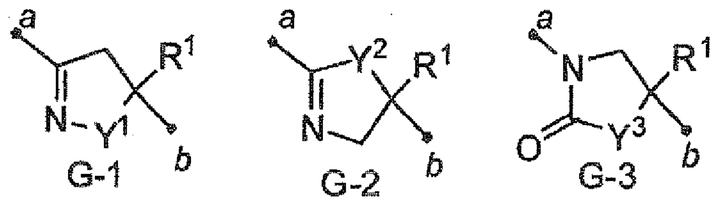

wherein each of a point (a) and a point (b) represents a bond, and the point (b) represents a bond with a phenyl ring" means a phenyl ring shown by the following formula:

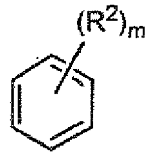

wherein $R^2$ and m represent the same meanings as above.

For example, "a hydrazide compound, wherein in the formula (1), G is a group shown by G-1" means a compound shown by following formula (a):

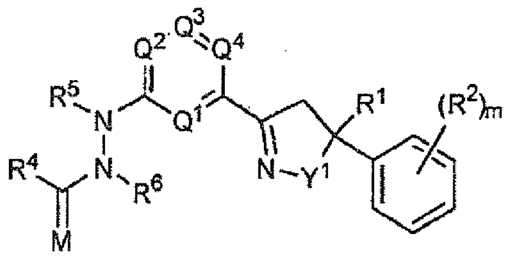

(a)

wherein $Q^1$, $Q^2$, $Q^3$, $Q^4$, $R^1$, $R^2$, $R^4$, $R^5$, $R^6$, M, $Y^1$, and m represent the same meanings as above.

In the present invention, a "halogen atom" means a fluorine atom, a chlorine atom, a bromine atom, or an iodine atom.

As used herein, for example, the description "C2 to C6" of a "C2 to C6 alkylaminocarbonyl group" means that the total number of carbon atoms constituting the alkylaminocarbonyl group is within a range of 2 to 6.

Examples of substituents of the present hydrazide compound are shown below.

The following groups can be given as examples of a group shown by any of G-1 to G-3.

G-1: 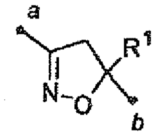 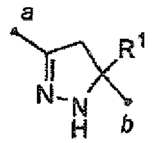 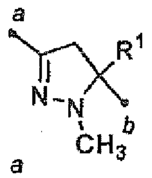

G-2: 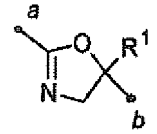 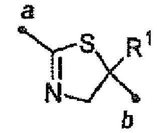 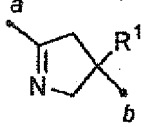

G-3: 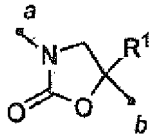 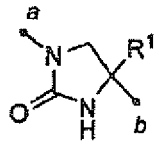 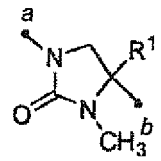 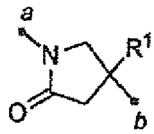

Herein,
examples of the "C1 to C4 haloalkyl group" include a fluoromethyl group, a difluoromethyl group, a dichloromethyl group, a trifluoromethyl group, a chlorofluoromethyl group, a bromofluoromethyl group, a chlorodifluoromethyl group, a bromodifluoromethyl group, a 1-fluoromethyl group, a 1,1-difluoroethyl group, a 1,1,2,2,2-pentafluoroethyl group, a 1,1,2,2,3,3,3-heptafluoropropyl group, and a 1,2,2,2-tetrafluoro-1-(trifluoromethyl)ethyl group.

Examples of the "(C1 to C6 alkyl group" include a methyl group, an ethyl group, a propyl group, an isopropyl group, a butyl group, an isobutyl group, a sec-butyl group, a tert-butyl group, a pentyl group, a 1-methylbutyl group, a 2-methylbutyl group, a 3-methylbutyl group, a 1-ethylpropyl group, a 1,1-dimethylpropyl group, a 1,2-dimethylpropyl group, a 2,2-dimethylpropyl group, a hexyl group, a 1-methylpentyl group, a 2-methylpentyl group, a 1,1-dimethylbutyl group, and a 1,3-dimethylbutyl group.

Examples of the "C2 to C6 alkenyl group" include a vinyl group, a 1-propenyl group, a 2-propenyl group, a 1-methyl-2-propenyl group, a 2-methyl-2-propenyl group, a 1-butenyl group, 2-butenyl group, a 3-butenyl group, a 1-pentenyl group, a 2-pentenyl group, a 3-pentenyl group, a 4-pentenyl group, a 1-hexenyl group, a 2-hexenyl group, a 3-hexenyl group, a 4-hexenyl group, and a 5-hexenyl group.

Examples of the "C2 to C6 alkynyl group" include an ethynyl group, a 1-propynyl group, a 2-propynyl group, a 1-butynyl group, a 2-butynyl group, a 3-butynyl group, a 1-pentynyl group, a 2-pentynyl group, a 3-pentynyl group, a 4-pentynyl group, a 1-hexynyl group, a 2-hexynyl group, a 3-hexynyl group, a 4-hexynyl group, and a 5-hexynyl group.

Examples of the "C3 to C6 cycloalkyl group" include a cyclopropyl group, a 1-methylcyclopropyl group, a 2-methylcyclopropyl group, a 2,2-dimethylcyclopropyl group, a cyclobutyl group, a cyclopentyl group, a 1-methylcyclopentyl group, a 2-methylcyclopentyl group, a 3-methylcyclopentyl group, and a cyclohexyl group.

Examples of the "C4 to C7 cycloalkylalkyl group" include a cyclopropylmethyl group, a 1-cyclopropylethyl group, a 2-cyclopropylethyl group, a cyclobutylmethyl group, a cyclopentylmethyl group, and a cyclohexylmethyl group.

Examples of the "C2 to C6 alkylcarbonyl group" include an acetyl group, a propionyl group, a butyloyl group, an isobutyloyl group, and a pivaloyl group.

Examples of the "C2 to C6 alkoxycarbonyl group" include a methoxycarbonyl group, an ethoxycarbonyl group, an isopropyloxycarbonyl group, a butoxycarbonyl group, a tert-butoxycarbonyl group, and a pentyloxycarbonyl group.

Examples of the "C2 to C6 alkylaminocarbonyl group" include an N-methylaminocarbonyl group, an N-ethylaminocarbonyl group, an N-propylaminocarbonyl group, an N-butylaminocarbonyl group, and an N-pentylaminocarbonyl group.

Examples of the "C3 to C9 dialkylaminocarbonyl group" include an N,N-dimethylaminocarbonyl group and an N,N-diethylaminocarbonyl group.

Examples of the "C1 to C6 alkoxy group" include a methoxy group, an ethoxy group, a propoxy group, an isopropoxy group, a butoxy group, an isobutoxy group, a sec-butoxy group, a tert-butoxy group, a pentyloxy group, and a hexyloxy group.

Examples of the "C1 to C6 alkyl group optionally substituted by a halogen atom or atoms" include C1 to C6 alkyl groups such as a methyl group, an ethyl group, a propyl group, an isopropyl group, a butyl group, an isobutyl group, a sec-butyl group, a tert-butyl group, a pentyl group, a 1-methylbutyl group, a 2-methylbutyl group, a 3-methylbutyl group, a 1-ethylpropyl group, a 1,1-dimethylpropyl group, a 1,2-dimethylpropyl group, a 2, 2-dimethylpropyl group, a hexyl group, a 1-methylpentyl group, a 2-methylpentyl group, a 1,1-dimethylbutyl group, and a 1,3-dimethylbutyl group; and C1 to C6 haloalkyl groups such as a fluoromethyl group, a difluoromethyl group, a dichloromethyl group, a trifluoromethyl

group, a chlorofluoromethyl group, a bromofluoromethyl group, a chlorodifluoromethyl group, a bromodifluoromethyl group, a 1-fluoroethyl group, a 1,1-difluoroethyl group, a 1,1,2,2,2-pentafluoroethyl group, a 1,1,2,2,3,3,3-heptafluoropropyl group, and a 1,2,2,2-tetrafluoro-1-(trifluoromethyl)ethyl group.

Examples of the "C1 to C6 alkoxy group optionally substituted by a halogen atom or atoms" include C1 to C6 alkoxy groups such as a methoxy group, an ethoxy group, a propoxy group, an isopropoxy group, a butoxy group, an isobutoxy group, a sec-butoxy group, a tert-butoxy group, a pentyloxy group, and a hexyloxy group; and C1 to C6 haloalkoxy groups such as a difluoromethoxy group, a trifluoromethoxy group, a chlorodifluoromethoxy group, a bromodifluoromethoxy group, a 2-fluoroethoxy group, a 2-chloroethoxy group, a 2,2,2-trifluoroethoxy group, a 1,1,2,2-tetrafluoroethoxy group, a 2-chloro-1,1,2-trifluoroethoxy group, a 2-bromo-1,1,2-trifluoroethoxy group, a 1,1,2,2,2-pentafluoroethoxy group, a 2,2-dichloro-1,1,2-trifluoroethoxy group, a 2,2,2-trichloro-1,1-difluoroethoxy group, a 2-bromo-1,1,2,2-tetrafluoroethoxy group, a 2,2,3,3-tetrafluoropropyloxy group, a 1,1,2,3,3,3-hexafluoropropyloxy group, a 2,2,2-trifluoro-1-(trifluoromethyl)ethoxy group, a 1,1,2,2,3,3,3-heptafluoropropyloxy group, and a 2-bromo-1,1,2,3,3,3-hexafluoropropyloxy group.

Examples of the "C1 to C6 alkylthio group" include a methylthio group, an ethylthio group, a propylthio group, an isopropylthio group, a butylthio group, a pentylthio group, and a hexylthio group.

Examples of the "C1 to C6 alkylsulfinyl group" include a methanesulfinyl group, an ethylsulfinyl group, a propylsulfinyl group, an isopropylsulfinyl group, a butylsulfinyl group, a pentylsulfinyl group, and a hexylsulfinyl group.

Examples of the "C1 to C6 alkylsulfonyl group" include a methanesulfonyl group, an ethylsulfonyl group, a propylsulfonyl group, an isopropylsulfonyl group, a butylsulfonyl group, a pentylsulfonyl group, and a hexylsulfonyl group.

Examples of the "C3 to C12 cycloalkyl group" include a cyclopropyl group, a 1-methylcyclopropyl group, a 2-methylcyclopropyl group, a 2,2-dimethylcyclopropyl group, a 2,2,3,3-tetramethylcyclopropyl group, a cyclobutyl group, a cyclopentyl group, a 1-methylcyclopentyl group, a 2-methylcyclopentyl group, a 3-methylcyclopentyl group, a cyclohexyl group, a 1-methylcyclohexyl group, a 2-methylcyclohexyl group, a 3-methylcyclohexyl group, a 4-methylcyclohexyl group, a cycloheptyl group, and a cyclooctyl group.

Examples of the "C2 to C9 alkanediyl group" include a 1,2-ethylene group, a 1,4-tetramethylene group, and a 1,5-pentamethylene group.

Examples of the C1 to C12 chain hydrocarbon group in the "C1 to C12 chain hydrocarbon group optionally substituted by a group or groups selected from Group E1" include a C1 to C12 alkyl group, a C2 to C12 alkenyl group, and a C2 to C12 alkynyl group.

Examples of the "C1 to C12 chain hydrocarbon group optionally substituted by a group or groups selected from Group E1" include C1 to C12 alkyl groups such as a methyl group, an ethyl group, a propyl group, an isopropyl group, a butyl group, an isobutyl group, a sec-butyl group, a tert-butyl group, a pentyl group, a 1-methylbutyl group, a 2-methylbutyl group, a 3-methylbutyl group, a 1-ethylpropyl group, a 1,1-dimethylpropyl group, a 1,2-dimethylpropyl group, a 2,2-dimethylpropyl group, a hexyl group, a 1-methylpentyl group, a 2-methylpentyl group, a 1,1-dimethylbutyl group, a 1,3-dimethylbutyl group, a heptyl group, an octyl group, a nonyl group, a decyl group, an undecyl group, and a dodecyl group;

C2 to C12 alkenyl groups such as a vinyl group, a 1-methylvinyl group, a 1-phenylvinyl group, a 1-propenyl group, a 2-propenyl group, a 2-butenyl group, a 1-methyl-2-propenyl group, a 2-methyl-2-propenyl group, a 2-pentenyl group, a 2-methyl-2-butenyl group, a 3-methyl-2-butenyl group, a 2-ethyl-2-propenyl group, a 1,1-dimethyl-2-propenyl group, a 2-hexenyl group, a 2-methyl-2-pentenyl group, a 2,4-dimethyl-2,6-heptadienyl group, and a 3,7-dimethyl-2,6-octadienyl group;

C2 to C12 alkynyl groups such as a 2-propynyl group, a 2-butynyl group, a 1-methyl-2-propynyl group, a 2-pentynyl group, a 1-methyl-2-butynyl group, a 1,1-dimethyl-2-propynyl group, and a 2-hexynyl group;

C1 to C12 haloalkyl groups such as a fluoromethyl group, a chloromethyl group, a bromomethyl group, an iodomethyl group, a difluoromethyl group, a chlorofluoromethyl group, a dichloromethyl group, a bromofluoromethyl group, a trifluoromethyl group, a chlorodifluoromethyl group, a dichlorofluoromethyl group, a trichloromethyl group, a bromodifluoromethyl group, a bromochlorofluoromethyl group, a difluoroiodomethyl group, a 2-fluoroethyl group, a 2-chloroethyl group, a 2-bromoethyl group, a 2,2-difluoroethyl group, a 2-chloro-2-fluoroethyl group, a 2,2-dichloroethyl group, a 2-bromo-2-fluoroethyl group, a 2,2,2-trifluoroethyl group, a 2-chloro-2,2-difluoroethyl group, a 2,2-dichloro-2-fluoroethyl group, a 2,2,2-trichloroethyl group, a 2-bromo-2,2-difluoroethyl group, a 1,1,2,2-tetrafluoroethyl group, a 1,1,2,2,2-pentafluoroethyl group, a 1-chloro-1,2,2,2-tetrafluoroethyl group, a 2-chloro-1,1,2,2-tetrafluoroethyl group, a 1,2-dichloro-1,2,2-trifluoroethyl group, a 1-bromo-1,2,2,2-tetrafluoroethyl group, a 2-bromo-1,1,2,2-tetrafluoroethyl group, a 2-fluoropropyl group, a 2-chloropropyl group, a 2,3-dichloropropyl group, a 3,3,3-trifluoropropyl group, a 3-bromo-3,3-difluoropropyl group, a 2,2,3,3-tetrafluoropropyl group, a 2,2,3,3,3-pentafluoropropyl group, a 1,1,2,3,3,3-hexafluoropropyl group, a 1,1,2,2,3,3,3-heptafluoropropyl group, a 2,3-dichloro-1,1,2,3,3-pentafluoropropyl group, a 2-fluoro-1-methylethyl group, a 2-chloro-1-methylethyl group, a 2-bromo-1-methylethyl group, a 2,2,2-trifluoro-1-(trifluoromethyl)ethyl group, a 1,2,2,2-tetrafluoro-1-(trifluoromethyl)ethyl group, and a 1,1,2,2,3,3,4,4,4-nonafluorobutyl group;

C2 to C12 haloalkenyl groups such as a 2,2-fluorovinyl group, a 2,2-dichlorovinyl group, a 2-fluoro-2-propenyl

group, a 2-chloro-2-propenyl group, a 3-chloro-2-propenyl group, a 2-bromo-2-propenyl group, a 3-bromo-2-propenyl group, a 3,3-difluoro-2-propenyl group, a 2,3-diohloro-2-propenyl group, a 3,3-dichloro-2-propenyl group, a 2, 3-dibromo-2-propenyl group, a 2,3,3-trifluoro-2-propenyl group, a 2,3,3-trichloro-2-propenyl group, a 3-chloro-2-butenyl group, a 3-bromo-2-butenyl group, a 4,4-difluoro-3-butenyl group, a 3,4,4-trifluoro-3-butenyl group, a 3-chloro-3,4,4-trifluoro-2-butenyl group, and a 3-bromo-2-methyl-2-propenyl group;

C2 to C12 haloalkynyl groups such as a 3-chloro-2-propynyl group, a 3-bromo-2-propynyl group, and a 3-iodo-2-propynyl group;

cyano (C1 to C1 alkyl) groups such as a cyanomethyl group;

C1 to C12 nitroalkyl groups such as a nitromethyl group;

(C2 to C12 dialkylamino) C1 to C12 alkyl groups such as an N,N-dimethylaminomethyl group;

(C1 to C6 alkylamino) C1 to C12 alkyl groups such as an N-ethylaminomethyl group;

(C1 to C6 alkoxy) C1 to C12 alkyl groups such as a methoxymethyl group, a 2-methoxyethyl group, a 2-ethoxyethyl group, a 2-propoxyethyl group, a 2-isopropoxyethyl group, a 2-butoxyethyl group, a 2-isobutoxyethyl group, a 2-(1-methylpropyloxy)ethyl group, and a 2-(1,1-dimethylethoxy)ethyl group;

(C2 to C6 alkoxycarbonyl) C1 to C12 alkyl groups such as a methoxycarbonylmethyl group, an ethoxycarbonylmethyl group, a propoxycarbonylmethyl group, an isopropoxycarbonylmethyl group, and a tert-butoxycarbonylmethyl group;

(C2 to C6 alkylcarbonyl) C1 to C12 alkyl groups such as an acetylmethyl group, and a 2-acetylethyl group;

phenoxy group-substituted C1 to C12 alkyl groups optionally substituted by a group or groups selected from Group E2 such as a phenoxymethyl group, a 4-chlorophenoxymethyl group, and a phenoxyethyl group;

phenylamino group-substituted C1 to C12 alkyl groups optionally substituted by a group or groups selected from Group E2 such as a phenylaminomethyl group, a 4-chlorophenylaminomethyl group, and a phenylaminoethyl group;

5- to 6-membered heterocyclic group-substituted C1 to C12 alkyl groups optionally substituted by a group or groups selected from Group E2 such as groups shown by the following formulae:

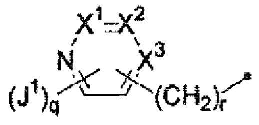

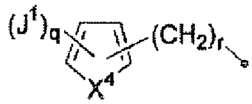

wherein $X^1$, $X^2$, and $X^3$ represent a combination in which all three show a CH group or a combination in which any one is a nitrogen atom and the other two show a CH group, $X^4$ represents an oxygen atom, a sulfur atom, or an NH group, r represents an integer of 1 to 12, q represents an integer of 0 to 3, $J^1$ represents a C1 to C6 alkyl group optionally substituted by a halogen atom or atoms, a C1 to C6 alkoxy group optionally substituted by a halogen atom or atoms, a C2 to C6 alkylcarbonyl group, a C2 to C6 alkoxycarbonyl group, a C1 to C6 alkylamino group, a C2 to C12 dialkylamino group, a nitro group, a cyano group, a formyl group, or a halogen atom; and

C3 to C12 cyclic hydrocarbon group-substituted C1 to C12 alkyl groups optionally substituted by a group or groups selected from Group E2 such as a benzyl group, a phenethyl group, a 4-methylbenzyl group, a 4-chlorobenzyl group, a 2-(cyclopropyl)ethyl group, and a cyclopropylmethyl.

Examples of the "benzoyl group optionally substituted by a group or groups selected from Group E2" include a benzoyl group, a 2-fluorobenzoyl group, a 3-fluorobenzoyl group, a 4-fluorobenzoyl group, a 2-chlorobenzoyl group, a 3-chlorobenzoyl group, a 4-chlorobenzoyl group, a 2-methoxybenzoyl group, a 3-methoxybenzoyl group, a 4-methoxybenzoyl group, a 2-cyanobenzoyl group, a 3-cyanobenzoyl group, a 4-cyanobenzoyl group, a 2-nitrobenzoyl group, a 3-nitrobenzoyl group, and a 4-nitrobenzoyl group.

Examples of the C3 to C12 cyclic hydrocarbon group in the present hydrazide compound include a phenyl group, a naphthyl group, and a C3 to C12 cycloalkyl group,

Examples of the "C3 to C12 cyclic hydrocarbon group optionally substituted by a group or groups selected from Group E2" include

phenyl groups such as a phenyl group, a 2-methylphenyl group, a 3-methylphenyl group, a 4-methylphenyl group, a 2-(trifluoromethyl)phenyl group, a 3-(trifluoromethyl)phenyl group, a 4- (trifluoromethyl) phenyl group, a 2-fluorophenyl group, a 3-fluorophenyl group, a 4-fluorophenyl group, a 2-chlorophenyl group, a 3-chlorophenyl group, a 4-chlorophenyl group, a 2-bromophenyl group, a 3-bromophenyl group, a 4-bromophenyl group, a 2-iodophenyl group, a 3-iodophenyl group, a 4-iodophenyl group, a 2-cyanophenyl group, a 3-cyanophenyl group, a 4-cyanophenyl group, a 2-nitrophenyl group, a 3-nitrophenyl group, a 4-nitrophenyl group, a 2-methoxyphenyl group, a 3-methoxyphenyl group, a 4-methoxyphenyl group, a 2- (trifluoromethoxy) phenyl group, a 3-(trifluoromethoxy) phenyl group, a 4- (trifluoromethoxy) phenyl group, a 2, 3-dichlorophenyl group, a 2,4-dichlorophenyl group, a 2,5-dichlorophenyl group, a 2,6-dichlorophenyl group, a 3,4-dichlorophenyl group, a 3,5-dichlorophenyl group, a 2,3,4-trichlorophenyl group, a 2,3,5-trichlorophenyl group, a 3,4,5-trichlorophenyl group, a 2,4,6-trichlorophenyl group, a 2,3-difluorophenyl group, a 2,4-difluorophenyl group, a 2,5-difluorophenyl group, a 2,6-difluorophenyl group, a 3,4-difluorophenyl group, a 3,5-difluorophenyl group, a 3,4,5-trifluorophenyl group, a 2,3,5,6-tetrafluorophenyl group, a 2,3,4,5,6-pentafluorophenyl group, a 3,5-bis(trifluoromethyl)phenyl group, a 2,3-dimethoxyphenyl group, a 2,4-dimethoxyphenyl group, a 2,5-dimethoxyphenyl group, a 2,6-dimethoxyphenyl group, a 3,4-dimethoxyphenyl group, a 3,5-dimethoxyphenyl group, a 4-methoxycarbonylphenyl group, and a 4-ethoxycarbonylphenyl group;
C3 to C12 cycloalkyl groups such as a cyclopropyl group, a 1-methylcyclopropyl group, a 2-methylcyclopropyl group, a 2,2-dimethylcyclopropyl group, a 2,2,3,3-tetramethylcyclopropyl group, a cyclobutyl group, a cyclopentyl group, a 1-methylcyclopentyl group, a 2-methylcyclopentyl group, a 3-methylcyclopentyl group, a cyclohexyl group, a 1-methylcyclohexyl group, a 2-methylcyclohexyl group, a 3-methylcyclohexyl group, a 4-methylcyclohexyl group, a cycloheptyl group, and a cyclooctyl group.

Examples of the 5- to 6-membered heterocyclic group in the "5- to 6-membered heterocyclic group optionally substituted by a group or groups selected from Group E2" include
5-membered heterocyclic groups such as a tetrahydrofuranyl group, a furanyl group, a thienyl group, a pyrrolyl group, an imidazolyl group, and an isothiazolyl group, in which the hetero atom is an oxygen atom, a sulfur atom and/or a nitrogen atom;
6-membered heterocyclic groups such as a piperidino group, a morpholino group, a pyridyl group, a pyridazinyl group, a pyrimidyl group, and a pyrazinyl group, in which the hetero atom is an oxygen atom, a sulfur atom and/or a nitrogen atom.

Examples of the "5- to 6-membered heterocyclic group optionally substituted by a group or groups selected from Group E2" include a 1-pyrrolyl group, a 2-pyrrolyl group, a 3-pyrrolyl group, a 2-furanyl group, a 3-furanyl group, a 2-thienyl group, a 3-thienyl group, a 1-pyrazolyl group, a 3-pyrazolyl group, a 4-pyrazolyl group, a 3-isoxazolyl group, a 4-isoxazolyl group, a 5-isoxazolyl group, a 3-isothiazolyl group, a 4-isothiazolyl group, a 5-isothiazolyl group, a 1-imidazolyl group, a 2-imidazolyl group, a 4-imidazolyl group, a 2-oxazolyl group, a 4-oxazolyl group, a 5-oxazolyl group, a 2-thiazolyl group, a 4-thiazolyl group, a 5-thiazolyl group, a 1,2,4-triazol-1-yl group, a 1, 2, 4-triazol-3-yl group, a 1, 3, 4-oxadiazol-2-yl group, a 1,3,4-thiadiazol-2-yl group, a 1,2,4-oxadiazol-3-yl group, a 1,2,4-oxadiazol-5-yl group, a 1,2,4-thiadiazol-3-yl group, a 1,2,4-thiadiazole-5-yl group, a 1-pyrrolidinyl group, a 2-pyrrolidinyl group, a 3-pyrrolidinyl group, a 2-tetrahydrofuranyl group, a 3-tetrahydrofuranyl group, a 1-piperidino group, a 1-morpholino group, a 2-pyridyl group, a 3-pyridyl group, a 4-pyridyl group, a 3-pyridazinyl group, a 4-pyridazinyl group, a 2-pyrimidinyl group, a 4-pyrimidinyl group, a 5-pyrimidinyl group, a 2-pyrazinyl group, a 1,2,4-triazin-3-yl group, a 1,2,4-triazin-5-yl group, a 1,2,4-triazin-6-yl group, and a 1,3,5-triazin-2-yl group.

Examples of the "C1 to C6 alkylamino group" include an N-methylamino group, an N-ethylamino group, an N-propylamino group, an N-butylamino group, and an N-pentylamino group.

Examples of the "C2 to C12 dialkylamino group" include an N,N-dimethylamino group and an N,N-diethylamino group.

Examples of the above $OR^8$ group include a methoxy group, an ethoxy group, a propoxy group, an isopropoxy group, a butoxy group, an isobutoxy group, a sec-butoxy group, a tert-butoxy group, a vinyloxy group, an allyloxy group, a 2,2,2-trifluoroethoxy group, a cyanomethoxy group, a 2-(N,N-dimethylamino)ethoxy group, a 2-(N-methylamino)ethoxy group, a 2-methoxyethoxy group, a methoxycarbonylmethoxy group, an acetylmethoxy group, a 2-phenoxyethoxy group, a 2-(phenylamino)ethoxy group, a 2-pyridyloxy group, a benzyloxy group, a phenoxy group, a cyclohexyloxy group, a furan-2-oxy group, a pyridin-2-oxy group, and the like.

Examples of the above $N(R^9)R^{10}$ group include an N-methylamino group, an N,N-dimethylamino group, an N-ethylamino group, an N,N-diethylamino group, an N-methyl-N-ethylamino group, an N-allylamino group, an N-(2,2,2-trifluoroethyl)amino group, an N-(2-(N,N-dimethylamino)ethyl)amino group, an N-(2-(N-methylamino)ethyl)amino group, an N-(2-methoxyethyl)amino group, an N-(methoxycarbonylmethyl)amino group, an N-(2-phenoxyethyl)amino group, an N-(2-(phenylamino)ethyl)amino group, an N-benzylamino group, N-phenylamino group, an N-phenyl-N-methylamino group, an N-cyclohexylamino group, an N-cyclohexyl-N-methylamino group, a pyrrolidin-1-yl group, a piperizin-1-yl group, and the like.

The following hydrazide compounds can be given as examples of embodiments of the present hydrazide compound.

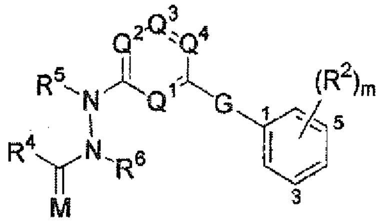

(1)

Hydrazide compounds, wherein in the formula (1), G is a group shown by G-1;
Hydrazide compounds, wherein in the formula (1), G is a group shown by G-1, and $Y^1$ is an oxygen atom;
Hydrazide compounds, wherein in the formula (1), $R^1$ is a trifluoromethyl group;
Hydrazide compounds, wherein in the formula (1), $Q^1$, $Q^2$, $Q^3$, and $Q^4$ are CH groups;
Hydrazide compounds, wherein in the formula (1), $Q^1$, $Q^3$, and $Q^4$ are CH groups, $Q^2$ is a $CR^3$ group, and $R^3$ is as defined above;
Hydrazide compounds, wherein in the formula (1), $Q^1$, $Q^3$, and $Q^4$ are CH groups, $Q^2$ is a $CR^3$ group, and $R^3$ is a C1 to C6 alkyl group;
Hydrazide compounds, wherein in the formula (1), $Q^1$, $Q^3$, and $Q^4$ are CH groups, $Q^2$ is a $CR^3$ group, and $R^3$ is a methyl group;
Hydrazide compounds, wherein in the formula (1), $Q^1$, $Q^3$, and $Q^9$ are CH groups, $Q^2$ is a $CR^3$ group, and $R^3$ is an ethyl group;
Hydrazide compounds, wherein in the formula (1), $Q^1$, $Q^3$, and $Q^4$ are CH groups, $Q^2$ is a $CR^3$ group, and $R^3$ is a halogen atom;
Hydrazide compounds, wherein in the formula (1), $Q^1$, $Q^3$, and $Q^4$ are CH groups, $Q^2$ is a $CR^3$ group, and $R^3$ is a fluorine atom;
Hydrazide compounds, wherein in the formula (1), $Q^1$, $Q^3$, and $Q^4$ are CH groups, $Q^2$ is a $CR^3$ group, and $R^3$ is a chlorine atom;
Hydrazide compounds, wherein in the formula (1), $Q^1$, $Q^3$, and $Q^4$ are CH groups, $Q^2$ is a $CR^3$ group, and $R^3$ is a bromine atom;
Hydrazide compounds, wherein in the formula (1), m is 2 and $R^2$ is respectively a halogen atom;
Hydrazide compounds, wherein in the formula (1), m is 2 and $R^2$ is respectively a chlorine atom;
Hydrazide compounds, wherein in the formula (1), $(R^2)_m$ is a substituent at 3- and 5-positions, m is 2, and $R^2$ is respectively a chlorine atom;
Hydrazide compounds, wherein in the formula (1), $R^4$ is a C1 to C12 chain hydrocarbon group optionally substituted by a group or groups selected from Group E1;
Hydrazide compounds, wherein in the formula (1), $R^4$ is a C3 to C12 cyclic hydrocarbon group optionally substituted by a group or groups selected from Group E2;
Hydrazide compounds, wherein in the formula (1), $R^4$ is a 5- to 6-membered heterocyclic group optionally substituted by a group or groups selected from Group E2;
Hydrazide compounds, wherein in the formula (1), $R^4$ is an $OR^8$ group, and $R^8$ is as defined above;
Hydrazide compounds, wherein in the formula (1), $R^4$ is an $N(R^9)R^{10}$ group, and $R^9$ and $R^{10}$ are as defined above;
Hydrazide compounds, wherein in the formula (1), $R^5$ is a hydrogen atom;
Hydrazide compounds, wherein in the formula (1), $R^5$ is a C1 to C12 chain hydrocarbon group optionally substituted by a group or groups selected from Group E1;
Hydrazide compounds, wherein in the formula (1), $R^5$ is a methyl group;
Hydrazide compounds, wherein in the formula (1), $R^5$ is a C2 to C6 alkoxycarbonyl group;
Hydrazide compounds, wherein in the formula (1), $R^5$ is a C2 to C6 alkylcarbonyl group;
Hydrazide compounds, wherein in the formula (1), $R^5$ is an acetyl group;
Hydrazide compounds, wherein in the formula (1), $R^5$ is a benzoyl group optionally substituted by a group or groups selected from Group E2;
Hydrazide compounds, wherein in the formula (1), $R^5$ is a benzoyl group;
Hydrazide compounds, wherein in the formula (1), $R^6$ is a hydrogen atom;
Hydrazide compounds, wherein in the formula (1), $R^6$ is a C1 to C12 chain hydrocarbon group optionally substituted by a group or groups selected from Group E1;
Hydrazide compounds, wherein in the formula (1), $R^6$ is a methyl group;
Hydrazide compounds, wherein in the formula (1), G is a group shown by G-1, $Y^1$ is an oxygen atom, and $R^1$ is a trifluoromethyl group;
Hydrazide compounds, wherein in the formula (1), G is a group shown by G-1, $Y^1$ is an oxygen atom, $R^1$ is a trifluoromethyl group, and $Q^1$, $Q^2$, $Q^3$, and $Q^4$ are CH groups;
Hydrazide compounds, wherein in the formula (1), G is a group shown by G-1, $Y^1$ is an oxygen atom, $R^1$ is a trifluoromethyl group, $Q^1$, $Q^3$, and $Q^4$ are CH groups, $Q^2$ is a $CR^3$ group, and $R^3$ is as defined above;
Hydrazide compounds shown by the following formula (1-A) wherein in the formula (1), G is a group shown by G-1, $Y^1$ is an oxygen atom, $R^1$ is a trifluoromethyl group, $Q^1$, $Q^3$, and $Q^4$ are CH groups, $Q^2$ is a $CR^3$ group, $R^3$ is as defined above, $(R^2)_m$ is a substituent at 3- and 5-position, m is 2, and $R^2$ is respectively a chlorine atom;

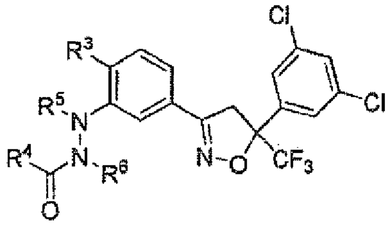
( 1-A )

Hydrazide compounds, wherein in the formula (1-A), $R^3$ is a hydrogen atom;
Hydrazide compounds, wherein in the formula (1-A), $R^3$ is a methyl group;
Hydrazide compounds, wherein in the formula (1-A), $R^3$ is an ethyl group;
Hydrazide compounds, wherein in the formula (1-A), $R^3$ is a halogen atom;
Hydrazide compounds, wherein in the formula (1-A), $R^3$ is a fluorine atom;
Hydrazide compounds, wherein in the formula (1-A), $R^3$ is a chlorine atom;
Hydrazide compounds, wherein in the formula (1-A), $R^3$ is a bromine atom;
Hydrazide compounds, wherein in the formula (1), $R^5$ is a hydrogen atom;
Hydrazide compounds, wherein in the formula (1), $R^5$ is a methyl group;
Hydrazide compounds, wherein in the formula (1-A), $R^6$ is a hydrogen atom;
Hydrazide compounds, wherein in the formula (1-A), $R^6$ is a methyl group;
Hydrazide compounds, wherein in the formula (1-A), $R^5$ is a hydrogen atom, and $R^6$ is a hydrogen atom;
Hydrazide compounds, wherein in the formula (1-A), $R^5$ is a hydrogen atom, and $R^6$ is a methyl group;
Hydrazide compounds, wherein in the formula (1-A), $R^5$ is a methyl group, and $R^6$ is a hydrogen atom;
Hydrazide compounds, wherein in the formula (1-A), $R^5$ is a methyl group, and $R^6$ is a methyl group;
Hydrazide compounds, wherein in the formula (1-A), $R^3$ is a halogen atom, and $R^5$ is a hydrogen atom;
Hydrazide compounds, wherein in the formula (1-A), $R^3$ is a halogen atom, and $R^5$ is a methyl group;
Hydrazide compounds, wherein in the formula (1-A), $R^3$ is a halogen atom, and $R^6$ is a hydrogen atom;
Hydrazide compounds, wherein in the formula (1-A), $R^3$ is a halogen atom, and $R^6$ is a methyl group;
Hydrazide compounds, wherein in the formula (1-A), $R^3$ is a chlorine atom, and $R^5$ is a hydrogen atom;
Hydrazide compounds, wherein in the formula (1-A), $R^3$ is a chlorine atom, and $R^5$ is a methyl group;
Hydrazide compounds, wherein in the formula (1-A), $R^3$ is a chlorine atom, and $R^6$ is a hydrogen atom;
Hydrazide compounds, wherein in the formula (1-A), $R^3$ is a chlorine atom, and $R^6$ is a methyl group;
Hydrazide compounds, wherein in the formula (1-A), $R^3$ is a hydrogen atom, $R^5$ is a hydrogen atom, and $R^6$ is a hydrogen atom;
Hydrazide compounds, wherein in the formula (1-A), $R^3$ is a methyl group, $R^5$ is a hydrogen atom, and $R^6$ is a hydrogen atom;
Hydrazide compounds, wherein in the formula (1-A), $R^3$ is an ethyl group, $R^5$ is a hydrogen atom, and $R^6$ is a hydrogen atom;
Hydrazide compounds, wherein in the formula (1-A), $R^3$ is a halogen atom, $R^5$ is a hydrogen atom, and $R^6$ is a hydrogen atom;
Hydrazide compounds, wherein in the formula (1-A), $R^3$ is a fluorine atom, $R^5$ is a hydrogen atom, and $R^6$ is a hydrogen atom;
Hydrazide compounds, wherein in the formula (1-A), $R^3$ is a chlorine atom, $R^5$ is a hydrogen atom, and $R^6$ is a hydrogen atom;
Hydrazide compounds, wherein in the formula (1-A), $R^3$ is a bromine atom, $R^5$ is a hydrogen atom, and $R^6$ is a hydrogen atom;
Hydrazide compounds, wherein in the formula (1-A), $R^3$ is a hydrogen atom, $R^5$ is a methyl group, and $R^6$ is a hydrogen atom;
Hydrazide compounds, wherein in the formula (1-A), $R^3$ is a methyl group, $R^5$ is a methyl group, and $R^6$ is a hydrogen atom;
Hydrazide compounds, wherein in the formula (1-A), $R^3$ is an ethyl group, $R^5$ is a methyl group, and $R^6$ is a hydrogen atom;
Hydrazide compounds, wherein in the formula (1-A), $R^3$ is a halogen atom, $R^5$ is a methyl group, and $R^6$ is a hydrogen atom;
Hydrazide compounds, wherein in the formula (1-A), $R^3$ is a fluorine atom, $R^5$ is a methyl group, and $R^6$ is a hydrogen atom;
Hydrazide compounds, wherein in the formula (1-A), $R^3$ is a chlorine atom, $R^5$ is a methyl group, and $R^6$ is a hydrogen atom;

Hydrazide compounds, wherein in the formula (1-A), $R^3$ is a bromine atom, $R^5$ is a methyl group, and $R^6$ is a hydrogen atom;
Hydrazide compounds, wherein in the formula (1-A), $R^3$ is a hydrogen atom, $R^5$ is a hydrogen atom, and $R^6$ is a methyl group;
Hydrazide compounds, wherein in the formula (1-A), $R^3$ is a methyl group, $R^5$ is a hydrogen atom, and $R^6$ is a methyl group;
Hydrazide compounds, wherein in the formula (1-A), $R^3$ is an ethyl group, $R^5$ is a hydrogen atom, and $R^6$ is a methyl group;
Hydrazide compounds, wherein in the formula (1-A), $R^3$ is a halogen atom, $R^5$ is a hydrogen atom, and $R^6$ is a methyl group;
Hydrazide compounds, wherein in the formula (1-A), $R^3$ is a fluorine atom, $R^5$ is a hydrogen atom, and $R^6$ is a methyl group;
Hydrazide compounds, wherein in the formula (1-A), $R^3$ is a chlorine atom, $R^5$ is a hydrogen atom, and $R^5$ is a methyl group;
Hydrazide compounds, wherein in the formula (1-A), $R^3$ is a bromine atom, $R^5$ is a hydrogen atom, and $R^6$ is a methyl group;
Hydrazide compounds, wherein in the formula (1-A), $R^3$ is a hydrogen atom, $R^5$ is a methyl group, and $R^6$ is a methyl group;
Hydrazide compounds, wherein in the formula (1-A), $R^3$ is a methyl group, $R^5$ is a methyl group, and $R^6$ is a methyl group;
Hydrazide compounds, wherein in the formula (1-A), $R^3$ is an ethyl group, $R^5$ is a methyl group, and $R^6$ is a methyl group;
Hydrazide compounds, wherein in the formula (1-A), $R^3$ is a halogen atom, $R^5$ is a methyl group, and $R^6$ is a methyl group;
Hydrazide compounds, wherein in the formula (1-A), $R^3$ is a fluorine atom, $R^5$ is a methyl group, and $R^6$ is a methyl group;
Hydrazide compounds, wherein in the formula (1-A), $R^3$ is a chlorine atom, $R^5$ is a methyl group, and $R^6$ is a methyl group;
Hydrazide compounds, wherein in the formula (1-A), $R^3$ is a bromine atom, $R^5$ is a methyl group, and $R^6$ is a methyl group;
Hydrazide compounds, wherein in the formula (1-A), $R^4$ is a C2 to C6 alkyl, a C1 to C6 haloalkyl, a C3 to C6 cycloalkyl group, or a (C1 to C6 alkoxy) C1 to C6 alkyl group;
Hydrazide compounds, wherein in the formula (1-A), $R^4$ is a C2 to C6 alkyl, a C1 to C6 haloalkyl, a C3 to C6 cycloalkyl group, or a (C1 to C6 alkoxy) C1 to C6 alkyl group, and $R^5$ is a hydrogen atom;
Hydrazide compounds, wherein in the formula (1-A), $R^4$ is a C2 to C6 alkyl, a C1 to C6 haloalkyl, a C3 to C6 cycloalkyl group, or a (C1 to C6 alkoxy) C1 to C6 alkyl group, and $R^5$ is a methyl group;
Hydrazide compounds, wherein in the formula (1-A), $R^4$ is a C2 to C6 alkyl, a C1 to C6 haloalkyl, a C3 to C6 cycloalkyl group, or a (C1 to C6 alkoxy) C1 to C6 alkyl group, and $R^6$ is a hydrogen atom;
Hydrazide compounds, wherein in the formula (1-A), $R^4$ is a C2 to C6 alkyl, a C1 to C6 haloalkyl, a C3 to C6 cycloalkyl group, or a (C1 to C6 alkoxy) C1 to C6 alkyl group, $R^5$ is a hydrogen atom, and $R^6$ is a hydrogen atom;
Hydrazide compounds, wherein in the formula (1-A), $R^4$ is a C2 to C6 alkyl, a C1 to C6 haloalkyl, a C3 to C6 cycloalkyl group, or a (C1 to C6 alkoxy) C1 to C6 alkyl group, $R^5$ is a methyl group, and $R^6$ is a hydrogen atom;
Hydrazide compounds, wherein in the formula (1-A), $R^3$ is a C1 to C6 alkyl group, a halogen atom, or a hydrogen atom, and $R^4$ is a C2 to C6 alkyl, a C1 to C6 haloalkyl, a C3 to C6 cycloalkyl group, or a (C1 to C6 alkoxy) C1 to C6 alkyl group;
Hydrazide compounds, wherein in the formula (1-A), $R^3$ is a C1 to C6 alkyl group, a halogen atom, or a hydrogen atom, $R^4$ is a C2 to C6 alkyl, a C1 to C6 haloalkyl, a C3 to C6 cycloalkyl group, or a (C1 to C6 alkoxy) C1 to C6 alkyl group, and $R^5$ is a hydrogen atom;
Hydrazide compounds, wherein in the formula (1-A), $R^3$ is a C1 to C6 alkyl group, a halogen atom, or a hydrogen atom, $R^4$ is a C2 to C6 alkyl, a C1 to C6 haloalkyl, a C3 to C6 cycloalkyl group, or a (C1 to C6 alkoxy) C1 to C6 alkyl group, and $R^5$ is a methyl group;
Hydrazide compounds, wherein in the formula (1-A), $R^3$ is a C1 to C6 alkyl group, a halogen atom, or a hydrogen atom, $R^4$ is a C2 to C6 alkyl, a C1 to C6 haloalkyl, a C3 to C6 cycloalkyl group, or a (C1 to C6 alkoxy) C1 to C6 alkyl group, and $R^6$ is a hydrogen atom;
Hydrazide compounds, wherein in the formula (1-A), $R^3$ is a C1 to C6 alkyl group, a halogen atom, or a hydrogen atom, $R^4$ is a C2 to C6 alkyl, a C1 to C6 haloalkyl, a C3 to C6 cycloalkyl group, or a (C1 to C6 alkoxy) C1 to C6 alkyl group, $R^5$ is a hydrogen atom, and $R^6$ is a hydrogen atom;
Hydrazide compounds, wherein in the formula (1-A), $R^3$ is a C1 to C6 alkyl group, a halogen atom, or a hydrogen

atom, $R^4$ is a C2 to C6 alkyl, a C1 to C6 haloalkyl, a C3 to C6 cycloalkyl group, or a (C1 to C6 alkoxy) C1 to C6 alkyl group, $R^5$ is a methyl group, and $R^6$ is a hydrogen atom;

Hydrazide compounds, wherein in the formula (1-A), $R^3$ is a chlorine atom, and $R^4$ is a C2 to C6 alkyl, a C1 to C6 haloalkyl, a C3 to C6 cycloalkyl group, or a (C1 to C6 alkoxy) C1 to C6 alkyl group;

Hydrazide compounds, wherein in the formula (1-A), $R^3$ is a chlorine atom, $R^4$ is a C2 to C6 alkyl, a C1 to C6 haloalkyl, a C3 to C6 cycloalkyl group, or a (C1 to C6 alkoxy) C1 to C6 alkyl group, and $R^5$ is a hydrogen atom;

Hydrazide compounds, wherein in the formula (1-A), $R^3$ is a chlorine atom, $R^4$ is a C2 to C6 alkyl, a C1 to C6 haloalkyl, a C3 to e6 cycloalkyl group, or a (C1 to C6 alkoxy) C1 to C6 alkyl group, and $R^5$ is a methyl group;

Hydrazide compounds, wherein in the formula (1-A), $R^3$ is a chlorine atom, $R^4$ is a C2 to C6 alkyl, a C1 to C6 haloalkyl, a C3 to C6 cycloalkyl group, or a (C1 to C6 alkoxy) C1 to C6 alkyl group, and $R^6$ is a hydrogen atom;

Hydrazide compounds, wherein in the formula (1-A), $R^3$ is a chlorine atom, $R^4$ is a C2 to C6 alkyl, a C1 to C6 haloalkyl, a C3 to C6 cycloalkyl group, or a (C1 to C6 alkoxy) C1 to C6 alkyl group, $R^5$ is a hydrogen atom, and $R^6$ is a hydrogen atom;

Hydrazide compounds, wherein in the formula (1-A), $R^3$ is a chlorine atom, $R^4$ is a C2 to C6 alkyl, a C1 to C6 haloalkyl, a C3 to C6 cycloalkyl group, or a (C1 to C6 alkoxy) C1 to C6 alkyl group, $R^5$ is a methyl group, and $R^6$ is a hydrogen atom;

Hydrazide compounds, wherein in the formula (1-A), $R^4$ is a C1 to C6 alkyl group;

Hydrazide compounds, wherein in the formula (1-A), $R^4$ is a C2 to C6 alkyl group;

Hydrazide compounds, wherein in the formula (1-A), $R^4$ is a methyl group;

Hydrazide compounds, wherein in the formula (1-A), $R^4$ is an ethyl group;

Hydrazide compounds, wherein in the formula (1-A), $R^4$ is a propyl group;

Hydrazide compounds, wherein in the formula (1-A), $R^4$ is an isopropyl group;

Hydrazide compounds, wherein in the formula (1-A), $R^4$ is a butyl group;

Hydrazide compounds, wherein in the formula (1-A), $R^4$ is a t-butyl group;

Hydrazide compounds, wherein in the formula (1-A), $R^4$ is a 2-methylpropyl group;

Hydrazide compounds, wherein in the formula (1-A), $R^4$ is a pentyl group;

Hydrazide compounds, wherein in the formula (1-A), $R^3$ is a halogen atom, and $R^4$ is a C2 to C6 alkyl group;

Hydrazide compounds, wherein in the formula (1-A), $R^3$ is a halogen atom, $R^4$ is a C2 to C6 alkyl group, and $R^6$ is a hydrogen atom;

Hydrazide compounds, wherein in the formula (1-A), $R^3$ is a halogen atom, $R^4$ is a C2 to C6 alkyl group, $R^5$ is a hydrogen atom, and $R^6$ is a hydrogen atom;

Hydrazide compounds, wherein in the formula (1-A), $R^3$ is a halogen atom, $R^4$ is a C2 to C6 alkyl group, $R^5$ is a methyl group, and $R^6$ is a hydrogen atom;

Hydrazide compounds, wherein in the formula (1-A), $R^3$ is a chlorine atom, and $R^4$ is a C2 to C6 alkyl group;

Hydrazide compounds, wherein in the formula (1-A), $R^3$ is a chlorine atom, $R^4$ is a C2 to C6 alkyl group, and $R^6$ is a hydrogen atom;

Hydrazide compounds, wherein in the formula (1-A), $R^3$ is a chlorine atom, $R^4$ is a C2 to C6 alkyl group, $R^5$ is a hydrogen atom, and $R^6$ is a hydrogen atom;

Hydrazide compounds, wherein in the formula (1-A), $R^3$ is a chlorine atom, $R^4$ is a C2 to C6 alkyl group, $R^5$ is a methyl group, and $R^6$ is a hydrogen atom;

Hydrazide compounds, wherein in the formula (1-A), $R^4$ is a methyl group, and $R^5$ is a hydrogen atom;

Hydrazide compounds, wherein in the formula (1-A), $R^4$ is a methyl group, and $R^5$ is a methyl group;

Hydrazide compounds, wherein in the formula (1-A), $R^4$ is a methyl group, and $R^6$ is a hydrogen atom;

Hydrazide compounds, wherein in the formula (1-A), $R^4$ is a methyl group, and $R^6$ is a methyl group;

Hydrazide compounds, wherein in the formula (1-A), $R^4$ is a methyl group, $R^5$ is a hydrogen atom, and $R^6$ is a hydrogen atom;

Hydrazide compounds, wherein in the formula (1-A), $R^4$ is a methyl group, $R^4$ is a methyl group, and $R^6$ is a hydrogen atom;

Hydrazide compounds, wherein in the formula (1-A), $R^4$ is a methyl group, $R^4$ is a hydrogen atom, and $R^6$ is a methyl group;

Hydrazide compounds, wherein in the formula (1-A), $R^4$ is a methyl group, $R^4$ is a methyl group, and $R^6$ is a methyl group;

Hydrazide compounds, wherein in the formula (1-A), $R^4$ is an ethyl group, and $R^5$ is a hydrogen atom;

Hydrazide compounds, wherein in the formula (1-A), $R^4$ is an ethyl group, and $R^5$ is a methyl group;

Hydrazide compounds, wherein in the formula (1-A), $R^4$ is an ethyl group, and $R^6$ is a hydrogen atom;

Hydrazide compounds, wherein in the formula (1-A), $R^4$ is an ethyl group, and $R^6$ is a methyl group;

Hydrazide compounds, wherein in the formula (1-A), $R^4$ is an ethyl group, $R^5$ is a hydrogen atom, and $R^6$ is a hydrogen atom;

Hydrazide compounds, wherein in the formula (1-A), $R^4$ is an ethyl group, $R^5$ is a methyl group, and $R^6$ is a hydrogen atom;
Hydrazide compounds, wherein in the formula (1-A), $R^4$ is an ethyl group, $R^4$ is a hydrogen atom, and $R^6$ is a methyl group;
Hydrazide compounds, wherein in the formula (1-A), $R^4$ is an ethyl group, $R^5$ is a methyl group, and $R^6$ is a methyl group;
Hydrazide compounds, wherein in the formula (1-A), $R^4$ is a propyl group, and $R^5$ is a hydrogen atom;
Hydrazide compounds, wherein in the formula (1-A), $R^4$ is a propyl group, and $R^5$ is a methyl group;
Hydrazide compounds, wherein in the formula (1-A), $R^4$ is a propyl group, and $R^6$ is a hydrogen atom;
Hydrazide compounds, wherein in the formula (1-A), $R^4$ is a propyl group, and $R^6$ is a methyl group;
Hydrazide compounds, wherein in the formula (1-A), $R^4$ is a propyl group, $R^5$ is a hydrogen atom, and $R^6$ is a hydrogen atom;
Hydrazide compounds, wherein in the formula (1-A), $R^4$ is a propyl group, $R^4$ is a methyl group, and $R^6$ is a hydrogen atom;
Hydrazide compounds, wherein in the formula (1-A), $R^4$ is a propyl group, $R^5$ is a hydrogen atom, and $R^6$ is a methyl group;
Hydrazide compounds, wherein in the formula (1-A), $R^4$ is a propyl group, $R^5$ is a methyl group, and $R^6$ is a methyl group;
Hydrazide compounds, wherein in the formula (1-A), $R^4$ is an isopropyl group, and $R^5$ is a hydrogen atom;
Hydrazide compounds, wherein in the formula (1-A), $R^4$ is an isopropyl group, and $R^5$ is a methyl group;
Hydrazide compounds, wherein in the formula (1-A), $R^4$ is an isopropyl group, and $R^6$ is a hydrogen atom;
Hydrazide compounds, wherein in the formula (1-A), $R^4$ is an isopropyl group, and $R^6$ is a methyl group;
Hydrazide compounds, wherein in the formula (1-A), $R^4$ is an isopropyl group, $R^5$ is a hydrogen atom, and $R^6$ is a hydrogen atom;
Hydrazide compounds, wherein in the formula (1-A), $R^4$ is an isopropyl group, $R^5$ is a methyl group, and $R^6$ is a hydrogen atom;
Hydrazide compounds, wherein in the formula (1-A), $R^4$ is an isopropyl group, $R^5$ is a hydrogen atom, and $R^6$ is a methyl group;
Hydrazide compounds, wherein in the formula (1-A), $R^4$ is an isopropyl group, $R^4$ is a methyl group, and $R^6$ is a methyl group;
Hydrazide compounds, wherein in the formula (1-A), $R^4$ is a butyl group, and $R^5$ is a hydrogen atom;
Hydrazide compounds, wherein in the formula (1-A), $R^4$ is a butyl group, and $R^5$ is a methyl group;
Hydrazide compounds, wherein in the formula (1-A), $R^4$ is a butyl group, and $R^6$ is a hydrogen atom;
Hydrazide compounds, wherein in the formula (1-A), $R^4$ is a butyl group, and $R^6$ is a methyl group;
Hydrazide compounds, wherein in the formula (1-A), $R^4$ is a butyl group, $R^5$ is a hydrogen atom, and $R^6$ is a hydrogen atom;
Hydrazide compounds, wherein in the formula (1-A), $R^4$ is a butyl group, $R^5$ is a methyl group, and $R^6$ is a hydrogen atom;
Hydrazide compounds, wherein in the formula (1-A), $R^4$ is a butyl group, $R^5$ is a hydrogen atom, and $R^6$ is a methyl group;
Hydrazide compounds, wherein in the formula (1-A), $R^4$ is a butyl group, $R^5$ is a methyl group, and $R^6$ is a methyl group;
Hydrazide compounds, wherein in the formula (1-A), $R^4$ is a t-butyl group, and $R^5$ is a hydrogen atom;
Hydrazide compounds, wherein in the formula (1-A), $R^4$ is a t-butyl group, and $R^5$ is a methyl group;
Hydrazide compounds, wherein in the formula (1-A), $R^4$ is a t-butyl group, and $R^6$ is a hydrogen atom;
Hydrazide compounds, wherein in the formula (1-A), $R^4$ is a t-butyl group, and $R^6$ is a methyl group;
Hydrazide compounds, wherein in the formula (1-A), $R^4$ is a t-butyl group, $R^5$ is a hydrogen atom, and $R^6$ is a hydrogen atom;
Hydrazide compounds, wherein in the formula (1-A), $R^4$ is a t-butyl group, $R^5$ is a methyl group, and $R^6$ is a hydrogen atom;
Hydrazide compounds, wherein in the formula (1-A), $R^4$ is a t-butyl group, $R^5$ is a hydrogen atom, and $R^6$ is a methyl group;
Hydrazide compounds, wherein in the formula (1-A), $R^4$ is a t-butyl group, $R^5$ is a methyl group, and $R^6$ is a methyl group;
Hydrazide compounds, wherein in the formula (1-A), $R^4$ is a 2-methylpropyl group, and $R^5$ is a hydrogen atom;
Hydrazide compounds, wherein in the formula (1-A), $R^4$ is a 2-methylpropyl group, and $R^5$ is a methyl group;
Hydrazide compounds, wherein in the formula (1-A), $R^4$ is a 2-methylpropyl group, and $R^6$ is a hydrogen atom;
Hydrazide compounds, wherein in the formula (1-A), $R^4$ is a 2-methylpropyl group, and $R^6$ is a methyl group;
Hydrazide compounds, wherein in the formula (1-A), $R^4$ is a 2-methylpropyl group, $R^5$ is a hydrogen atom, and $R^6$

is a hydrogen atom;

Hydrazide compounds, wherein in the formula (1-A), $R^4$ is a 2-methylpropyl group, $R^5$ is a methyl group, and $R^5$ is a hydrogen atom;

Hydrazide compounds, wherein in the formula (1-A), $R^4$ is a 2-methylpropyl group, $R^5$ is a hydrogen atom, and $R^6$ is a methyl group;

Hydrazide compounds, wherein in the formula (1-A), $R^4$ is a 2-methylpropyl group, $R^5$ is a methyl group, and $R^6$ is a methyl group;

Hydrazide compounds, wherein in the formula (1-A), $R^4$ is a pentyl group, and $R^5$ is a hydrogen atom;

Hydrazide compounds, wherein in the formula (1-A), $R^4$ is a pentyl group, and $R^5$ is a methyl group;

Hydrazide compounds, wherein in the formula (1-A), $R^4$ is a pentyl group, and $R^6$ is a hydrogen atom;

Hydrazide compounds, wherein in the formula (1-A), $R^4$ is a pentyl group, and $R^6$ is a methyl group;

Hydrazide compounds, wherein in the formula (1-A), $R^4$ is a pentyl group, $R^5$ is a hydrogen atom, and $R^6$ is a hydrogen atom;

Hydrazide compounds, wherein in the formula (1-A), $R^4$ is a pentyl group, $R^5$ is a methyl group, and $R^6$ is a hydrogen atom;

Hydrazide compounds, wherein in the formula (1-A), $R^4$ is a pentyl group, $R^5$ is a hydrogen atom, and $R^6$ is a methyl group;

Hydrazide compounds, wherein in the formula (1-A), $R^4$ is a pentyl group, $R^5$ is a methyl group, and $R^6$ is a methyl group;

Hydrazide compounds, wherein in the formula (1-A), $R^4$ is a C1 to C6 haloalkyl group;

Hydrazide compounds, wherein in the formula (1-A), $R^4$ is a C1 to C6 fluoroalkyl group;

Hydrazide compounds, wherein in the formula (1-A), $R^4$ is a 2,2,2-trifluoroethyl group;

Hydrazide compounds, wherein in the formula (1-A), $R^4$ is a 3,3,3-trifluoropropyl group;

Hydrazide compounds, wherein in the formula (1-A), $R^4$ is a 4,4,4-trifluorobutyl group;

Hydrazide compounds, wherein in the formula (1-A), $R^3$ is a halogen atom, and $R^4$ is a C1 to C6 fluoroalkyl group;

Hydrazide compounds, wherein in the formula (1-A), $R^3$ is a halogen atom, and $R^4$ is a 2,2,2-trifluoroethyl group;

Hydrazide compounds, wherein in the formula (1-A), $R^3$ is a halogen atom, and $R^4$ is a 3,3,3-trifluoropropyl group;

Hydrazide compounds, wherein in the formula (1-A), $R^3$ is a halogen atom, and $R^4$ is a 4,4,4-trifluorobutyl group;

Hydrazide compounds, wherein in the formula (1-A), $R^3$ is a halogen atom, $R^4$ is a C1 to C6 fluoroalkyl group, and $R^5$ is a hydrogen atom;

Hydrazide compounds, wherein in the formula (1-A), $R^3$ is a halogen atom, $R^4$ is a C1 to C6 fluoroalkyl group, and $R^5$ is a methyl group;

Hydrazide compounds, wherein in the formula (1-A), $R^3$ is a halogen atom, $R^4$ is a C1 to C6 fluoroalkyl group, and $R^6$ is a hydrogen atom;

Hydrazide compounds, wherein in the formula (1-A), $R^3$ is a halogen atom, $R^4$ is a C1 to C6 fluoroalkyl group, and $R^6$ is a methyl group;

Hydrazide compounds, wherein in the formula (1-A), $R^3$ is a halogen atom, $R^4$ is a C1 to C6 fluoroalkyl group, $R^5$ is a hydrogen atom, and $R^6$ is a hydrogen atom;

Hydrazide compounds, wherein in the formula (1-A), $R^3$ is a halogen atom, $R^4$ is a C1 to C6 fluoroalkyl group, $R^5$ is a methyl group, and $R^6$ is a hydrogen atom;

Hydrazide compounds, wherein in the formula (1-A), $R^3$ is a chlorine atom, and $R^4$ is a C1 to C6 fluoroalkyl group;

Hydrazide compounds, wherein in the formula (1-A), $R^3$ is a chlorine atom, and $R^4$ is a 2,2,2-trifluoroethyl group;

Hydrazide compounds, wherein in the formula (1-A), $R^3$ is a chlorine atom, and $R^4$ is a 3,3,3-trifluoropropyl group;

Hydrazide compounds, wherein in the formula (1-A), $R^3$ is a chlorine atom, and $R^4$ is a 4,4,4-trifluorobutyl group;

Hydrazide compounds, wherein in the formula (1-A), $R^3$ is a chlorine atom, $R^4$ is a C1 to C6 fluoroalkyl group, and $R^5$ is a hydrogen atom;

Hydrazide compounds, wherein in the formula (1-A), $R^3$ is a chlorine atom, $R^4$ is a C1 to C6 fluoroalkyl group, and $R^5$ is a methyl group;

Hydrazide compounds, wherein in the formula (1-A), $R^3$ is a chlorine atom, $R^4$ is a C1 to C6 fluoroalkyl group, and $R^6$ is a hydrogen atom;

Hydrazide compounds, wherein in the formula (1-A), $R^3$ is a chlorine atom, $R^4$ is a C1 to C6 fluoroalkyl group, and $R^6$ is a methyl group;

Hydrazide compounds, wherein in the formula (1-A), $R^3$ is a chlorine atom, $R^4$ is a C1 to C6 fluoroalkyl group, $R^5$ is a hydrogen atom, and $R^6$ is a hydrogen atom;

Hydrazide compounds, wherein in the formula (1-A), $R^3$ is a chlorine atom, $R^4$ is a C1 to C6 fluoroalkyl group, $R^5$ is a methyl group, and $R^6$ is a hydrogen atom;

Hydrazide compounds, wherein in the formula (1-A), $R^4$ is a 2,2,2-trifluoroethyl group, and $R^5$ is a hydrogen atom;

Hydrazide compounds, wherein in the formula (1-A), $R^4$ is a 2,2,2-trifluoroethyl group, and $R^5$ is a methyl group;

Hydrazide compounds, wherein in the formula (1-A), $R^4$ is a 2,2,2-trifluoroethyl group, and $R^6$ is a hydrogen atom;
Hydrazide compounds, wherein in the formula (1-A), $R^4$ is a 2,2,2-trifluoroethyl group, and $R^6$ is a methyl group;
Hydrazide compounds, wherein in the formula (1-A), $R^4$ is a 2,2,2-trifluoroethyl group, $R^5$ is a hydrogen atom, and $R^6$ is a hydrogen atom;
Hydrazide compounds, wherein in the formula (1-A), $R^4$ is a 2,2,2-trifluoroethyl group, $R^5$ is a methyl group, and $R^6$ is a hydrogen atom;
Hydrazide compounds, wherein in the formula (1-A), $R^4$ is a 2,2,2-trifluoroethyl group, $R^5$ is a hydrogen atom, and $R^6$ is a methyl group;
Hydrazide compounds, wherein in the formula (1-A), $R^4$ is a 2,2,2-trifluoroethyl group, $R^5$ is a methyl group, and $R^6$ is a methyl group; '
Hydrazide compounds, wherein in the formula (1-A), $R^4$ is a 3,3,3-trifluoropropyl group, and $R^5$ is a hydrogen atom;
Hydrazide compounds, wherein in the formula (1-A), $R^4$ is a 3,3,3-trifluoropropyl group, and $R^5$ is a methyl group;
Hydrazide compounds, wherein in the formula (1-A), $R^4$ is a 3,3,3-trifluoropropyl group, and $R^6$ is a hydrogen atom;
Hydrazide compounds, wherein in the formula (1-A), $R^4$ is a 3,3,3-trifluoropropyl group, and $R^6$ is a methyl group;
Hydrazide compounds, wherein in the formula (1-A), $R^4$ is a 3,3,3-trifluoropropyl group, $R^5$ is a hydrogen atom, and $R^6$ is a hydrogen atom;
Hydrazide compounds, wherein in the formula (1-A), $R^4$ is a 3,3,3-trifluoropropyl group, $R^5$ is a methyl group, and $R^6$ is a hydrogen atom;
Hydrazide compounds, wherein in the formula (1-A), $R^4$ is a 3,3,3-trifluoropropyl group, $R^5$ is a hydrogen atom, and $R^6$ is a methyl group;
Hydrazide compounds, wherein in the formula (1-A), $R^4$ is a 3,3,3-trifluoropropyl group, $R^5$ is a methyl group, and $R^6$ is a methyl group;
Hydrazide compounds, wherein in the formula (1-A), $R^4$ is a 4,4,4-trifluorobutyl group, and $R^5$ is a hydrogen atom;
Hydrazide compounds, wherein in the formula (1-A), $R^4$ is a 4,4,4-trifluorobutyl group, and $R^5$ is a methyl group;
Hydrazide compounds, wherein in the formula (1-A), $R^4$ is a 4,4,4-trifluorobutyl group, and $R^6$ is a hydrogen atom;
Hydrazide compounds, wherein in the formula (1-A), $R^4$ is a 4,4,4-trifluorobutyl group, and $R^6$ is a methyl group;
Hydrazide compounds, wherein in the formula (1-A), $R^4$ is a 4,4,4-trifluorobutyl group, $R^5$ is a hydrogen atom, and $R^6$ is a hydrogen atom;
Hydrazide compounds, wherein in the formula (1-A), $R^4$ is a 4,4,4-trifluorobutyl group, $R^5$ is a methyl group, and $R^6$ is a hydrogen atom;
Hydrazide compounds, wherein in the formula (1-A), $R^4$ is a 4,4,4-trifluorobutyl group, $R^5$ is a hydrogen atom, and $R^6$ is a methyl group;
Hydrazide compounds, wherein in the formula (1-A), $R^4$ is a 4,4,4-trifluorobutyl group, $R^5$ is a methyl group, and $R^6$ is a methyl group;
Hydrazide compounds, wherein in the formula (1-A), $R^4$ is a C3 to C6 cycloalkyl group;
Hydrazide compounds, wherein in the formula (1-A), $R^4$ is a cyclopropyl group;
Hydrazide compounds, wherein in the formula (1-A), $R^4$ is a cyclobutyl group;
Hydrazide compounds, wherein in the formula (1-A), $R^4$ is a cyclopentyl group;
Hydrazide compounds, wherein in the formula (1-A), $R^4$ is a cyclohexyl group;
Hydrazide compounds, wherein in the formula (1-A), $R^3$ is a halogen atom, and $R^5$ is a cyclopropyl group;
Hydrazide compounds, wherein in the formula (1-A), $R^3$ is a halogen atom, $R^4$ is a cyclopropyl group, and $R^6$ is a hydrogen atom;
Hydrazide compounds, wherein in the formula (1-A), $R^3$ is a halogen atom, and $R^4$ is a cyclobutyl group;
Hydrazide compounds, wherein in the formula (1-A), $R^3$ is a halogen atom, $R^4$ is a cyclobutyl group, and $R^6$ is a hydrogen atom;
Hydrazide compounds, wherein in the formula (1-A), $R^3$ is a halogen atom, and $R^4$ is a cyclopentyl group;
Hydrazide compounds, wherein in the formula (1-A), $R^3$ is a halogen atom, $R^4$ is a cyclopentyl group, and $R^6$ is a hydrogen atom;
Hydrazide compounds, wherein in the formula (1-A), $R^3$ is a halogen atom, and $R^4$ is a cyclohexyl group;
Hydrazide compounds, wherein in the formula (1-A), $R^3$ is a halogen atom, $R^4$ is a cyclohexyl group, and $R^6$ is a hydrogen atom;
Hydrazide compounds, wherein in the formula (1-A), $R^3$ is a chlorine atom, and $R^4$ is a cyclopropyl group;
Hydrazide compounds, wherein in the formula (1-A), $R^3$ is a chlorine atom, $R^4$ is a cyclopropyl group, and $R^6$ is a hydrogen atom;
Hydrazide compounds, wherein in the formula (1-A), $R^3$ is a chlorine atom, and $R^4$ is a cyclobutyl group;
Hydrazide compounds, wherein in the formula (1-A), $R^3$ is a chlorine atom, $R^4$ is a cyclobutyl group, and $R^6$ is a hydrogen atom;
Hydrazide compounds, wherein in the formula (1-A), $R^3$ is a chlorine atom, and $R^4$ is a cyclopentyl group;

Hydrazide compounds, wherein in the formula (1-A), $R^3$ is a chlorine atom, $R^4$ is a cyclopentyl group, and $R^6$ is a hydrogen atom;
Hydrazide compounds, wherein in the formula (1-A), $R^3$ is a chlorine atom, and $R^4$ is a cyclohexyl group;
Hydrazide compounds, wherein in the formula (1-A), $R^3$ is a chlorine atom, $R^4$ is a cyclohexyl group, and $R^6$ is a hydrogen atom;
Hydrazide compounds, wherein in the formula (1-A), $R^4$ is a cyclopropyl group, and $R^5$ is a hydrogen atom;
Hydrazide compounds, wherein in the formula (1-A), $R^4$ is a cyclopropyl group, and $R^5$ is a methyl group;
Hydrazide compounds, wherein in the formula (1-A), $R^4$ is a cyclopropyl group, and $R^6$ is a hydrogen atom;
Hydrazide compounds, wherein in the formula (1-A), $R^4$ is a cyclopropyl group, and $R^6$ is a methyl group;
Hydrazide compounds, wherein in the formula (1-A), $R^4$ is a cyclopropyl group, $R^5$ is a hydrogen atom, and $R^6$ is a hydrogen atom;
Hydrazide compounds, wherein in the formula (1-A), $R^4$ is a cyclopropyl group, $R^5$ is a methyl group, and $R^6$ is a hydrogen atom;
Hydrazide compounds, wherein in the formula (1-A), $R^4$ is a cyclopropyl group, $R^5$ is a hydrogen atom, and $R^6$ is a methyl group;
Hydrazide compounds, wherein in the formula (1-A), $R^4$ is a cyclopropyl group, $R^5$ is a methyl group, and $R^6$ is a methyl group;
Hydrazide compounds, wherein in the formula (1-A), $R^4$ is a cyclobutyl group, and $R^5$ is a hydrogen atom;
Hydrazide compounds, wherein in the formula (1-A), $R^4$ is a cyclobutyl group, and $R^5$ is a methyl group;
Hydrazide compounds, wherein in the formula (1-A), $R^4$ is a cyclobutyl group, and $R^6$ is a hydrogen atom;
Hydrazide compounds, wherein in the formula (1-A), $R^4$ is a cyclobutyl group, and $R^6$ is a methyl group;
Hydrazide compounds, wherein in the formula (1-A), $R^4$ is a cyclobutyl group, $R^5$ is a hydrogen atom, and $R^6$ is a hydrogen atom;
Hydrazide compounds, wherein in the formula (1-A), $R^4$ is a cyclobutyl group, $R^5$ is a methyl group, and $R^6$ is a hydrogen atom;
Hydrazide compounds, wherein in the formula (1-A), $R^4$ is a cyclobutyl group, $R^5$ is a hydrogen atom, and $R^6$ is a methyl group;
Hydrazide compounds, wherein in the formula (1-A), $R^4$ is a cyclobutyl group, $R^5$ is a methyl group, and $R^6$ is a methyl group;
Hydrazide compounds, wherein in the formula (1-A), $R^4$ is a cyclopentyl group, and $R^5$ is a hydrogen atom;
Hydrazide compounds, wherein in the formula (1-A), $R^4$ is a cyclopentyl group, and $R^5$ is a methyl group;
Hydrazide compounds, wherein in the formula (1-A), $R^4$ is a cyclopentyl group, and $R^6$ is a hydrogen atom;
Hydrazide compounds, wherein in the formula (1-A), $R^4$ is a cyclopentyl group, and $R^6$ is a methyl group;
Hydrazide compounds, wherein in the formula (1-A), $R^4$ is a cyclopentyl group, $R^5$ is a hydrogen atom, and $R^6$ is a hydrogen atom;
Hydrazide compounds, wherein in the formula (1-A), $R^4$ is a cyclopentyl group, $R^5$ is a methyl group, and $R^6$ is a hydrogen atom;
Hydrazide compounds, wherein in the formula (1-A), $R^4$ is a cyclopentyl group, $R^5$ is a hydrogen atom, and $R^6$ is a methyl group;
Hydrazide compounds, wherein in the formula (1-A), $R^4$ is a cyclopentyl group, $R^5$ is a methyl group, and $R^6$ is a methyl group;
Hydrazide compounds, wherein in the formula (1-A), $R^4$ is a cyclohexyl group, and $R^5$ is a hydrogen atom;
Hydrazide compounds, wherein in the formula (1-A), $R^4$ is a cyclohexyl group, and $R^5$ is a methyl group;
Hydrazide compounds, wherein in the formula (1-A), $R^4$ is a cyclohexyl group, and $R^6$ is a hydrogen atom;
Hydrazide compounds, wherein in the formula (1-A), $R^4$ is a cyclohexyl group, and $R^6$ is a methyl group;
Hydrazide compounds, wherein in the formula (1-A), $R^4$ is a cyclohexyl group, $R^5$ is a hydrogen atom, and $R^6$ is a hydrogen atom;
Hydrazide compounds, wherein in the formula (1-A), $R^4$ is a cyclohexyl group, $R^5$ is a methyl group, and $R^6$ is a hydrogen atom;
Hydrazide compounds, wherein in the formula (1-A), $R^4$ is a cyclohexyl group, $R^5$ is a hydrogen atom, and $R^6$ is a methyl group;
Hydrazide compounds, wherein in the formula (1-A), $R^4$ is a cyclohexyl group, $R^5$ is a methyl group, and $R^6$ is a methyl group;
Hydrazide compounds, wherein in the formula (1-A), $R^4$ is a (C1 to C6 alkoxy) C1 to C6 alkyl group;
Hydrazide compounds, wherein in the formula (1-A), $R^4$ is a (C1 to C6 alkoxy)methyl group;
Hydrazide compounds, wherein in the formula (1-A), $R^4$ is a methoxymethyl group;
Hydrazide compounds, wherein in the formula (1-A), $R^4$ is an ethoxymethyl group;
Hydrazide compounds, wherein in the formula (1-A), $R^4$ is a (C1 to C6 alkoxy)methyl group, and $R^5$ is a hydrogen atom;

Hydrazide compounds, wherein in the formula (1-A), $R^4$ is a (C1 to C6 alkoxy)methyl group, and $R^5$ is a methyl group;
Hydrazide compounds, wherein in the formula (1-A), $R^4$ is a (C1 to C6 alkoxy)methyl group, and $R^6$ is a hydrogen atom;
Hydrazide compounds, wherein in the formula (1-A), $R^4$ is a (C1 to C6 alkoxy)methyl group, and $R^6$ is a methyl group;
Hydrazide compounds, wherein in the formula (1-A), $R^4$ is a (C1 to C6 alkoxy)methyl group, $R^5$ is a hydrogen atom, and $R^6$ is a hydrogen atom;
Hydrazide compounds, wherein in the formula (1-A), $R^4$ is a (C1 to C6 alkoxy)methyl group, $R^5$ is a methyl group, and $R^6$ is a hydrogen atom;
Hydrazide compounds, wherein in the formula (1-A), $R^4$ is a (C1 to C6 alkoxy)methyl group, $R^5$ is a hydrogen atom, and $R^6$ is a methyl group;
Hydrazide compounds, wherein in the formula (1-A), $R^4$ is a (C1 to C6 alkoxy)methyl group, $R^5$ is a methyl group, and $R^6$ is a methyl group;
Hydrazide compounds, wherein in the formula (1-A), $R^4$ is a methoxymethyl group, and $R^5$ is a hydrogen atom;
Hydrazide compounds, wherein in the formula (1-A), $R^4$ is a methoxymethyl group, and $R^5$ is a methyl group;
Hydrazide compounds, wherein in the formula (1-A), $R^4$ is a methoxymethyl group, and $R^6$ is a hydrogen atom;
Hydrazide compounds, wherein in the formula (1-A), $R^4$ is a methoxymethyl group, and $R^6$ is a methyl group;
Hydrazide compounds, wherein in the formula (1-A), $R^4$ is a methoxymethyl group, $R^5$ is a hydrogen atom, and $R^6$ is a hydrogen atom;
Hydrazide compounds, wherein in the formula (1-A) , $R^4$ is a methoxymethyl group, $R^5$ is a methyl group, and $R^6$ is a hydrogen atom;
Hydrazide compounds, wherein in the formula (1-A), $R^4$ is a methoxymethyl group, $R^5$ is a hydrogen atom, and $R^6$ is a methyl group;
Hydrazide compounds, wherein in the formula (1-A), $R^4$ is a methoxymethyl group, $R^5$ is a methyl group, and $R^6$ is a methyl group;
Hydrazide compounds, wherein in the formula (1-A), $R^3$ is a halogen atom, and $R^4$ is a (C1 to C6 alkoxy) methyl group;
Hydrazide compounds, wherein in the formula (1-A), $R^3$ is a halogen atom, $R^4$ is a (C1 to C6 alkoxy)methyl group, and $R^5$ is a hydrogen atom;
Hydrazide compounds, wherein in the formula (1-A), $R^3$ is a halogen atom, $R^4$ is a (C1 to C6 alkoxy)methyl group, and $R^5$ is a methyl group;
Hydrazide compounds, wherein in the formula (1-A), $R^3$ is a halogen atom, $R^4$ is a (C1 to C6 alkoxy) methyl group, and $R^6$ is a hydrogen atom;
Hydrazide compounds, wherein in the formula (1-A), $R^3$ is a halogen atom, $R^4$ is a (C1 to C6 alkoxy) methyl group, and $R^6$ is a methyl group;
Hydrazide compounds, wherein in the formula (1-A), $R^3$ is a halogen atom, $R^4$ is a (C1 to C6 alkoxy) methyl group, $R^5$ is a hydrogen atom, and $R^6$ is a hydrogen atom;
Hydrazide compounds, wherein in the formula (1-A), $R^3$ is a halogen atom, $R^4$ is a (C1 to C6 alkoxy) methyl group, $R^5$ is a methyl group, and $R^6$ is a hydrogen atom;
Hydrazide compounds, wherein in the formula (1-A), $R^3$ is a halogen atom, $R^4$ is a (C1 to C6 alkoxy)methyl group, $R^5$ is a hydrogen atom, and $R^6$ is a methyl group;
Hydrazide compounds, wherein in the formula (1-A), $R^3$ is a halogen atom, $R^4$ is a (C1 to C6 alkoxy)methyl group, $R^5$ is a methyl group, and $R^6$ is a methyl group;
Hydrazide compounds, wherein in the formula (1-A), $R^3$ is a chlorine atom, and $R^4$ is a (C1 to C6 alkoxy)methyl group;
Hydrazide compounds, wherein in the formula (1-A), $R^3$ is a chlorine atom, $R^4$ is a (C1 to C6 alkoxy) methyl group, and $R^5$ is a hydrogen atom;
Hydrazide compounds, wherein in the formula (1-A), $R^3$ is a chlorine atom, $R^4$ is a (C1 to C6 alkoxy)methyl group, and $R^5$ is a methyl group;
Hydrazide compounds, wherein in the formula (1-A), $R^3$ is a chlorine atom, $R^4$ is a (C1 to C6 alkoxy)methyl group, and $R^6$ is a hydrogen atom;
Hydrazide compounds, wherein in the formula (1-A), $R^3$ is a chlorine atom, $R^4$ is a (C1 to C6 alkoxy) methyl group, and $R^6$ is a methyl group;
Hydrazide compounds, wherein in the formula (1-A), $R^3$ is a chlorine atom, $R^4$ is a (C1 to C6 alkoxy) methyl group, $R^5$ is a hydrogen atom, and $R^6$ is a hydrogen atom;
Hydrazide compounds, wherein in the formula (1-A), $R^3$ is a chlorine atom, $R^4$ is a (C1 to C6 alkoxy) methyl group, $R^5$ is a methyl group, and $R^6$ is a hydrogen atom;
Hydrazide compounds, wherein in the formula (1-A), $R^3$ is a chlorine atom, $R^4$ is a (C1 to C6 alkoxy)methyl group, $R^5$ is a hydrogen atom, and $R^6$ is a methyl group; and
Hydrazide compounds, wherein in the formula (1-A), $R^3$ is a chlorine atom, $R^4$ is a (C1 to C6 alkoxy)methyl group, $R^5$ is a methyl group, and $R^6$ is a methyl group.
The following can be given as examples of embodiments of the pest control composition of the present invention.

Compositions containing one hydrazide compound selected from the group consisting of the present hydrazide compounds (1) to (63) shown in the production examples discussed below and imidacloprid;
Compositions containing one hydrazide compound selected from the group consisting of the present hydrazide compounds (1) to (63) and thiacloprid;
Compositions containing one hydrazide compound selected from the group consisting of the present hydrazide compounds (1) to (63) and acetamiprid;
Compositions containing one hydrazide compound selected from the group consisting of the present hydrazide compounds (1) to (63) and nitenpyram;
Compositions containing one hydrazide compound selected from the group consisting of the present hydrazide compounds (1) to (63) and clothianidin;
Compositions containing one hydrazide compound selected from the group consisting of the present hydrazide compounds (1) to (63) and thiamethoxam;
Compositions containing one hydrazide compound selected from the group consisting of the present hydrazide compounds (1) to (63) and imidacloprid in a weight ratio of hydrazide compound:imidacloprid = 1000:1 to 1:1000;
Compositions containing one hydrazide compound selected from the group consisting of the present hydrazide compounds (1) to (63) and thiacloprid in a weight ratio of hydrazide compound:thiacloprid = 1000:1 to 1:1000;
Compositions containing one hydrazide compound selected from the group consisting of the present hydrazide compounds (1) to (63) and acetamiprid in a weight ratio of hydrazide compound:acetamiprid = 1000:1 to 1:1000;
Compositions containing one hydrazide compound selected from the group consisting of the present hydrazide compounds (1) to (63) and nitenpyram in a weight ratio of hydrazide compound:nitenpyram = 1000:1 to 1:1000;
Compositions containing one hydrazide compound selected from the group consisting of the present hydrazide compounds (1) to (63) and clothianidin in a weight ratio of hydrazide compound:clothianidin = 1000:1 to 1:1000; and
Compositions containing one hydrazide compound selected from the group consisting of the present hydrazide compounds (1) to (63) and thiamethoxam in a weight ratio of hydrazide compound:thiamethoxam = 1000:1 to 1:1000.
The production of the present hydrazide compounds will be explained below.
Hereinafter, a compound shown by formula ($\alpha$) may be expressed as "compound ($\alpha$)".

(Production Method 1)

The present hydrazide compound can be produced by reacting a compound (2) with a compound (3).

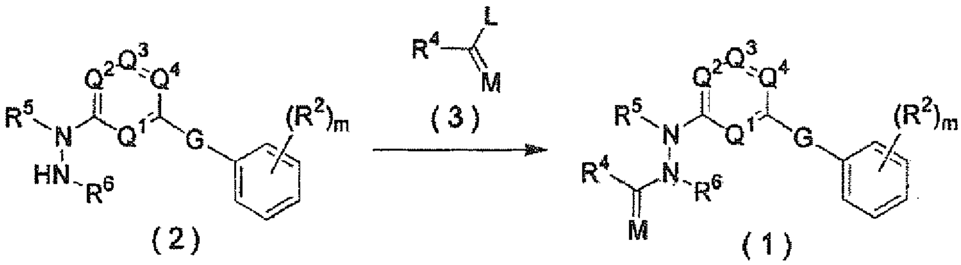

[In the formula, $Q^1$, $Q^2$ $Q^3$, $Q^4$, $R^2$, $R^4$, $R^5$, $R^6$, G, M, and m have the same meanings as above, and L represents a hydroxyl group or a chlorine atom.]

The reaction is generally carried out in a solvent.

Examples of the solvent used in the reaction include ethers such as tetrahydrofuran, diethyl ether, tert-butyl methyl ether, ethylene glycol dimethyl ether, and 1,4 4-dioxane; acid amides such as N,N-dimethylformamide; nitriles such as acetonitrile; aromatic hydrocarbons such as toluene and xylene; esters such as ethyl acetate; sulfoxides such as dimethyl sulfoxide; sulfolane; halogenated hydrocarbons such as 1,2-dichloroethane, chloroform, and chlorobenzene; and mixtures thereof.

When L is a chlorine atom, the reaction is generally carried out in the presence of a base.

Examples of the base used in the reaction include alkali metal hydrides such as sodium hydride; carbonates such as potassium carbonate; alkali metal alkoxides such as potassium tert-butoxide; and organic amines such as triethylamine and pyridine.

When L is a hydroxyl group, the reaction is carried out in the presence of a condensing agent.

Examples of the condensing agent used in the reaction include dicyclohexylcarbodiimide and 1-(3-dimethylaminopropyl)-3-ethylcarbodiimide hydrochloride.

The amounts of the reagents used in the reaction are generally a ratio of 1 to 10 mol of the compound (3) and a ratio of 1 to 10 mol of the base or the condensing agent, per mol of the compound (2).

The reaction temperature of this reaction is generally in a range from 0 to 100°C, and the reaction time is generally in a range from 0.5 to 24 hours.
After completion of the reaction, the reaction mixture is subjected to post-treatment procedures such as extraction with an organic solvent, drying and concentration, whereby the present hydrazide compound can be isolated. The isolated present hydrazide compound can also be further purified by chromatography, recrystallization, and the like.

(Production Method 2)

The present hydrazide compound can also be produced by reacting a compound (4) with a compound (5).

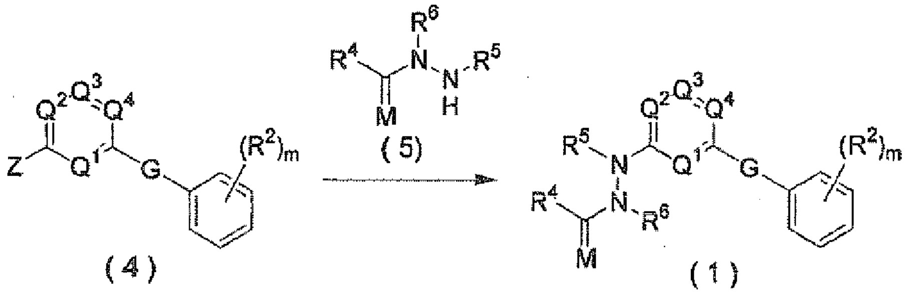

[In the formula, $Q^1$, $Q^2$, $Q^3$, $Q^4$, $R^2$, $R^4$, $R^5$, $R^6$, G, M, and m have the same meanings as above, and Z represents a leaving group such as a fluorine atom, a chlorine atom, a bromine atom, an iodine atom, a methanesulfonyloxy group, a p-toluenesulfonyloxy group, or a trifluoromethanesulfonyloxy group.]
The reaction is generally carried out in a solvent.
Examples of the solvent used in the reaction include ethers such as tetrahydrofuran, diethyl ether, tert-butyl methyl ether, ethylene glycol dimethyl ether, and 1, 4-dioxane; acid amides such as N,N-dimethylformamide; nitriles such as acetonitrile; aromatic hydrocarbons such as toluene and xylene; esters such as ethyl acetate; sulfoxides such as dimethyl sulfoxide; sulfolane; halogenated hydrocarbons such as 1, 2-dichloroethane, chloroform, and chlorobenzene; and mixtures thereof.
The reaction is carried out in the presence of a base if necessary.
Examples of the base used in the reaction include alkali metal hydrides such as sodium hydride; carbonates such as potassium carbonate; alkali metal alkoxides such as potassium tert-butoxide; and organic amines such as triethylamine and pyridine.
The amounts of the reagents used in the reaction are generally a ratio of 1 to 5 mol of a compound shown by a formula (5) and a ratio of 1 to 5 mol of the base, per mol of a compound shown by a formula (4).
The reaction temperature of this reaction is generally in a range from 0 to 100°C, and the reaction time is generally in a range from 0.1 to 24 hours.
After completion of the reaction, the reaction mixture is subjected to post-treatment procedures such as extraction with an organic solvent, drying and concentration, whereby the present hydrazide compound can be isolated. The isolated present hydrazide compound can also be further purified by chromatography, recrystallization, and the like.
This reaction can also be carried out by a coupling reaction using a common transition metal catalyst described in the literature, for example, in accordance with the reaction conditions described in Org. Lett., 3, 3803-3805 (2001).

(Production Method 3)

A compound (1-1) in which $R^5$ is a hydrogen atom, among the present hydrazide compounds, can also be produced by reacting a compound (1-2) in which $R^5$ is $R^{5-2}$, among the present hydrazide compounds, with an acid or a base.

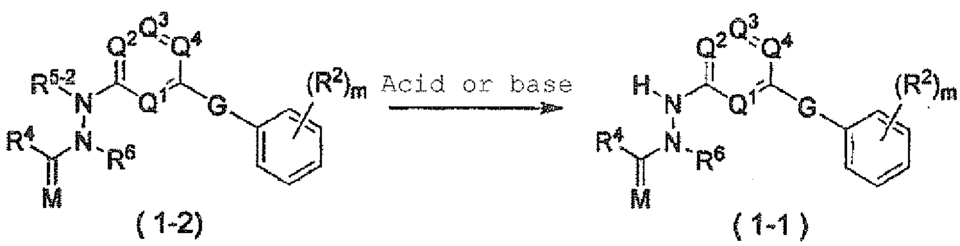

[In the formula, $Q^1$, $Q^2$, $Q^3$, $Q^4$, $R^2$, $R^4$, $R^6$, G, M, and m have the same meanings as above, and $R^{5-2}$ represents a benzoyl group optionally substituted by a group or groups selected from Group E2, a C2 to C6 alkylcarbonyl group, or a C2 to C6 alkoxycarbonyl group.]

The reaction can also be carried out using a solvent if necessary.

Examples of the solvent include water, ethers such as tetrahydrofuran, diethyl ether, tert-butyl methyl ether, ethylene glycol dimethyl ether, and 1, 4-dioxane; acid amides such as N,N-dimethylformamide; nitriles such as acetonitrile; aromatic hydrocarbons such as toluene and xylene; esters such as ethyl acetate; sulfoxides such as dimethyl sulfoxide; sulfolane; halogenated hydrocarbons such as 1,2-dichloroethane, chloroform, and chlorobenzene; alcohols such as methanol, ethanol, and n-propanol; and mixtures thereof.

Examples of the acid include organic acids such as acetic acid and trifluoroacetic acid; and inorganic acids such as hydrochloric acid, hydrobromic acid, and sulfuric acid.

Examples of the base include alkali metal hydrides such as sodium hydride; carbonates such as potassium carbonate; alkali metal alkoxides such as potassium tert-butoxide; and organic amines such as triethylamine and pyridine.

The above acid is generally used in an amount of 1 to 100 mol per mol of the compound (1-2).

The above base is generally used in an amount of 1 to 100 mol per mol of the compound (1-2).

The reaction temperature is generally in a range from 0 to 100°C, and the reaction time is generally in a range from 0.1 to 24 hours.

After completion of the reaction, the reaction mixture is subjected to post-treatment procedures such as extraction with an organic solvent, drying and concentration, whereby the present hydrazide compound can be isolated. The isolated present hydrazide compound can also be further purified by chromatography, recrystallization, and the like.

(Production Method 4)

Among the present hydrazide compounds, a compound (1-3), in which $R^4$ is an N ($R^9$) $R^{10}$ group and $R^{10}$ is a hydrogen atom, can also be produced by reacting a compound (2) with a compound (19).

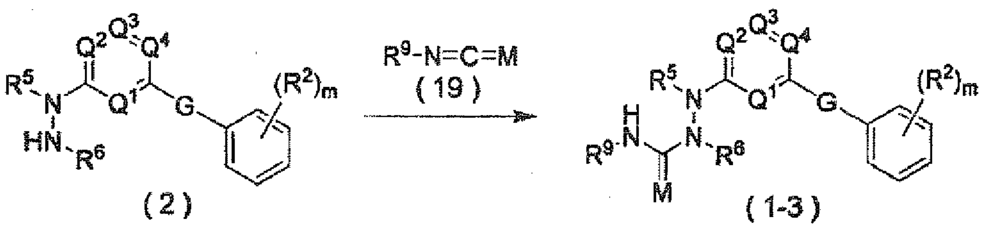


[In the formula, $Q^1$, $Q^2$, $Q^3$, $Q^4$, $R^2$, $R^5$, $R^6$, $R^9$, G, M, and m have the same meanings as above.]

The reaction is generally carried out in a solvent. It can also be carried out in the presence of a base if necessary.

Examples of the solvent include ethers such as tetrahydrofuran, diethyl ether, tert-butyl methyl ether, ethylene glycol dimethyl ether, and 1, 4-dioxane; acid amides such as N,N-dimethylformamide; nitriles such as acetonitrile; aromatic hydrocarbons such as toluene and xylene; esters such as ethyl acetate; sulfoxides such as dimethyl sulfoxide; sulfolane; halogenated hydrocarbons such as 1,2-dichloroethane, chloroform, and chlorobenzene; and mixtures thereof.

Examples of the above base include carbonates such as potassium carbonate and sodium carbonate; and organic amines such as triethylamine, pyridine, 4-(dimethylamino)pyridine, imidazole, and 1,8-diazabicyclo[5,4,0]-7-undecene.

In the above reaction, the compound (19) is generally used in an amount of 1 to mol per mol of the compound (2). When it is necessary to add the above base, the base is generally used in an amount of 1 to 5 mol per mol of the compound (2).

The reaction temperature is generally in a range from 0 to 100°C, and the reaction time is generally in a range from 0.1 to 24 hours.

After completion of the reaction, the reaction mixture is subjected to post-treatment procedures such as extraction with an organic solvent, drying and concentration, whereby the present Hydrazide compound can be isolated. The isolated present hydrazide compound can also be further purified by chromatography, recrystallization, and the like.

(Production Method 5)

Among the present hydrazide compounds, a compound (1-4) in which $R^4$ is $R^{4-1}$ and M is an oxygen atom, can be produced by reacting a compound (2) with a compound (21).

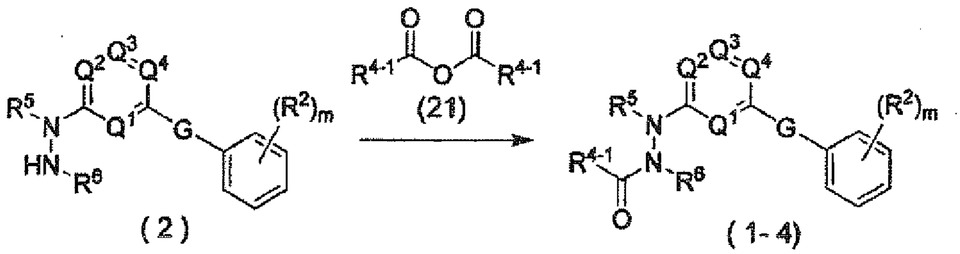

[In the formula, $Q^1$, $Q^2$, $Q^3$, $Q^4$, $R^2$, $R^5$, $R^6$, G, and m have the same meanings as above, $R^{4-1}$ represents a C1 to C12 chain hydrocarbon group optionally substituted by a group or groups selected from Group E1, a C3 to C12 cyclic hydrocarbon group optionally substituted by a group or groups selected from Group E2, a 5- to 6-membered heterocyclic group optionally substituted by a group or groups selected from Group E2, or an $OR^8$ group, and $R^8$ has the same meaning as above.]

The reaction can also be carried out using a solvent if necessary.

Examples of the solvent include ethers such as tetrahydrofuran, diethyl ether, tert-butyl methyl ether, ethylene glycol dimethyl ether, and 1, 4-dioxane; acid amides such as N,N-dimethylformamide; nitriles such as acetonitrile; aromatic hydrocarbons such as toluene and xylene; esters such as ethyl acetate; sulfoxides such as dimethyl sulfoxide; sulfolane; halogenated hydrocarbons such as 1,2-dichloroethane, chloroform, and chlorobenzene; and mixtures thereof.

The reaction can also be carried out in the presence of a base if necessary.

Examples of the base include alkali metal hydrides such as sodium hydride; carbonates such as potassium carbonate; alkali metal alkoxides such as potassium tert-butoxide; and organic bases such as triethylamine, pyridine, 4-(dimethylamino)pyridine, and imidazole.

In the above reaction, the compound (21) is generally used in an amount of 1 to 10 mol per mol of the compound (2) and can also be used as a solvent if necessary. When it is necessary to add the above base, the base is generally used in an amount of 1 to 10 mol per mol of the compound (2).

The reaction temperature is generally in a range from 0 to 100°C, and the reaction time is generally in a range from 0.5 to 24 hours.

After completion of the reaction, the reaction mixture is subjected to post-treatment procedures such as extraction with an organic solvent, drying and concentration, whereby the composition (1-4) can also be isolated. The isolated compound (1-4) can also be further purified by chromatography, recrystallization, and the like.

Next, process for producing intermediates used for the preparation of the present hydrazide compounds will be explained.

(Reference Production Method 1)

Among compounds (2), a compound (2-1) in which $R^6$ is a hydrogen atom, can be produced by reacting a compound (6) and a nitrite compound (7), and then reacting with a reducing agent (8).

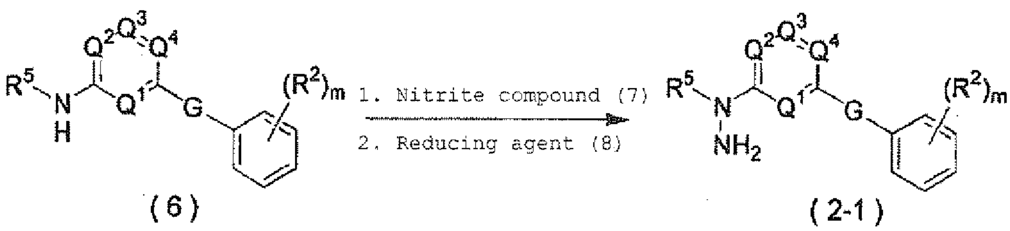


[In the formula, $Q^1$, $Q^2$, $Q^3$, $Q^4$, $R^2$, $R^5$, G, and m have the same meanings as above.]

The reaction is generally carried out in a solvent.

Examples of the solvent used in the reaction include water, ethers such as tetrahydrofuran, diethyl ether, tert-butyl methyl ether, ethylene glycol dimethyl ether, and 1,4-dioxane; acid amides such as N,N-dimethylformamide; aromatic hydrocarbons such as toluene and xylene; sulfoxides such as dimethyl sulfoxide; sulfolane; halogenated hydrocarbons such as 1,2-dichloroethane, chloroform, and chlorobenzene; and mixtures thereof.

Examples of the nitrite compound (7) used in the reaction include nitrite salts such as sodium nitrite and nitrite esters such as ethyl nitrite.

Examples of the reducing agent (8) used in the reaction include sulfites such as sodium sulfite; metals such

as zinc; tin(II) chloride; and the like.

The amounts of the reagents used in the reaction are generally a ratio of 1 to 10 mol of the nitrite compound (7) and a ratio of 1 to 10 mol of the reducing agent (8), per mol of a compound shown by a formula (6).

The reaction temperature in the step of reacting the compound shown by the formula (6) with the nitrite compound (7) in the reaction is generally in a range from -20 to 30°C, and the reaction time is generally in a range from 0.5 to 24 hours.

The reaction mixture obtained in the step of reacting the compound (6) with the nitrite compound (7) can be directly used in the step of reacting with the reducing agent (8). The reaction temperature in that step is generally in a range from -20 to 50 °C, and the reaction time is generally in a range up to 24 hours.

After completion of the reaction, the reaction mixture is subjected to post-treatment procedures such as extraction with an organic solvent, drying and concentration, whereby the compound (2-1) can also be isolated. The isolated compound (2-1) can also be further purified by chromatography, recrystallization, and the like.

(Reference Production Method 2)

Among compounds (2), a compound (2-1) in which $R^6$ is a hydrogen atom, can also be produced by reacting a compound (6) with an aminating agent (9).

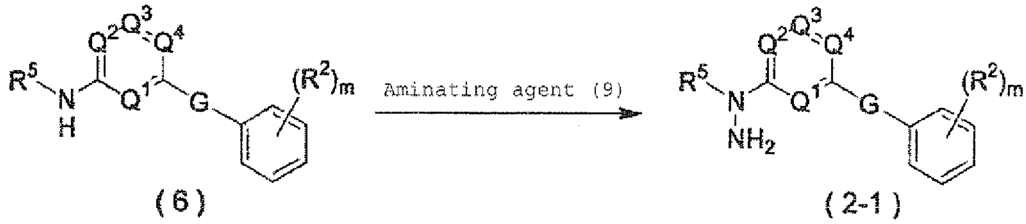


(6) (2-1)

[In the formula, $Q^1$, $Q^2$, $Q^3$, $Q^4$, $R^2$, $R^5$, G, and m have the same meanings as above.]

The reaction is generally carried out in a solvent.

Examples of the solvent used in the reaction include water, ethers such as tetrahydrofuran, diethyl ether, tert-butyl methyl ether, ethylene glycol dimethyl ether, and 1,4-dioxane; acid amides such as N, N-dimethylformamide; aromatic hydrocarbons such as toluene and xylene; sulfoxides such as dimethyl sulfoxide; sulfolane; halogenated hydrocarbons such as 1,2-dichloroethane, chloroform, and chlorobenzene; and mixtures thereof.

The reaction is generally carried out in the presence of a base.

Examples of the base used in the reaction include alkali metal hydrides such as sodium hydride; carbonates such as potassium carbonate; alkali metal alkoxides such as potassium tert-butoxide; metal hydroxides such as sodium hydroxide; and organic amines such as triethylamine and pyridine.

Examples of the aminating agent (9) used in the reaction include chloramines such as chloramine; O-acylhydroxylamines such as O-mesitoylhydroxylamine; O-sulfonylhydroxylamines; and hydroxylamine-O-sulfonic acid.

The above aminating agent (9) can also be generated in the reaction system in the reaction. For example, when chloramine is used as the above aminating agent (9), chloramine may be generated by using sodium hypochlorite and ammonia as raw materials and mixing them in the reaction system.

The amounts of the reagents used in the reaction are generally a ratio of 1 to 10 mol of the aminating agent (9) and a ratio of 1 to 10 mol of the base, per mol of a compound shown by a formula (6).

The reaction temperature of this reaction is generally in a range from 0 to 100°C, and the reaction time is generally in a range from 0.5 to 24 hours.

After completion of the reaction, the reaction mixture is subjected to post-treatment procedures such as extraction with an organic solvent, drying and concentration, whereby the compound (2-1) can also be isolated. The isolated compound (2-1) can also be further purified by chromatography, recrystallization, and the like.

(Reference Production Method 3)

Among compounds (2), a compound (2-2) in which $R^6$ is $R^{6-1}$, can be produced by reacting a compound (2-1) with a compound (10).

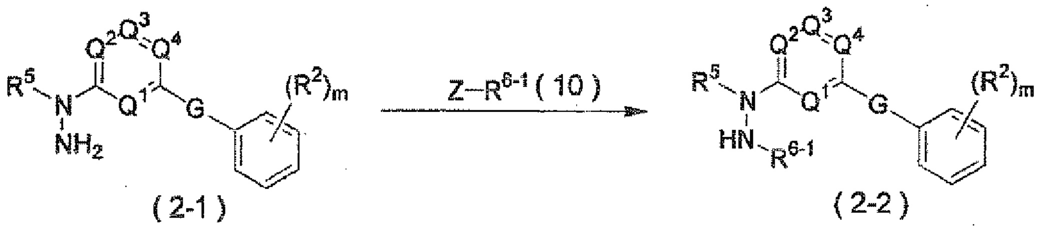

(2-1) (2-2)

[In the formula, $Q^1$, $Q^2$, $Q^3$, $Q^4$, $R^2$, $R^5$, G, Z, and m have the same meanings as above, and $R^{6-1}$ represents a C1 to C12 chain hydrocarbon group optionally substituted by a group or groups selected from Group E1, a benzoyl group optionally substituted by a group or groups selected from Group E2, a C2 to C6 alkylcarbonyl group, a C2 to C6 alkoxycarbonyl group, a C3 to C12 cycloalkyl group, or a formyl group.]

The reaction is generally carried out in a solvent.

Examples of the solvent used in the reaction include ethers such as tetrahydrofuran, diethyl ether, tert-butyl methyl ether, ethylene glycol dimethyl ether, and 1, 4-dioxane; acid amides such as N,N-dimethylformamide; nitriles such as acetonitrile; aromatic hydrocarbons such as toluene and xylene; esters such as ethyl acetate; sulfoxides such as dimethyl sulfoxide; sulfolane; halogenated hydrocarbons such as 1,2-dichloroethane, chloroform, and chlorobenzene; and mixtures thereof.

The reaction is generally carried out in the presence of a base.

Examples of the base used in the reaction include alkali metal hydrides such as sodium hydride; carbonates such as potassium carbonate; alkali metal alkoxides such as potassium tert-butoxide; and organic amines such as triethylamine and pyridine.

The amounts of the reagents used in the reaction are generally a ratio of 1 to 10 mol of the compound (10) and a ratio of 1 to 10 mol of the base, per mol of a compound shown by a formula (2-1).

The reaction temperature of this reaction is generally in a range from 0 to 100°C, and the reaction time is generally in a range from 0.5 to 24 hours.

After completion of the reaction, the reaction mixture is subjected to post-treatment procedures such as extraction with an organic solvent, drying and concentration, whereby the compound (2-2) can also be isolated. The isolated compound (2-2) can also be further purified by chromatography, recrystallization, and the like.

(Reference Production Method 4)

Among compounds (2), a compound (2-3) in which $R^6$ is $R^{6-2}$, can be produced by reacting a compound (2-1) with a compound (22).

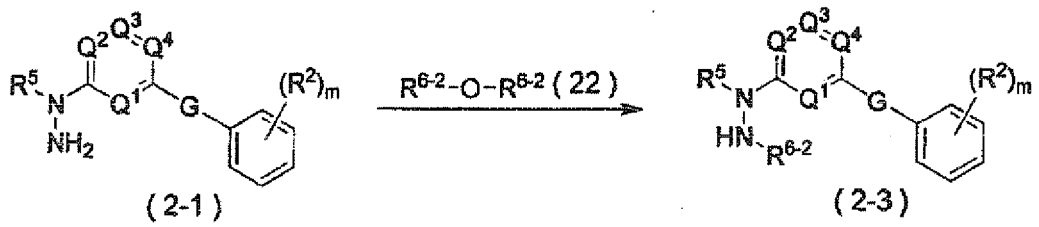

(2-1) (2-3)

[In the formula, $Q^1$, $Q^2$, $Q^3$, $Q^4$, $R^2$, $R^5$, G, and m have the same meanings as above, and $R^{6-2}$ represents a benzoyl group optionally substituted by a group or groups selected from Group E2, a C2 to C6 alkylcarbonyl group, or a C2 to C6 alkoxycarbonyl group.]

The reaction can also be carried out using a solvent if necessary.

Examples of the solvent include ethers such as tetrahydrofuran, diethyl ether, tert-butyl methyl ether, ethylene glycol dimethyl ether, and 1, 4-dioxane; acid amides such as N,N-dimethylformamide; nitriles such as acetonitrile; aromatic hydrocarbons such as toluene and xylene; esters such as ethyl acetate; sulfoxides such as dimethyl sulfoxide; sulfolane; halogenated hydrocarbons such as 1,2-dichloroethane, chloroform, and chlorobenzene; and mixtures thereof.

The reaction can also be carried out in the presence of a base if necessary.

Examples of the base include alkali metal hydrides such as sodium hydride; carbonates such as potassium carbonate; alkali metal alkoxides such as potassium tert-butoxide; and organic bases such as triethylamine, pyridine, 4-(dimethylamino)pyridine, and imidazole.

In the above reaction, the compound (22) is generally used in an amount of 1 to 10 mol per mol of the compound

(2-1). If necessary, the compound (22) can also be used as a solvent.

When it is necessary to add the above base, the base is generally used in an amount of 1 to 10 mol per mol of the compound (2-1).

The reaction temperature is generally in a range from 0 to 100°C, and the reaction time is generally in a range from 0.5 to 24 hours.

After completion of the reaction, the reaction mixture is subjected to post-treatment procedures such as extraction with an organic solvent, drying and concentration, whereby the compound (2-3) can also be isolated. The isolated compound (2-3) can also be further purified by chromatography, recrystallization, and the like.

(Reference Production Method 5)

Among compounds (6), a compound (6-1) in which $R^5$ is a hydrogen atom, can be produced by reducing a compound (11) by any of the following methods (i) to (iii).

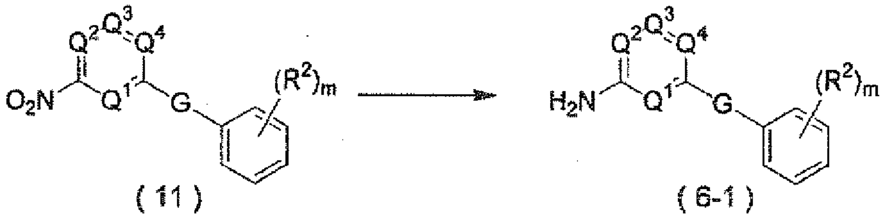

(11) (6-1)

[In the formula, $Q^1$, $Q^2$, $Q^3$, $Q^4$, $R^2$, G, and m have the same meanings as above.]

(i) Method of reacting with hydrogen gas in the presence of a transition metal catalyst.
The reaction is carried out in a solvent.
Examples of the solvent used in the reaction include esters such as ethyl acetate; alcohols such as ethanol and methanol; water, acetic acid, hydrochloric acid, and mixtures thereof.
Examples of the transition metal catalyst used in the reaction include Raney nickel, palladium-carbon, platinum dioxide, and the like.
The amount of the transition metal catalyst used in the reaction is generally a ratio of 0.01 to 0.5 mol per mol of the compound (11).
The amount of the hydrogen gas is generally a ratio of 1 to 100 mol per mol of the compound (11).
The reaction temperature of this reaction is generally in a range from 0 to 80°C, and the reaction time is generally in a range from 0.1 to 24 hours.
After completion of the reaction, the reaction mixture is filtered and subjected to post-treatment procedures such as extraction with an organic solvent, drying and concentration, as needed, whereby a compound shown by a formula (6-1) can be isolated. The isolated compound shown by the formula (6-1) can also be further purified by chromatography, recrystallization, and the like.
(ii) Method of reacting with hydrazine in the presence of a base.
The reaction is carried out in a solvent.
Examples of the solvent used in the reaction include ethers such as diethylene glycol and triethylene glycol; water, and mixtures thereof.
Examples of the base used in the reaction include alkali metal hydroxides such as potassium hydroxide.
Examples of the hydrazine used in the reaction include hydrazine hydrate.
The amounts of the reagents used in the reaction are generally a ratio of 1 to 10 mol of the base and a ratio of 1 to 10 mol of the hydrazine, per mol of the compound (11).
The reaction temperature of this reaction is generally in a range from 0 to 100°C, and the reaction time is generally in a range from 0.5 to 24 hours.
After completion of the reaction, the reaction mixture is subjected to post-treatment procedures such as extraction with an organic solvent, drying and concentration, whereby a compound shown by a formula (6-1) can be isolated. The isolated compound shown by the formula (6-1) can also be further purified by chromatography, recrystallization, and the like.
(iii) Method of reacting with a metal in the presence of an acid.
The reaction is generally carried out in a solvent.
Examples of the solvent used in the reaction include alcohols such as ethanol; water, and mixtures thereof.
Examples of the metal used in the reaction include iron, tin, and tin (II) chloride.

Examples of the acid used in the reaction include acetic acid, hydrochloric acid, and sulfuric acid.
The amounts of the reagents used in the reaction are generally a ratio of 2 to 20 mol of the metal and a ratio of 0.1 to 10 mol of the acid, per mol of the compound (11).
The reaction temperature of this reaction is generally in a range from 0 to 100°C, and the reaction time is generally in a range from 0.5 to 12 hours.
After completion of the reaction, the reaction mixture is filtered, and subjected to post-treatment procedures such as extraction with an organic solvent, drying and concentration, as needed, whereby a compound shown by a formula (6-1) can be isolated. The isolated compound shown by the formula (6-1) can also be further purified by chromatography, recrystallization, and the like.

(Reference Production Method 6)

Among compounds (6), a compound (6-2) in which $R^5$ is $R^{5-1}$, can be produced by reacting a compound (6-1) with a compound (12).

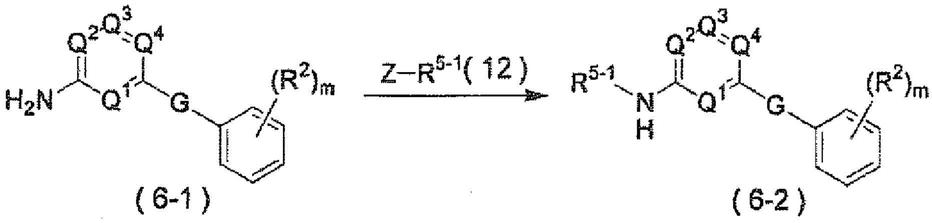


(6-1) (6-2)

[In the formula, $Q^1$, $Q^2$, $Q^3$, $Q^4$, $R^2$, G, Z, and m have the same meanings as above, and $R^{5-1}$ represents a C1 to C12 chain hydrocarbon group optionally substituted by a group or groups selected from Group E1, a benzoyl group optionally substituted by a group or groups selected from Group E2, a C2 to C6 alkylcarbonyl group, a C2 to C6 alkoxycarbonyl group, a C3 to C12 cycloalkyl group, or a formyl group.]
The reaction is generally carried out in a solvent.
Examples of the solvent used in the reaction include ethers such as tetrahydrofuran, diethyl ether, tert-butyl methyl ether, ethylene glycol dimethyl ether, and 1,4-dioxane; acid amides such as N,N-dimethylformamide; nitriles such as acetonitrile; aromatic hydrocarbons such as toluene and xylene; esters such as ethyl acetate; sulfoxides such as dimethyl sulfoxide; sulfolane; halogenated hydrocarbons such as 1,2-dichloroethane, chloroform, and chlorobenzene; and mixtures thereof.
The reaction is generally carried out in the presence of a base.
Examples of the base used in the reaction include alkali metal hydrides such as sodium hydride; carbonates such as potassium carbonate; alkali metal alkoxides such as potassium tert-butoxide; and organic amines such as triethylamine and pyridine.
The amounts of the reagents used in the reaction are generally a ratio of 1 to 10 mol of the compound (12) and a ratio of 1 to 10 mol of the base, per mol of the compound (6-1).
The reaction temperature of this reaction is generally in a range from 0 to 100°C, and the reaction time is generally in a range from 0.5 to 24 hours.
After completion of the reaction, the reaction mixture is subjected to post-treatment procedures such as extraction with an organic solvent, drying and concentration, whereby the compound (6-2) can also be isolated. The isolated compound (6-2) can also be further purified by chromatography, recrystallization, and the like.

(Reference Production Method 7)

Among compounds (6), a compound (6-3) in which $R^5$ is $R^{5-2}$, can be produced by reacting a compound (6-1) with a compound (20).

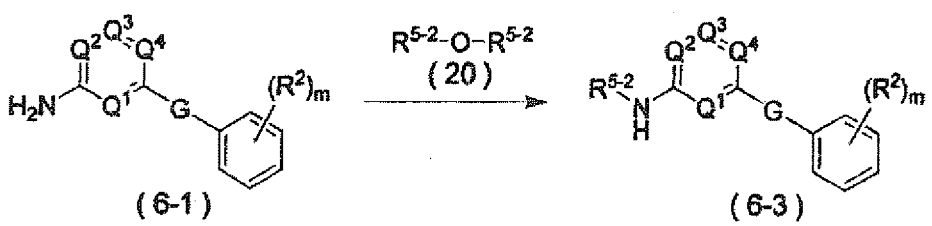


(6-1) (6-3)

[In the formula, $Q^1$, $Q^2$, $Q^3$, $Q^4$, $R^2$, $R^{5-2}$ , G, and m have the same meanings as above.]

The reaction can also be carried out using a solvent if necessary.

Examples of the solvent include ethers such as tetrahydrofuran, diethyl ether, tert-butyl methyl ether, ethylene glycol dimethyl ether, and 1, 4-dioxane; acid amides such as N,N-dimethylformamide; nitriles such as acetonitrile; aromatic hydrocarbons such as toluene and xylene; esters such as ethyl acetate; sulfoxides such as dimethyl sulfoxide; sulfolane; halogenated hydrocarbons such as 1,2-dichloroethane, chloroform, and chlorobenzene; and mixtures thereof.

The reaction can also be carried out in the presence of a base if necessary.

Examples of the base include alkali metal hydrides such as sodium hydride; carbonates such as potassium carbonate; alkali metal alkoxides such as potassium tert-butoxide; and organic bases such as triethylamine, pyridine, 4-(dimethylamino)pyridine, and imidazole.

In the above reaction, the compound (20) is generally used in an amount of 1 to 10 mol per mol of the compound (6-1) and can also be used as a solvent if necessary. When it is necessary to add the above base, the base is generally used in an amount of 1 to 10 mol per mol of the compound (6-1).

The reaction temperature is generally in a range from 0 to 100°C, and the reaction time is generally in a range from 0.5 to 24 hours.

After completion of the reaction, the reaction mixture is subjected to post-treatment procedures such as extraction with an organic solvent, drying and concentration, whereby the compound (6-3) can also be isolated. The isolated compound (6-3) can also be further purified by chromatography, recrystallization, and the like.

(Reference Production Method 8)

Among compounds (11), a compound (11-1) in which G is a group shown by G-1 and $Y^1$ is an oxygen atom, can be produced by reacting a compound (13) with a base and then reacting with a compound (14).

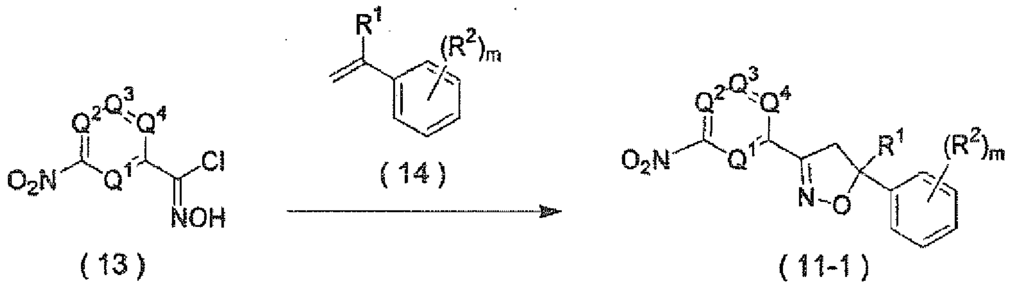


[In the formula, $Q^1$, $Q^2$, $Q^3$, $Q^4$, $R^1$, $R^2$, and m have the same meanings as above.]

The reaction is generally carried out in a solvent.

Examples of the solvent used in the reaction include ethers such as tetrahydrofuran, diethyl ether, tert-butyl methyl ether, ethylene glycol dimethyl ether, and 1, 4-dioxane; acid amides such as N,N-dimethylformamide; nitriles such as acetonitrile; hydrocarbons such as toluene; esters such as ethyl acetate; sulfoxides such as dimethyl sulfoxide; and mixtures thereof.

Examples of the base used in the reaction include alkali metal hydrides such as sodium hydride; carbonates such as potassium carbonate; alkali metal alkoxides such as potassium tert-butoxide; and organic amines such as triethylamine and pyridine.

The amounts of the reagents used in the reaction are generally a ratio of 1 to 10 mol of the compound (14) and a ratio of 1 to 10 mol of the base, per mol of the compound (13).

The reaction temperature in the step of reacting the compound (13) with the base in the reaction is generally in a range from 0 to 80°C, and the reaction time is generally in a range from 0.5 to 24 hours.

The reaction mixture obtained by the step of reacting the compound (13) with the base can be directly used in the step of reacting with the compound (14). The reaction temperature in that step is generally in a range from 0 to 80°C, and the reaction time is generally in a range from 0.5 to 24 hours.

After completion of the reaction, the reaction mixture is subjected to post-treatment procedures such as extraction with an organic solvent, drying and concentration, whereby the compound (11-1) can be isolated. The isolated compound (11-1) can also be further purified by chromatography, recrystallization, and the like.

(Reference Production Method 9)

A compound (13) can be produced by reacting a compound (15) with a chlorinating agent (16).

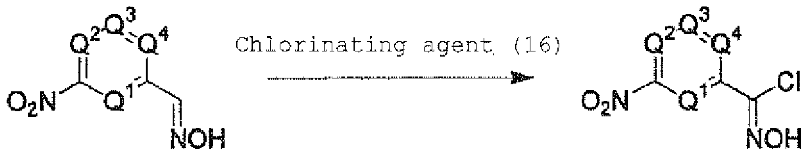


(15) (13)

[In the formula, $Q^1$, $Q^2$, $Q^3$, and $Q^4$ have the same meanings as above.]

The reaction is generally carried out in a solvent.

Examples of the solvent used in the reaction include ethers such as tetrahydrofuran, diethyl ether, tert-butyl methyl ether, ethylene glycol dimethyl ether, and 1,4-dioxane; hydrocarbons such as toluene; esters such as ethyl acetate; acid amides such as N,N-dimethylformamide; nitriles such as acetonitrile; sulfoxides such as dimethyl sulfoxide; and mixtures thereof.

Examples of the chlorinating agent (16) used in the reaction include chlorine gas and N-chlorosuccinimide.

The amount of the reagent used in the reaction is generally a ratio of 1 to 10 mol of the chlorinating agent (16) per mol of the compound (15).

The reaction temperature of this reaction is generally in a range from -20 to 80°C, and the reaction time is generally in a range from 0.5 to 24 hours.

After completion of the reaction, the reaction mixture is subjected to post-treatment procedures such as extraction with an organic solvent, drying and concentration, whereby the compound (13) can also be isolated. The isolated compound (13) can also be further purified by chromatography, recrystallization, and the like.

(Reference Production Method 10)

A compound (15) can be produced by reacting a compound (17) with hydroxylamine.

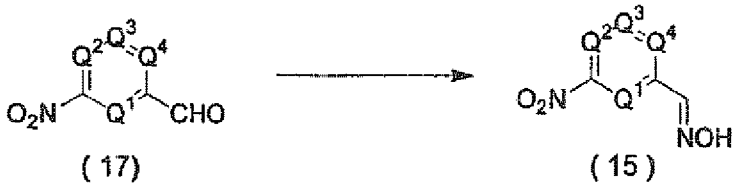

(17) (15)

[In the formula, $Q^1$, $Q^2$, $Q^3$, and $Q^4$ have the same meanings as above.]

The reaction is generally carried out in a solvent.

Examples of the solvent used in the reaction include ethers such as tetrahydrofuran, diethyl ether, tert-butyl methyl ether, ethylene glycol dimethyl ether, and 1,4-dioxane; hydrocarbons such as toluene; esters such as ethyl acetate; acid amides such as N, N-dimethylformamide; alcohols such as ethanol and methanol; nitrides such as acetonitrile; sulfoxides such as dimethyl sulfoxide; water; and mixtures thereof.

Examples of the hydroxylamine used in this reaction include hydroxylamines having a form of a salt of hydroxylamine and being a mineral acid salt such as hydroxylamine hydrochloride and hydroxylamine sulfate, which can produce hydroxylamine in the reaction system. The reaction is carried out in the presence of a base in such cases. Examples of the base used in such cases include organic amines such as triethylamine; carbonates such as sodium carbonate; and alkali metal hydroxides such as sodium hydroxide.

The amount of the reagent used in the reaction is generally a ratio of 1 to 10 mol of the hydroxylamine per mol of the compound (17). When the salt of hydroxylamine with a mineral acid is used, the amount of the base used is generally a ratio of 1 to 10 mol per mol of the salt of hydroxylamine with a mineral acid.

The reaction temperature of this reaction is generally in a range from 0 to 80°C, and the reaction time is generally in a range from 0.5 to 24 hours.

After completion of the reaction, the reaction mixture is subjected to post-treatment procedures such as extraction with an organic solvent, drying and concentration, whereby the compound (15) can be isolated. The isolated compound (15) can also be further purified by chromatography, recrystallization, and the like.

(Reference Production Method 11)

Among compounds (4), a compound (4-1) in which G is a group shown by G-1 and $Y^1$ is an oxygen atom, can

be produced by reacting a compound (18) with a base and then reacting with a compound (14).

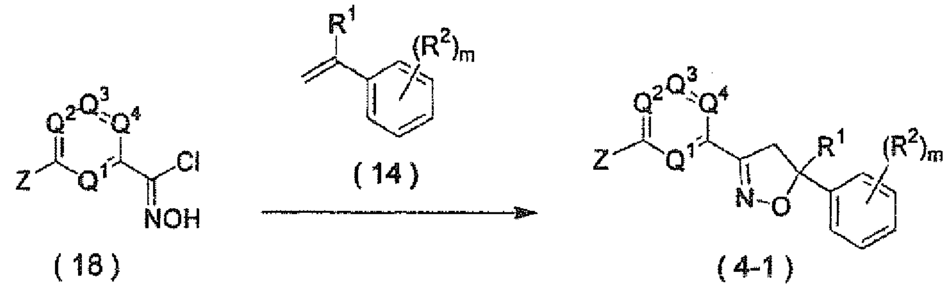

[In the formula, $Q^1$, $Q^2$, $Q^3$, $Q^4$, $R^1$, $R^2$, Z, and m have the same meanings as above.]

The reaction is generally carried out in a solvent.

Examples of the solvent used in the reaction include ethers such as tetrahydrofuran, diethyl ether, tert-butyl methyl ether, ethylene glycol dimethyl ether, and 1, 4-dioxane; acid amides such as N,N-dimethylformamide; nitriles such as acetonitrile; hydrocarbons such as toluene; esters such as ethyl acetate; sulfoxides such as dimethyl sulfoxide; and mixtures thereof.

Examples of the base used in the reaction include alkali metal hydrides such as sodium hydride; carbonates such as potassium carbonate; alkali metal alkoxides such as potassium tert-butoxide; and organic amines such as triethylamine and pyridine.

The amounts of the reagents used in the reaction is generally a ratio of 1 to 10 mol of the compound (14) and a ratio of 1 to 10 mol of the base, per mol of the compound (18).

The reaction temperature in the step of this reaction that reacts the compound (18) and the base is generally in a range from 0 to 80°C, and the reaction time is generally in a range from 0.5 to 24 hours.

The reaction mixture obtained by the step of reacting the compound (18) and the base can be directly used in the step of reacting with the compound (14). The reaction temperature in that step is generally in a range from 0 to 80 °C, and the reaction time is generally in a range from 0.5 to 24 hours.

After completion of the reaction, the reaction mixture is subjected to post-treatment procedures such as extraction with an organic solvent, drying and concentration, whereby the compound (4-1) can be isolated. The isolated compound (4-1) can also be further purified by chromatography, recrystallization, and the like.

(Reference Production Method 12)

Among compounds (4), a compound shown by a formula (4-2) in which G is a group shown by G-1 and $Y^1$ is an $NR^7$ group, can be produced in accordance with the method described, for example, in WO 2007/123855.

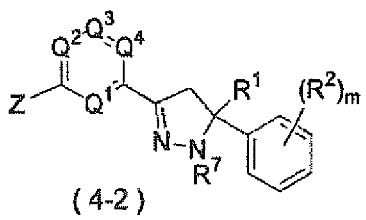

(4-2)

[In the formula, $Q^1$, $Q^2$, $Q^3$, $Q^4$ $R^1$, $R^2$, $R^7$, Z, and m have the same meanings as above.]

(Reference Production Method 13)

Among compounds (4), a compound shown by a formula (4-3) in which G is a group shown by G-2 and $Y^2$ is $Y^{2-1}$, can be produced in accordance with the method described, for example, in JP-A-2007-91708.

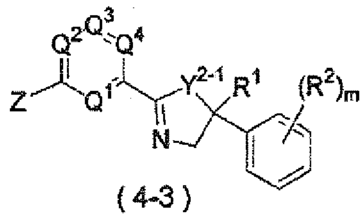

( 4-3 )

[In the formula, $Q^1$, $Q^2$, $Q^3$, $Q^4$, $R^1$, $R^2$, Z, and m have the same meanings as above, and $Y^{2-1}$ represents an oxygen atom, a sulfur atom, or a methylene group.]

(Reference Production Method 14)

Among compounds (4), a compound shown by a formula (4-4) in which G is a group shown by G-3 and $Y^3$ is $Y^{3-1}$, can be produced in accordance with the method described, for example, in WO 2007/123853.

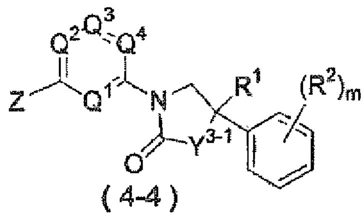

( 4-4 )

[In the formula, $Q^1$, $Q^2$, $Q^3$, $Q^4$, $R^1$, $R^2$, Z, and m have the same meanings as above, and $Y^{3-1}$ represents an oxygen atom or $NR^7$, and $R^7$ has the same meaning as above. ]

(Reference Production Method 15)

Among compounds (4), a compound shown by a formula (4-5) in which G is a group shown by G-3 and $Y^3$ is a methylene group, can be produced in accordance with the method described, for example, in JP-A-2008-110971.

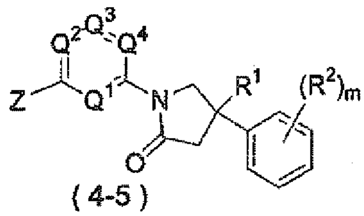

( 4-5 )

[In the formula, $Q^1$, $Q^2$, $Q^3$, $Q^4$, $R^1$, $R^2$, Z, and m have the same meanings as above.]

The present neonicotinoid compound is a compound selected from a group consisting of imidacloprid, thiacloprid, acetamiprid, nitenpyram, clothianidin, and thiamethoxam. Imidacloprid, is a compound shown by the following formula (IIa), thiacloprid is a compound shown by the following formula (IIb), acetamiprid is a compound shown by the following formula (IIc), nitenpyram is a compound shown by the following formula (IId), clothianidin is a compound shown by the following formula (IIe), and thiamethoxam is a compound shown by the following formula (IIf).

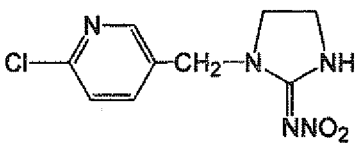

(IIa)

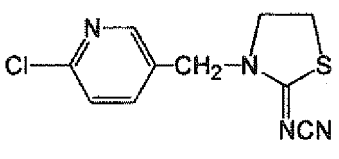

(IIb)

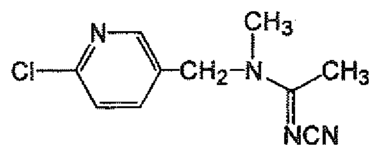

(IIc)

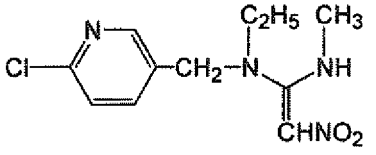

(IId)

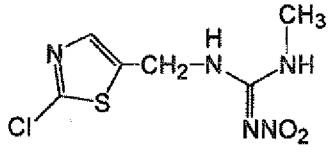

(IIe)

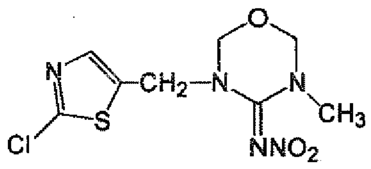

(IIe)

The present neonicotinoid compound can be produced by a method described in JP-A-07-278094, JP-A-02-209868, JP-A-01-070467, JP-A-03-218354, JP-A-04-273863, and the like. In addition, commercially available compounds can also be used.

Examples of pests against which the pest control composition of the present invention exhibits an effect include noxious arthropods such as insect pests and acarine pests. More specifically, examples thereof are as follows.

Hemiptera pests: Delphacidae such as *Laodelphax striatellus, Nilaparvata lugens, Sogatella furcifera;* Deltocephalidae such as *Nephotettix cincticeps, Nephotettix virescens, Empoasca onukii;* Aphididae such as *Aphis gossypii, Myzus persicae, Brevicoryne brassicae, Aphis spiraecola, Macrosiphum euphorbiae, Aulacorthum solani, Rhopalosiphum padi, Toxoptera citricidus, Hyalopterus pruni;* Pentatomidae such as *Nezara antennata*, *Riptortus clavetus, Leptocorisa chinensis, Eysarcoris parvus, Halyomorpha mista;* Reduviidae such as *Triatoma rubrofasciata;* Aleyrodidae such as *Trialeurodes vaporariorum, Bemisia tabaci, Dialeurodes citri, Aleurocanthus spiniferus;* Coccoidae such as *Aonidiella aurantii, Comstockaspis perniciosa, Unaspis citri, Ceroplastes rubens, Icerya purchasi, Planococcus kraunhiae, Planococcus citri, Pseudococcus longispinis, Pseudaulacaspis pentagona;* Tingidae; Cimicidae such as *Cimex lectularius, Cimex hemipterus;* Psyllidae such as *Cacopsylla pyricola,* etc.

Lepidoptera pests: Pyralidae such as *Chilo suppressalis, Tryporyza incertulas, Cnaphalocrocis medinalis, Notarcha derogata, Plodia interpunctella, Ostrinia furnacalis, Hellula undalis, Pediasia teterrellus;* Noctuidae such as *Spodoptera litura*, *Spodoptera exigua, Pseudaletia separata, Namestra brassicae, Agrotis ipsilon, Autographa nigrisigna, Trichoplusia spp.* , *Heliothis spp.,* and *Helicoverpa spp.* ; Pieridae such as *Pieris rapae;* Tortricidae such as *Adoxophyes spp.,*

*Grapholita molesta, Leguminivora glycinivorella, Matsumuraeses azukivora, Adoxophyes orana fasciata, Adoxophyes honmai., Homona magnanima, Archips fuscocupreanus, Cydia pomonella;* Gracillariidae such as *Caloptilia theivora, Phyllonorycter ringoneella*; Carposinidae such as *Carposina niponensis;* Lyonetiidae such as *Tuta absoluta, Lyonetia spp.* ; Lymantriidae such as *Lymantria spp., Euproctis spp.;* Yponomeutidae such as *Plutella xylostella;* Gelechiidae such as *Pectinophora gossypiella, Phthorimaea operculella;* Arctiidae such as *Hyphantria cunea;* Tineidae such as *Tinea translucens, Tineola bisselliella,* etc.

Thysanoptera pests: Thripidae such as *Frankliniella occidentalis, Thrips parmi, Scirtothrips dorsalis, Thrips tabaci, Frankliniella intonsa,* etc.

Diptera pests: Culex such as *Culex pipiens pallens, Culex tritaeniorhynchus, Culex quinquefasciatus; Aedes spp.* such as *Aedes aegypti, Aedes albopictus; Anopheles spp.* such as *Anopheles sinensis, Anopheles gambiae, Anopheles minimus, Anopheles stephensi, Anopheles albimanus;* Chironomidae; Muscidae such as *Musca domestica, Muscina stabulans*; Calliphoridae; Sarcophagidae; *Fannia canicularis;* Hippoboscidae such as *Glossina palpalis;* Anthomyiidae such as *Delia platura*, *Delia antiqua;* Agromyzidae such as *Agromyza oryzae, Hydrellia griseola, Liriomyza salivae, Liriomyza trifolii, Chromatomyia horticola;* Chloropidae such as *Chlorops oryzae;* Tephritidae such as *Dacus cucurbitae*, *Ceratitis capitata;* Drosophilidae; Phoridae such as *Megaselia spiracularis;* Psychodidae such as *Clogmia albipunctata, Phlebotomus squamirostris, Lutzomyia longipalpis;* Simuliidae; Tabanidae such as *Tabanus trigonus;* Culicoides, etc.

Coleoptera pests: corn rootworms such as *Diabrotica virgifera virgifera, Diabrotica undecimpunctata howardi;* Scarabaeidae such as *Anomala cuprea, Anomala rufocuprea, Popillia japonica;* Curculionidae such as *Sitophilus zeamais, Lissorhoptrus oryzophilus, Callosobruchuys chienensis, Echinocnemus squameus, Anthonomus grandis, Sphenophorus venatus, Rhynchophorus ferrugineus;* Tenebrionidae such as *Tenebrio molitor, Tribolium castaneum;* Chrysomelidae such as *Oulema oryzae, Aulacophora femoralis, Phyllotreta striolata, Leptinotarsa decemlineata;* Dermestidae such as *Anthrenus verbasci, Dermestes maculates;* Anobiidae such as *Lasioderma serricorne;* Epilachna such as *Epilachna vigintioctopunctata;* Limnoridae such as *Lyctus brunneus, Tomicus piniperda ;* Bostrychidae; Ptinidae ; Cerambycidae such as *Anoplophora malasiaca; Agriotes spp.* , *Paederus fuscipes,* etc.

Orthoptera pests: *Locusta migratoria, Gryllotalpa africana, Oxya yezoensis, Oxya japonica,* Gryllidae, etc.

Siphonaptera pests: *Pulex irritans, Ctenocephalides felis, Ctenocephalides canis, Xenopsylla cheopis, Tunga penetrans, Echidnophaga gallinacea, Nosopsyllus fasciatus,* etc.

Anoplura pests: *Pediculus humanus corporis*, *Pediculus humanus humans, Phthirus pubis, Haematopinus eurysternus, Dalmalinia ovis*, *Haematopinus suis,* etc.

Hymenoptera pests: Formicidae such as *Monomorium pharaosis, Formica fusca japonica, Ochetellus glaber, Pristomyrmex pungens, Pheidole noda, Acromyrmex spp. , Solenopsis spp. , Linepithema humile*; Vespidae; Bethylidae; Tenthredinidae such as *Athalia rosae, Athalia japonica,* etc.

Blattaria pests: Blattidae such as *Blattella germanica, Periplaneta fuliginosa, Periplaneta americana, Periplaneta brunnea, Blatta orientalis;* Termitidae such as *Reticulitermes speratus, Coptotermes formosanus, Incisitermes minor, Cryptotermes domesticus, Odontotermes formosanus, Neotermes koshunensis, Glyptotermes satsumensis, Glyptotermes nakajimai, Glyptotermes fuscus, Glyptotermes kodamai, Glyptotermes kushimensis, Hodotermopsis japonica, Coptotermes guangzhoensis, Reticulitermes miyatakei, Reticulitermes flaviceps amamianus, Reticulitermes sp., Nasutitermes takasagoensis, Pericapritermes nitobei, Sinocapritermes mushae,* etc.

Acarine pests: Tetranychidae such as *Tetranychus urticae, Tetranychus kanzawai, Panonychus citri, Panonychus ulmi, Oligonychus spp. ;* Eriophyidae such as *Aculops pelekassi, Phyllocoptruta citri, Aculops lycopersici, Calacarus carinatus, Acaphylla theavagrans, Eriophyes chibaensis, Aculus schlechtendali;* Tarsonemidae such as *Polyphagotarsonemus latus, Acarapis woodi ;* Tenuipalpidae such as *Brevipalpus phoenicis;* Tuckerellidae; Ixodidae such as *Amblyomma americanum, Ambryomma maculatum, Boophilus microplus, Boophilus annulatus, Dermacentor variabilis, Dermacentor taiwanicus, Dermacentor andersoni, Haemaphysalis longicornis, Haemaphysalis flava, Haemaphysalis campanulata, Ixodes ovatus, Ixodes persulcatus, Ixodes scapularis, Ixodes pacificus, Ixodes holocyclus, Rhipicephalus sanguineus, Rhipicephalus appendiculatus;* Argasidae such as *Argas persicus;* Psoroptidae such as *Octodectes cynotis;* Sarcoptidae such as *Sacroptes scabiei;* Listrophoridae such as *Listrophorus gibbus;* Demodicidae such as *Demodex canis;* Acaridae such as *Tyrophagus putrescentiae, Tyrophagus similis;* Pyroglyphidae such as *Dermatophagoides farinae, Dermatophagoides ptrenyssnus;* Cheyletidae such as *Cheyletus eruditus, Cheyletus malaccensis, Cheyletus moorei;* Laelapidae such as *Ornithonyssus bacoti, Ornithonyssus sylvairum, Varroa jacobsoni;* Dermanyssidae such as *Dermanyssus gallinae;* Trombiculidae such as *Leptotrombidium akamushi;* Araneae such as *Chiracanthium japonicum, Latrodectus hasseltii*.

Chilopoda: Thereuonema *hilgendorfi, Scolopendra subspinipes,* etc.

Diplopoda: *Oxidus gracilis, Nedyopus tambanus,* etc.

Isopoda: *Armadillidium vulgare*, etc.

Gastropoda: *Limax marginatus, Limax flavus,* etc.

The pest control composition of the present invention may contain only the present hydrazide compound and the present neonicotinoid compound, but they are generally mixed with a solid carrier, a liquid carrier and/or a gaseous carrier and can be formulated into a liquid such as an emulsion, an oil solution, an oily liquid, an aqueous liquid, a solution, a shampoo or a suspension, dusts, granules, a paste formulation, a cream, an ointment, a microencapsulated formulation, a foam, an aerosol formulation, a carbon dioxide formulation, a tablet, a chewable tablet, a bolus, a capsule, an animal feed premix, a syrup, a sheet formulation, a film formulation, a resin formulation, an injection, an implant, a suppository, or the like, by further adding a surfactant and any other formulation adjuvants as needed. These formulations may be also used processed into a poison bait, a mosquito coil, an electric mosquito repellant mat, a smoking agent, a fumigant, or a sheet.

These formulations generally contain 0.1 to 95% by weight of the total weight of the present hydrazide compound and the present neonicotinoid compound.

Examples of the solid carrier used in making a formulation include fine powders and granules of clays (kaolin clay, diatomaceous earth, bentonite, Fubasami clay, activated clay, etc.), synthetic hydrous silicon oxide, talc, ceramics, other inorganic minerals (sericite, quartz, sulfur, activated carbon, calcium carbonate, hydrated silica, etc.), chemical fertilizers (ammonium sulfate, ammonium phosphate, ammonium nitrate, ammonium chloride, urea, etc.), and the like.

Examples of the liquid carrier include aromatic or aliphatic hydrocarbons (xylene, toluene, alkylnaphthalene, phenylxylylethane, kerosene, light oil, hexane, cyclohexane, etc.), halogenated hydrocarbons (chlorobenzene, dichloromethane, dichloroethane, trichloroethane, etc.), alcohols (methanol, ethanol, isopropyl alcohol, butanol, hexanol, ethylene glycol, etc.), ethers (diethyl ether, ethylene glycol dimethyl ether, diethylene glycol monomethyl ether, diethylene glycol monoethyl ether, propylene glycol monomethyl ether, tetrahydrofuran, dioxane, etc.), esters (ethyl acetate, butyl acetate, etc.), fatty acid esters (diisopropyl adipate, diisobutyl adipate, isopropyl myristate, etc.), ketones (acetone, methyl ethyl ketone, methyl isobutyl ketone, cyclohexanone, etc.), nitriles (acetonitrile, isobutyronitrile, etc.), sulfoxides (dimethyl sulfoxide, etc.), acid amides (N,N-dimethylformamide, N,N-dimethylacetamide, etc.), pyrrolidones (N-methyl-2-pyrrolidone, N-octyl-2-pyrrolidone, etc.), propylene carbonate, ethyl lactate, 1,3-dimethyl-2-imidazolidinone, vegetable oils (soybean oil, cotton seed oil, etc.), vegetable essential oils (orange oil, hyssop oil, lemon oil, etc.), silicone oils (dimethyl silicone oil, highly polymerized dimethyl silicone oil, cyclic silicone oil, polyether-modified silicone oil, amino-modified silicone oil, methyl phenyl silicone oil, etc.), water, and the like.

Examples of the gaseous carrier include butane gas, flon gas, liquefied petroleum gas (LPG), dimethyl ether, carbon dioxide gas, and the like.

Examples of the surfactant include alkylsulfate ester salts, alkylsulfonate salts, alkylarylsulfonate salts, alkylaryl

ethers and polyoxyethylenated forms thereof, polyethylene glycol ethers, polyhydric alcohol esters, and sugar alcohol derivatives.

Examples of the other formulation adjuvant include binders, dispersants, stabilizers, and the like. Specific examples include casein, gelatin, polysaccharides (starch, gum Arabic, cellulose derivatives, alginic acid, etc.), lignin derivatives, bentonite, saccharides, synthetic water-soluble polymers (polyvinyl alcohol, polyvinylpyrrolidine, polyacrylic acids, etc.), PAP (acidic isopropyl phosphate), BHT (2,6-di-t-butyl-4-methylphenol), BHA (mixture of 2-t-butyl-4-methoxyphenol and 3-t-butyl-4-methoxyphenol), vegetable oils, mineral oils, fatty acids, and fatty acid esters.

Examples of the base material of a resin formulation include vinyl chloride polymers, polyurethane, and the like. A plasticizer such as phthalic acid esters (dimethyl phthalate, dioctyl phthalate, etc.), adipic acid esters, or stearic acid may be added as needed to these base materials. After kneading the compound into the base material using an ordinary kneading machine, a resin formulation is obtained by injection molding, extrusion molding, press molding, or the like, and can also undergo further processes such as molding and cutting, as needed, processing into a plate, film, tape, net, or string resin formulation. These resin formulations are processed into, for example, an animal collar, an animal ear tag, a sheet formulation, a lead, or a horticultural post.

Examples of the base material of a poison bait include grain powder, vegetable oils, sugars, crystalline cellulose, and the like. Further, antioxidants such as dibutylhydroxytoluene and nordihydroguaiuretic acid, preservatives such as dehydroacetic acid, agents to prevent accidental ingestion by children and pets such as chili pepper; and pest attractive flavors such as cheese flavor, onion flavor, and peanut oil, and the like are added as needed.

The pest control method of the present invention comprises a step of applying effective amounts of the present hydrazide compound and the present neonicotinoid compound directly to pests and/or to habitats of pests (plant body, animal body, indoor, soil, etc.). The present hydrazide compound and the present neonicotinoid compound may be applied separately within the same time frame, but they are generally applied as the pest control composition of the present invention for the sake of simplicity during application.

When the present hydrazide compound and the present neonicotinoid compound are used to control agricultural and forestry pests, the amount applied is generally 1 to 10,000 g/ha and preferably 10 to 500 g/ha, by the total weight of the present hydrazide compound and the present neonicotinoid compound. Emulsifiable concentrates, wettable powders, flowables, microcapsule formulations, and the like are generally used diluted with water so that the total of the present hydrazide compound and the present neonicotinoid compound will be 1 to 1, 000 ppm. Powders, granules, and the like are generally used as they are. These formulations may be dispersed directly on the plants that are to be protected from pests. Pests that live in the soil can also be controlled by treating the soil with these formulations. These formulations can also be used to treat the seedling bed before planting or to treat the planting hole or stalk at the time of planting. Furthermore, a sheet formulation of the pest control agent of the present invention can also be used by the method of wrapping it around plants, placing it near plants, covering the soil surface near the stalk, and the like.

The pest control agent of the present invention can be used in farmland where the following "crops" are grown.

Agricultural crops: corn, rice, wheat, barley, rye, oats, sorghum, cotton, soybeans, peanuts, buckwheat, sugar beet, rapeseed, sunflower, sugarcane, tobacco, etc.,

Vegetables: solanaceous vegetables (eggplant, tomato, pepper, hot pepper, potato, etc.), cucurbitaceous vegetables (cucumber, pumpkin, zucchini, watermelon, melon, etc.), cruciferous vegetables (radishes, turnips, horseradish, kohlrabi, Chinese cabbage, cabbage, mustard greens, broccoli, cauliflower, etc.), Compositae vegetables (burdock, crown daisy, artichoke, lettuce, etc.), Liliaceae vegetables (leek, onion, garlic, and asparagus), Umbelliferae vegetables (carrot, parsley, celery, parsnip, etc.), Chenopodiaceae vegetables (spinach, Swiss chard, etc.), Labiatae vegetables (beef steak plant, mint, basil, etc.), strawberries, sweet potato, yam, taro, etc.,

Flowers,

Foliage plants,

Fruit trees: pomaceous fruits (apple, pear, Japanese pear, Chinese quince, quince, etc.), stone fleshy fruits (peach, plum, nectarine, Japanese plum, cherry, apricot, prune, etc.), citrus plants (Satsuma mandarin, orange, lemon, lime, grapefruit, etc.), nuts (chestnut, walnut, hazelnut, almond, pistachio, cashew, macadamia nut, etc.), berry fruits (blueberry, cranberry, blackberry, raspberry, etc.), grapes, persimmon, olive, loquat, banana, coffee, date, coconut, etc.,

Trees other than fruit trees: tea, mulberry, flowering trees and shrubs, street trees (ash tree, birch, dogwood, eucalyptus, ginkgo, lilac, maple tree, oak, poplar, cercis, Chinese sweet gum, plane tree, zelkova, Japanese arbovitae, fir trees, Japanese hemlock, needle juniper, pine, spruce, yew), etc.

The "crops" also include genetically engineered crops.

When both of the present hydrazide compound and the present neonicotinoid compound are used for preventing epidemics, the application amount is generally 0.001 to 10 mg/$m^3$ in the case of application in a space, and is 0.001 to 100 mg/$m^2$ in the case of application on a plane, by the total of the present hydrazide compound and the present

neonicotinoid compound. Emulsifiable concentrates, wettable powders, flowables, and the like are generally applied diluted with water so that the concentration of the total of the present hydrazide compound and the present neonicotinoid compound will be 0.01 to 10, 000 ppm. Oil solutions, aerosols, smoking agents, poison baits, and the like are generally applied as they are.

When the present hydrazide compound and the present neonicotinoid compound are used for controlling ectoparasites on livestock such as cattle, horse, pig, sheep, goat and chicken, and small animals such as dog, cat, rat and mouse, they can be used for the animals by an oral or parenteral administration method known in veterinary medicine. Specific methods of use include, when used for systemic control, oral preparations such as tablets (including pills, sugar-coated tablets, and film-coated tablets), chewable tablets, capsules, granules (coarse granules, fine granules, powders, and the like), liquids (emulsions, suspensions, and the like), and film formulations; parenteral preparations such as injections (e.g., subcutaneous injections, intravenous injections, intramuscular injections, intraperitoneal injections, infusions, and sustained-release injections), suppositories (e.g., rectal suppositories and vaginal suppositories), implants (including implant tablets, those formed using a biodegradable polymer as the base material, those that release the active ingredients at a constant rate by encapsulation in a capsule made of biocompatible metal such as titanium); and the like. Furthermore, in the case of oral administration, the above formulations can be used mixed with feed.

Also, when used for non-systemic control, the present hydrazide compound and the present ester compound are used by the methods such as spraying an oily or aqueous liquid, moistening with a cream, ointment, or the like, pour-on or spot-on treatment by a liquid, washing the animal with a shampoo formulation, making a resin preparation into a collar or ear tag and attaching it to the animal, or the like. When administered to an animal body, the total amount of the present hydrazide compound and the present neonicotinoid compound is generally in a range from 0.1 to 1, 000 mg per 1 kg of an animal body weight.

Various organic and inorganic carriers commonly used as formulation materials are used in the pharmaceutical dosage forms used in the above veterinary methods of administration, and formulated as excipients, lubricants, binders, disintegrants, and the like in solid formulations; and solvents, solubilizers, suspending agents, isotonizing agents, buffers, analgesic agents, and the like in liquid formulations. In addition, formulation additives such as preservatives, antioxidants, colorings, and sweeteners can also be used as needed. Preferred examples of the excipients include lactose, saccharose, D-mannitol, D-sorbitol, starch, α-starch, dextrin, crystalline cellulose, low-substituted hydroxypropylcellulose, carboxymethylcellulose sodium, gum arabic, dextrin, pullulan, light silicic anhydride, synthetic aluminum silicate, magnesium aluminometasilicate, and the like. Preferred examples of the lubricants include magnesium stearate, calcium stearate, talc, colloidal silica, and the like. Preferred examples of the binders include α-starch, sucrose, gelatin, gum arabic, methylcellulose, carboxymethylcellulose, carboxymethylcellulose sodium, crystalline cellulose, saccharose, D-mannitol, trehalose, dextrin, pullulan, hydroxypropylcellulose, hydroxypropylmethylcellulose, polyvinylpyrrolidine, and the like. Preferred examples of the disintegrants include lactose, saccharose, starch, carboxymethylcellulose, carboxymethylcellulose calcium, croscarmellose sodium, carboxymethylstarch sodium, light silicic anhydride, low-substituted hydroxypropylcellulose, and the like. Preferred examples of the solvents include water for injection, physiological saline, Ringer's solution, alcohol, propylene glycol, polyethylene glycol, sesame oil, corn oil, olive oil, cottonseed oil, and the like. Preferred examples of the solubilizers include polyethylene glycol, propylene glycol, D-mannitol, trehalose, benzyl benzoate, ethanol, tris aminomethane, cholesterol, triethanolamine, sodium carbonate, sodium citrate, sodium salicylate, sodium acetate, and the like. Preferred examples of the suspending agents include surfactants such as stearyl triethanolamine, sodium lauryl sulfate, lauryl aminopropionic acid, lecithin, benzalkonium chloride, benzethonium chloride, and glycerin monostearate; hydrophilic polymers, for example, polyvinyl alcohol, polyvinylpyrrolidone, carboxymethylcellulose sodium, methylcellulose, hydroxymethylcellulose, hydroxyethylcellulose, hydroxypropylcellulose, and the like; polysorbates, polyoxyethylene hydrogenated castor oil, and the like. Preferred examples of the isotonizing agents include sodium chloride, glycerin, D-mannitol, D-sorbitol, glucose, and the like. Preferred examples of the buffers include buffers such as phosphate, acetate, carbonate, and citrate. Preferred examples of the analgesics include benzyl alcohol and the like. Preferred examples of the preservatives include paraoxybenzoic acid esters, chlorobutanol, benzyl alcohol, phenethyl alcohol, dehydroacetic acid, sorbic acid, and the like. Preferred examples of the antioxidants include sulfites, ascorbates, and the like. Preferred examples of the colorings include water-soluble food coal-tar dyes (e.g., food dyes such as Food Red Nos. 2 and 3, Food Yellow Nos. 4 and 5, and Food Blue Nos. 1 and 2, water-insoluble Lake dyes (e.g., aluminum salts of the aforementioned water-soluble food coal-tar dyes, and the like), natural colors (e.g., β-carotene, chlorophyll, colcothar, and the like), and the like.

Also, injections are produced by dissolving, suspending, or emulsifying the active ingredients together with a dispersing agent (e.g., Polysorbate 80, polyoxyethylene hydrogenated castor oil 60, and the like, polyethylene glycol, carboxymethylcellulose, sodium alginate, and the like), a preservative (e.g., methylparaben, propylparaben, benzyl alcohol, chlorobutanol, phenol, and the like), an isotonizing agent (e.g., sodium chloride, glycerin, D-mannitol, D-sorbitol, glucose, and the like), and the like in an aqueous solvent (e.g., distilled water, physiological saline, Ringer's solution, and the like) or an oily solvent (e.g., vegetable oils such as olive oil, sesame oil, cottonseed oil, and corn oil, propylene glycol, and the like) and the like. In this case, additives such as solubilizers (e.g., sodium salicylate, sodium acetate,

and the like), stabilizers (e.g., human serum albumin and the like), and analgesics (e.g., benzyl alcohol and the like) may also be used as desired. Injections are generally packaged in suitable ampules.

The pest control composition of the present invention can also be used as a sustained-release formulation. The sustained-release formulations include microcapsules (for example, microspheres, microcapsules, microparticles, etc.), those formed from biocompatible polymers, and the like, produced by methods such as in-water drying (o/w methods, w/o/w methods, etc.), phase separation, spray drying, and methods that accord with these.

The pest control composition of the present invention can also be used in combination with or as a mixture with other insecticides, nematocides, miticides, fungicides, herbicides, plant growth regulators, synergists, fertilizers, soil conditioners, animal feeds, and the like.

## EXAMPLES

Hereinafter, the present invention will be described in more detail by way of production examples of the present hydrazide compound and production intermediates of the present Hydrazide compound, formulation examples of the pest control composition of the present invention, and test examples, but the present invention is not limited to these examples.

Here, the abbreviations used herein have the following meanings.

Me: methyl group, Et: ethyl group, Ph: phenyl group.

First, production examples of the present hydrazide compound will be given below.

### Production Example 1

In 12 mL of tetrahydrofuran were dissolved 1.39 g of the tert-butyl N-{3-[5-(3,5-dichlorophenyl)-5-trifluoromethyl-4,5-dihydroisoxazol-3-yl]phenyl}carbazate obtained by Reference Production Example 6 and 330 mg of triethylamine. After adding 257 mg of acetyl chloride dropwise thereto at 0°C and stirring at 0°C for 1 hour, the mixture was allowed to stand at room temperature for 13 hours. Water was added to the reaction mixture, and the mixture was extracted with ethyl acetate. The organic layer was dried over anhydrous sodium sulfate and then concentrated under reduced pressure. The resulting residue was subjected to silica gel column chromatography to obtain 1.30 g of tert-butyl N'-acetyl-N-{3-[5-(3,5-dichlorophenyl)-5-trifluoromethyl-4,5-dihydroisoxazol-3-yl]phenyl}carbazate (herein referred to as the present hydrazide compound (1).).

Present hydrazide compound (1)

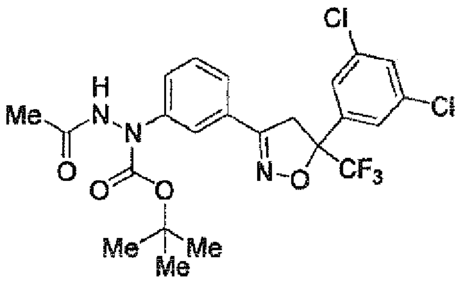

Melting point: 111°C

### Production Example 2

In 1 mL of tetrahydrofuran was dissolved 250 mg of the tert-butyl N'-acetyl-N-{3-[5-(3,5-dichlorophenyl)-5-trifluoromethyl-4,5-dihydroisoxazol-3-yl]phenyl}carbazate obtained by Production Example 1, and 1.5 mL of trifluoroacetic acid was added dropwise thereto at room temperature, and then the mixture was stirred for 1 hour. A saturated sodium bicarbonate aqueous solution was added to the reaction mixture, and the mixture was extracted with ethyl acetate. The organic layer was dried over anhydrous sodium sulfate and then concentrated under reduced pressure. The resulting residue was subjected to silica gel column chromatography to obtain 179 mg of N'-{3-[5-(3,5-dichlorophenyl)-5-trifluoromethyl-4,5-dihydroisoxazol-3-yl]phenyl}acetohydrazide (herein referred to as the present hydrazide compound (2).).

Present hydrazide compound (2)

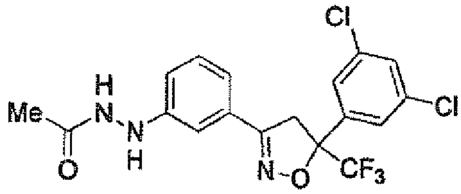

Melting point: 100°C

Production Example 3

In 4 mL of tetrahydrofuran, 78 mg of sodium hydride (60% in oil) was suspended. A solution of 800 mg of the tert-butyl N'-acetyl-N-{3-[5-(3,5-dichlorophenyl)-5-trifluoromethyl-4,5 -dihydroisoxazol-3-yl]phenyl}carbazate obtained by Production Example 1 dissolved in 6 mL of tetrahydrofuran was added dropwise thereto at room temperature. After stirring for 30 minutes at the same temperature, 355 mg of methyl iodide was added thereto at room temperature, and the mixture was stirred at the same temperature for 10 hours. A saturated sodium bicarbonate aqueous solution was added to the mixture, and the mixture was extracted with ethyl acetate. The organic layer was dried over anhydrous sodium sulfate and then concentrated under reduced pressure. The resulting residue was subjected to silica gel column chromatography to obtain 697 mg of tert-butyl N'-acetyl-N'-methyl-N-{3-[5-(3,5-dichlorophenyl)-5-trifluoro methyl-4,5-dihydroisoxazol-3-yl]phenyl}carbazate (herein referred to as the present hydrazide compound (3).).

Present hydrazide compound (3)

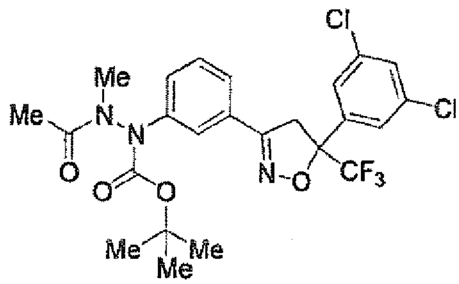

Melting point: 79°C

Production Example 4

To 324 mg of the tert-butyl N'-acetyl-N'-methyl-N-{3-[5-(3,5-dichlorophenyl)-5-trifluoromethyl-4,5-dihydroisoxazo 1-3-yl]phenyl}carbazate obtained by Production Example 3 was added 5 mL of trifluoroacetic acid at room temperature, and the mixture was stirred at the same temperature for 30 minutes. The reaction mixture was concentrated under reduced pressure, and ethyl acetate was added to the residue. The organic layer was washed with a saturated sodium bicarbonate aqueous solution. The organic layer was dried over anhydrous sodium sulfate and then concentrated under reduced pressure. The resulting residue was subjected to silica gel column chromatography to obtain 290 mg of N-methyl-N'-{3-[5-(3,5-dichlorophenyl)-5-trifluoromethyl-4,5 -dihydroisoxazol-3-yl]phenyl}acetohydrazide (herein referred to as the present hydrazide compound (4).).

Present hydrazide compound (4)

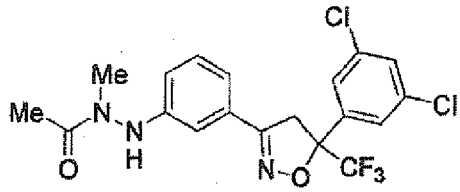

Melting point: 88°C

Production Example 5

In 3 mL of tetrahydrofuran were dissolved 1.00 g of the crude N-methyl-N-{3-[5-(3,5-dichlorophenyl)-5-trifluoromethyl-4,5-dihydroisoxazol-3-yl]phenyl}hydrazine obtained by Reference Production Example 10 and 180 mg of triethylamine, and 139 mg of acetyl chloride was added dropwise thereto at 0°C, and then the mixture was stirred at the same temperature for 1 hour. A saturated sodium bicarbonate aqueous solution was added to the reaction mixture, and the mixture was extracted with ethyl acetate. The organic layer was dried over anhydrous sodium sulfate and then concentrated under reduced pressure. The resulting residue was subjected to silica gel column chromatography to obtain 498 mg of N'-methyl-N'-{3-[5-(3,5-dichlorophenyl)-5-trifluoromethyl-4, 5-dyhydroisoxazol-3-yl]phenyl}acetohydrazide (herein referred to as the present hydrazide compound (5).).

Present hydrazide compound (5)

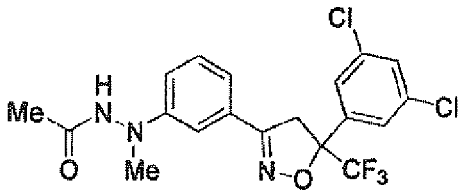

Melting point: 87°C

Production Example 6

In 2 mL of N,N-dimethylformamide was dissolved 248 mg of the N'-methyl-N'-{3-[5-(3,5-dichlorophenyl)-5-trifluoromethyl-4,5-dihydroisoxazol-3-yl]phenyl}acetohydrazi de obtained by Production Example 5, and 92 mg of sodium carbonate and 87 mg of methyl iodide were added thereto at room temperature. After stirring for 5 hours at the same temperature, 20 mL of tert-butyl methyl ether was added, and the precipitate was filtered out. The filtrate was concentrated under reduced pressure, and the resulting residue was subjected to silica gel column chromatography to obtain 140 mg of N-methyl-N'-methyl-N'-{3-[5-(3,5-dichlorophenyl)-5-trifluoro methyl-4,5-dihydroisoxazol-3-yl]phenyl}acetohydrazide (herein referred to as the present hydrazide compound (6).).

Present hydrazide compound (6)

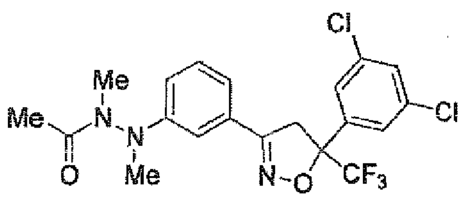

Melting point: 151°C

Production Example 7

In 5 mL of tetrahydrofuran were dissolved 1.04 g of the crude 3-[5-(3,5-dichlorophenyl)-5-trifluoromethyl-4,5-dihydroisoxazol-3-yl]phenylhydrazine obtained by Reference Production Example 3 and 269 mg of triethylamine, and 391 mg of 3,3,3-trifluoropropionyl chloride was added dropwise thereto at 0°C, and then the mixture was stirred at room temperature for 4 hours. A saturated sodium bicarbonate aqueous solution was added to the reaction mixture, and the mixture was extracted with ethyl acetate. The organic layer was dried over anhydrous sodium sulfate and then concentrated under reduced pressure. The resulting residue was subjected to silica gel column chromatography to obtain 639 mg of N'-{3-[5-(3,5-dichlorophenyl)-5-trifluoromethyl-4,5-dihydroi soxazol-3-yl]phenyl}-3,3,3-trifluoropropionohydrazide (herein referred to as the present hydrazide compound (7).).

Present hydrazide compound (7)

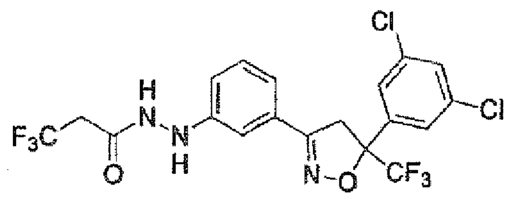

Melting point: 75°C

Production Example 8

In 2 mL of ethyl acetate were dissolved 220 mg of the crude 5-[5-(3,5-dichlorophenyl)-5-trifluoromethyl-4,5-dihydroisoxa zol-3-yl]-2-methylphenylhydrazine obtained by Reference Production Example 14 and 43 mg of pyridine, and 80 mg of 3, 3, 3-trifluoropropionyl chloride was added dropwise thereto at 0°C, and then the mixture was stirred at room temperature for 4 hours. Water was added to the reaction mixture, and the mixture was extracted with ethyl acetate. The organic layer was dried over anhydrous sodium sulfate and then concentrated under reduced pressure. The resulting residue was subjected to silica gel column chromatography to obtain 162 mg of N'-{5-[5-(3,5-dichlorophenyl)-5-trifluoromethyl-4,5-dihydroi soxazol-3-yl]-2-methylphenyl}-3,3,3-trifluoropropionohydrazi de (herein referred to as the present hydrazide compound (8).).

Present hydrazide compound (8)

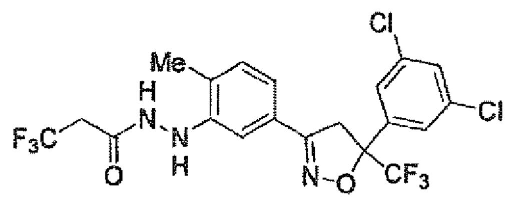

$^1$H-NMR($CDCl_3$)δ: 8.01-7.96(1H, br m), 7.48-7.42(3H, m), 7.15-7.04(3H, m), 6.11-6.00(1H, m), 4.04-4.00(1H, m), 3.63 (1H, d, J=17.1 Hz), 3.25-3.19(2H, m), 2.26(3H, s).

Production Example 9

In 2 mL of ethyl acetate were dissolved 220 mg of the crude 5-[5-(3,5-dichlorophenyl)-5-trifluoromethyl-4,5-dihydroisoxa zol-3-yl]-2-methylphenylhydrazine obtained by Reference Production Example 14 and 43 mg of pyridine, and 80 mg of isopropyl chloroformate was added dropwise thereto at 0°C, and then the mixture was stirred at room temperature for 4 hours. Water was added to the reaction mixture, and the mixture was extracted with ethyl acetate. The organic layer was dried over anhydrous sodium sulfate and then concentrated under reduced pressure. The resulting

residue was subjected to silica gel column chromatography to obtain 127 mg of isopropyl N'-{5-[5-(3,5-dichlorophenyl)-5-trifluoromethyl-4,5-dihydroi soxazol-3-yl]-2-methylphenyl}carbazate (herein referred to as the present hydrazide compound (9).).

Present hydrazide compound (9)

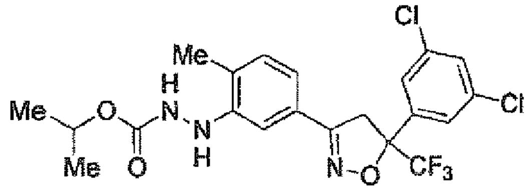

Melting point: 71°C

Production Example 10

In 6 mL of tetrahydrofuran were dissolved 412 mg of the crude 5-[5-(3,5-dichlorophenyl)-5-trifluoromethyl-4,5-dihydroisoxazol-3-yl]-2-fluorophenylhydrazine obtained by Reference Production Example 18 and 102 mg of triethylamine, and 148 mg of 3,3,3-trifluoropropionyl chloride was added dropwise thereto at 0°C, and then the mixture was stirred at room temperature for 4 hours. A saturated sodium bicarbonate aqueous solution was added to the reaction mixture, and the mixture was extracted with ethyl acetate. The organic layer was dried over anhydrous sodium sulfate and then concentrated under reduced pressure. The resulting residue was subjected to silica gel column chromatography to obtain 167 mg of N'-{5-[5-(3,5-dichlorophenyl)-5-trifluoromethyl-4,5-dihydroi soxazol-3-yl]-2-fluorophenyl}-3,3,3-trifluoropropionohydrazi de (herein referred to as the present hydrazide compound (10).).

Present hydrazide compound (10)

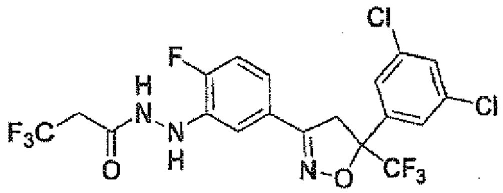

$^1$H-NMR($CDCl_3$)$\delta$: 7.89-7.55(1H, m),7.46-7.42(3H, m), 7.28(1H, dd, J=7.9, 1.8 Hz), 7.18-7.00(2H, m), 6.39-6.29 (1H, m), 4.03-3.98(1H, m), 3.65-3.60(1H, m), 3.21(2H, q, J=10.3 Hz).

Production Example 11

In 6 mL of tetrahydrofuran were dissolved 510 mg of the crude 5-[5-(3,5-dichlorophenyl)-5-trifluoromethyl-4,5-dihydroisoxazol-3-yl]-2-chlorophenylhydrazine obtained by Reference Production Example 21 and 123 mg of triethylamine, and 179 mg of 3,3,3-trifluoropropionyl chloride was added dropwise thereto at 0°C, and then the mixture was stirred at room temperature for 2.5 hours. A saturated sodium bicarbonate aqueous solution was added to the reaction mixture, and the mixture was extracted with ethyl acetate. The organic layer was dried over anhydrous sodium sulfate and then concentrated under reduced pressure. The resulting residue was subjected to silica gel column chromatography to obtain 175 mg of N'-{5-[5-(3,5-dichlorophenyl)-5-trifluoromethyl-4,5-dihydroi soxazol-3-yl]-2-chlorophenyl}-3,3,3-trifluoropropionohydrazi de (herein referred to as the present hydrazide compound (11).).

Present hydrazide compound (11)

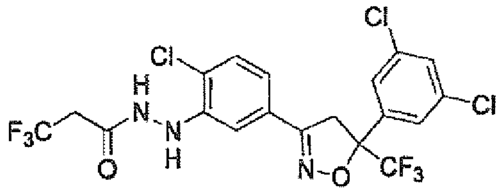

Melting point: 89°C

Production Example 12

In 2 mL of tetrahydrofuran were dissolved 110 mg of tert-butyl N-{2-chloro-5-[5-(3,5-dichlorophenyl)-5-trifluoromethyl-4,5-dihydroisoxazol-3-yl]phenyl}carbazate obtained by Reference Production Example 23 and 22 mg of triethylamine, and 23 mg of cyclopropanecarbonyl chloride was added dropwise thereto at °C, and then the mixture was stirred at room temperature for 1 hour. Water was added to the reaction mixture, and the mixture was extracted with ethyl acetate. The organic layer was dried over anhydrous sodium sulfate and then concentrated under reduced pressure. The resulting residue was subjected to silica gel column chromatography to obtain 101 mg of tert-butyl N'-cyclopropanecarbonyl-N-{2-chloro-5-[5-(3,5-dichlorophenyl )-5-trifluoromethyl-4,5-dihydroisoxazol-3-yl]phenyl}carbazat e (herein referred to as the present hydrazide compound (12).).

Present hydrazide compound (12)

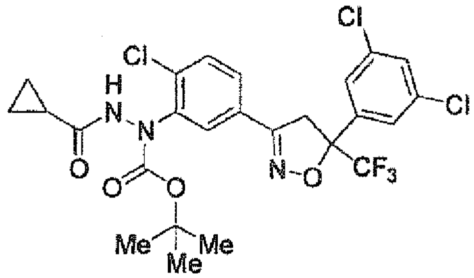

Melting point: 180°C

Production Example 13

To 272 mg of the tert-butyl N'-cyclopropanecarbonyl-N-{2-chloro-5-[5-(3,5-dichlorophenyl)-5-trifluoromethyl-4,5-di hydroisoxazol-3-yl]phenyl}carbazate obtained by Production Example 12 was added 2 mL of trifluoroacetic acid at room temperature, and the mixture was stirred at the same temperature for 1 hour. The reaction mixture was concentrated under reduced pressure, and then ethyl acetate was added to the residue, and the mixture was washed witch a saturated sodium bicarbonate aqueous solution. The organic layer was dried over anhydrous sodium sulfate and then concentrated under reduced pressure. The resulting residue was subjected to silica gel column chromatography to obtain 214 mg of N'-{2-chloro-5-[5-(3,5-dichlorophenyl)-5-trifluoromethyl-4,5 -dihydroisoxazol-3-yl]phenyl}cyclopropanecarbohydrazide (herein referred to as the present hydrazide compound (13).).

Present hydrazide compound (13)

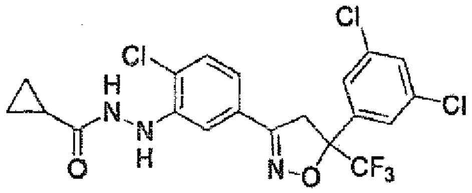

Melting point: 100°C

Production Example 14

In 2 mL of tetrahydrofuran were dissolved 262 mg of the tert-butyl N-{2-chloro-5-[5-(3,5-dichlorophenyl)-5-trifluoromethyl-4,5-dihydroisoxazol-3-yl]phenyl}carbazate obtained by Reference Production Example 23 and 61 mg of triethylamine, and 72 mg of isovaleryl chloride was added dropwise thereto at 0°C, and then the mixture was stirred at room temperature for 9 hours. Water was added to the reaction mixture, and the mixture was extracted with ethyl acetate. The organic layer was dried over anhydrous sodium sulfate and then concentrated under reduced pressure. The resulting residue was subjected to silica gel column chromatograph to obtain 280 mg of tert-butyl N'-isovaleryl-N-{2-chloro-5-[5-(3,5-dichlorophenyl)-5-triflu oromethyl-4,5-dihydroisoxazol-3-yl]phenyl}carbazate (herein referred to as the present hydrazide compound (14).).

Present hydrazide compound (14)

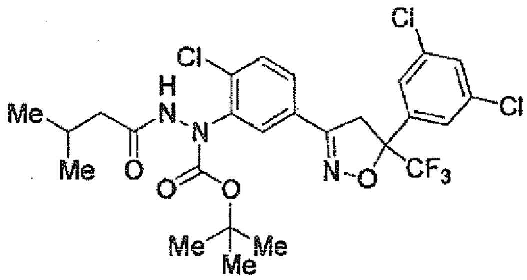

$^1$H-NMR($CDCl_3$)$\delta$: 7.82-7.70(3H, m),7.48-7.44(4H,m), 4.08-4.04(1H, m), 3.71-3.66(1H, m), 2.18-2.10(3H, m), 1.45(9H, d, J=52.9 Hz), 0.98(6H, d, J=6.1 Hz).

Production Example 15

To 280 mg of the tert-butyl N'-isovaleryl-N-{2-chloro-5-[5-(3,5-dichlorophenyl)-5-triflu oromethyl-4,5-dihydroisoxazol-3-yl]phenyl}carbazate obtained by Production Example 14 was added 2 mL of trifluoroacetic acid at room temperature, and the mixture was stirred at the same temperature for 2 hours. The reaction mixture was concentrated under reduced pressure, and ethyl acetate was added to the residue. The organic layer was washed with a saturated sodium bicarbonate aqueous solution. The organic layer was dried over anhydrous sodium sulfate and then concentrated under reduced pressure. The resulting residue was subjected to silica gel column chromatography to obtain 222 mg of N'-{2-chloro-5-[5-(3,5-dichlorophenyl)-5-trifluoromethyl-4,5 -dihydroisoxazol-3-yl]phenyl}isovalerohydrazide (herein referred to as the present hydrazide compound (15).).

Present hydrazide compound (15)

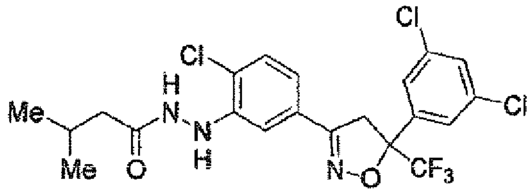

Melting point: 84°C

Production Example 16

In 1.5 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 57 mg of triethylamine, and 79 mg of benzoyl chloride was added dropwise thereto at 0°C, and then the mixture was stirred at room temperature for 2 hours. The reaction mixture was concentrated under reduced pressure, and the resulting residue was subjected to silica gel column chromatography to obtain 220 mg of N'-{5-[5-(3,5-dichlorophenyl)-5-trifluoromethyl-4,5-dihydroi soxazol-3-yl]-2-chlorophenyl}benzohydrazide (herein referred to as the present hydrazide compound (16).).

Present hydrazide compound (16)

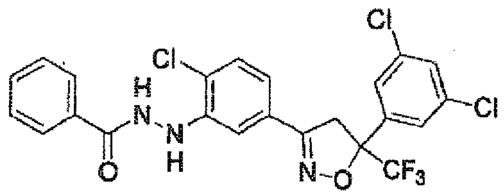

Melting point: 105°C

Production Example 17

In 2 mL of tetrahydrofuran were dissolved 200 mg of the crude 5-[5-(3,5-diohlorophenyl)-5-trifluoromethyl-4,5-dihydroisoxazol-3-yl]-2-chlorophenylhydrazine obtained by Reference Production Example 21 and 57 mg of triethylamine, and 90 mg of 4,4,4-trifluorobutyryl chloride was added dropwise thereto at 0°C, and then the mixture was stirred at room temperature for 2 hours. The reaction mixture was concentrated under reduced pressure, and the resulting residue was subjected to silica gel column chromatography to obtain 201 mg of N'-{5-[5-(3,5-dichlorophenyl)-5-trifluoromethyl-4,5-dihydroi soxazol-3-yl]-2-chlorophenyl}-4,4,4-trifluorobutyrohydrazide (herein referred to as the present hydrazide compound (17).).

Present hydrazide compound (17)

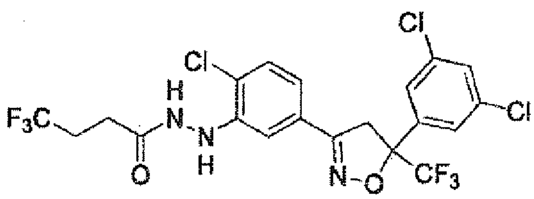

Melting point: 82°C

Production Example 18

In 4 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 57 mg of triethylamine, and 44 mg of acetyl chloride was added dropwise thereto at 0°C, and then the mixture was stirred at room temperature for 2 hours. The reaction mixture was concentrated under reduced pressure, and the resulting residue was subjected to silica gel column chromatography to obtain 168 mg of N'-{5-[5-(3,5-dichlorophenyl)-5-trifluoromethyl-4,5-dihydroi soxazol-3-yl]-2-chlorophenyl}acetohydrazide (herein referred to as the present hydrazide compound (18).).

Present hydrazide compound (18)

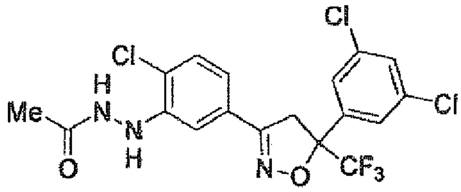

Melting point: 94°C

Production Example 19

In 4 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 57 mg of triethylamine, and 52 mg of propionyl chloride was added dropwise thereto at 0°C, and then the mixture was stirred at room temperature for 2 hours. The reaction mixture was concentrated under reduced pressure, and the resulting residue was subjected to silica gel column chromatography to obtain 195 mg of N'-{5-[5-(3,5-dichlorophenyl)-5-trifluoromethyl-4,5-dihydroi soxazol-3-yl]-2-chlorophenyl}propionohydrazide (herein referred to as the present hydrazide compound (19).).

Present hydrazide compound (19)

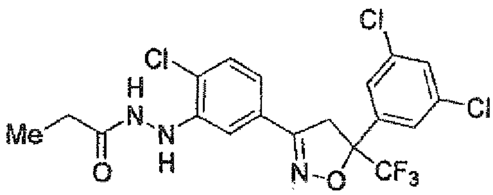

Melting point: 92°C

Production Example 20

In 2 mL of tetrahydrofuran were dissolved 260 mg of the crude 5-[5-(3,5-dichlorophenyl)-5-trifluoromethyl-4,5-dihydroisoxazol-3-yl]-2-chlorophenylhydrazine obtained by Reference Production Example 21 and 60 mg of triethylamine, and 150 mg of trifluoroacetic anhydride was added dropwise thereto at 0°C, and then the mixture was stirred at room temperature for 2 hours. The reaction mixture was concentrated under reduced pressure, and the resulting residue was subjected to silica gel column chromatography to obtain 175 mg of N'-{5-[5-(3,5-dichlorophenyl)-5-trifluoromethyl-4,5-dihydroi soxazol-3-yl]-2-chlorophenyl}-2,2,2-trifluoroacetohydrazide (herein referred to as the present hydrazide compound (20).).

Present hydrazide compound (20)

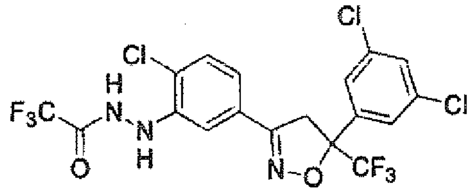

Melting point: 64°C

Production Example 21

In 4 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 95 mg of triethylamine, and 84 mg of nicotinoyl chloride hydrochloride was added thereto at 0°C, and then the mixture was stirred at room temperature for 2 hours. The reaction mixture was concentrated under reduced pressure, and the resulting residue was subjected to silica gel column chromatography to obtain 198 mg of N'-{5-[5-(3,5-dichlorophenyl)-5-trifluoromethyl-4,5-dihydroi soxazol-3-yl]-2-chlorophenyl}nicotinohydrazide (herein referred to as the present hydrazide compound (21).).

Present hydrazide compound (21)

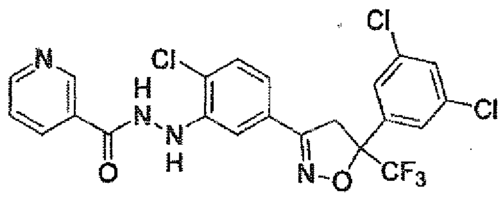

Melting point: 205°C

Production Example 22

In 4 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 95 mg of triethylamine, and 84 mg of isonicotinoyl chloride hydrochloride was added thereto at 0°C, and then the mixture was stirred at room temperature for 2 hours. The reaction mixture was concentrated under reduced pressure, and the resulting residue was subjected to silica gel column chromatography to obtain 169 mg of N'-{5-[5-(3,5-dichlorophenyl)-5-trifluoromethyl-4,5-dihydroi soxazol-3-yl]-2-chlorophenyl}isonicotinohydrazide (herein referred to as the present hydrazide compound (22).).

Present hydrazide compound (22)

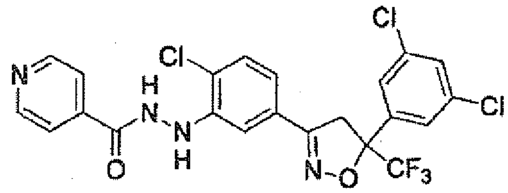

Melting point: 115°C

Production Example 23

In 4 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 52 mg of triethylamine, and 62 mg of pivaloyl chloride was added thereto at 0°C, and then the mixture was stirred at room temperature for 2 hours. The reaction mixture was concentrated under reduced pressure, and the resulting residue was subjected to silica gel column chromatography to obtain 198 mg of N'-{5-[5-(3,5-dichlorophenyl)-5-trifluoromethyl-4,5-dihydroi soxazol-3-yl]-2-chlorophenyl}pivalohydrazide (herein referred to as the present hydrazide compound (23).).

Present hydrazide compound (23)

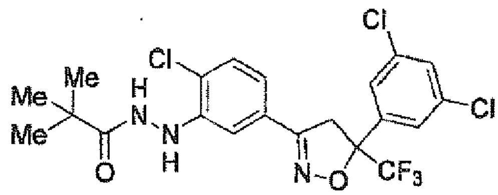

Melting point: 106°C

Production Example 24

In 4 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 57 mg of triethylamine, and 67 mg of dimethylcarbamoyl chloride was added thereto at 0°C, and then the mixture was stirred at room temperature for 2 hours. The reaction mixture was concentrated under reduced pressure, and the resulting residue was subjected to silica gel column chromatography to obtain 70 mg of 4,4-dimethyl-1-{5-[5-(3,5-dichlorophenyl)-5-trifluoromethyl-4,5-dihydroisoxazol-3-yl]-2-chlorophenyl}semicarbazide (herein referred to as the present hydrazide compound (24).).

Present hydrazide compound (24)

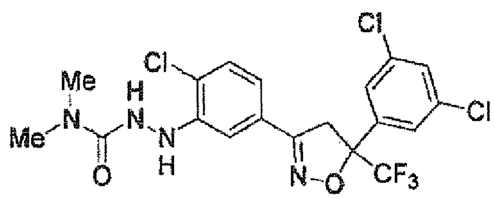

Melting point: 212°C

Production Example 25

In 4 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 51 mg of triethylamine, and 37 mg of ethyl isocyanate was added thereto at 0°C, and then the mixture was stirred at room temperature for 2 hours. The reaction mixture was concentrated under reduced pressure, and the resulting residue was subjected to silica gel column chromatography to obtain 149 mg of 4-ethyl-1-{5-[5-(3,5-diohlorophenyl)-5-trifluoromethyl-4,5-d ihydroisoxazol-3-yl]-2-chlorophenyl}semicarbazide (herein referred to as the present hydrazide compound (25).).

Present hydrazide compound (25)

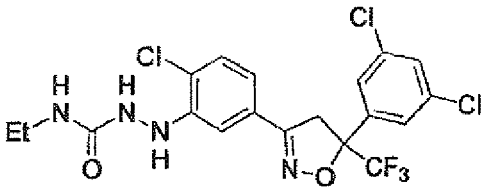

Melting point: 102°C

Production Example 26

In 4 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 57 mg of triethylamine, and 79 mg of phenylacetyl chloride was added thereto at 0°C, and then the mixture was stirred at room temperature for 2 hours. The reaction mixture was concentrated under reduced pressure, and the resulting residue was subjected to silica gel column chromatography to obtain 211 mg of N'-{5-[5-(3,5-dichlorophenyl)-5-trifluoromethyl-4,5-dihydroi soxazol-3-yl]-2-chlorophenyl}phenylacetohydrazide (herein referred to as the present hydrazide compound (26).).

Present hydrazide compound (26)

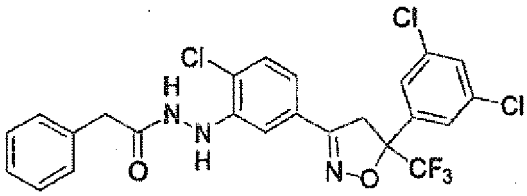

Melting point: 173°C

Production Example 27

In 2 mL of N,N-dimethylformamide were dissolved 500 mg of tert-butyl N-{2-chloro-5-[5-(3,5-dichlorophenyl)-5-trifluoromethyl-4,5-dihydroisoxazol-3-yl]phenyl}carbazate obtained by Reference Production Example 23 and 122 mg of acrylic acid, and 319 mg of 1-ethyl-3-(3-dimethylaminopropyl) carbodiimide hydrochloride was added thereto at room temperature, and then the mixture was stirred for 3 hours. Water was added to the reaction mixture, and the mixture was extracted with ethyl acetate. The organic layer was dried over anhydrous sodium sulfate and then concentrated under reduced pressure. The resulting residue was subjected to silica gel column chromatography to obtain 384 mg of tert-butyl N'-acryloyl-N-{2-chloro-5-[5-(3,5-dichlorophenyl)-5-trifluor omethyl-4,5-dihydroisoxazol-3-yl]phenyl}carbazate (herein referred to as the present hydrazide compound (27).).

Present hydrazide compound (27)

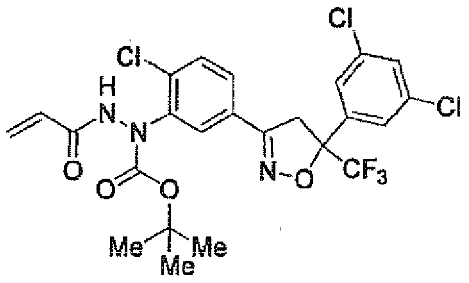

Melting point: 119°C

Production Example 28

To 309 mg the of the tert-butyl N'-acryloyl-N-{2-chloro-5-[5-(3,5-dichlorophenyl)-5-trifluor omethyl-4,5-dihydroisoxazol-3-yl]phenyl}carbazate obtained by Production Example 27 was added 2 mL of trifluoroacetic acid at room temperature, and the mixture was stirred at the same temperature for 2 hours. The reaction mixture was concentrated under reduced pressure, and ethyl acetate was added to the residue. The organic layer was washed with a saturated sodium bicarbonate aqueous solution. The organic layer was dried over anhydrous sodium sulfate and then concentrated under reduced pressure. The resulting residue was subjected to silica gel column chromatography to obtain 238 mg of N'-{2-chloro-5-[5-(3,5-dichlorophenyl)-5-trifluoromethyl-4,5 -dihydroisoxazol-3-yl]phenyl}acrylohydrazide (herein referred to as the present hydrazide compound (28).).

Present hydrazide compound (28)

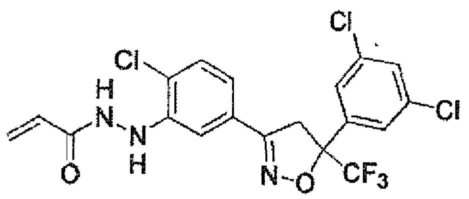

Melting point: 90°C

Production Example 29

In 2.0 mL of tetrahydrofuran were dissolved 200 mg of the N-methyl-N-{2-chloro-5-[5-(3,5-dichlorophenyl)-5-trifluorome thyl-4,5-dihydroisoxazol-3-yl]phenyl}hydrazine obtained by Reference Production Example 28 and 57 mg of triethylamine, and 58 mg of cyclopropanecarbonyl chloride was added dropwise thereto at room temperature. After stirring for 30 minutes at the same temperature, the reaction mixture was concentrated under reduced pressure. The resulting residue was subjected to silica gel column chromatography to obtain 225 mg of N'-methyl-N'-{2-chloro-5-[5-(3,5-dichlorophenyl)-5-trifluoro methyl-4,5-dihydroisoxazol-3-yl]phenyl}cyclopropanecarbohydr azide (herein referred to as the present hydrazide compound (29).).

Present hydrazide compound (29)

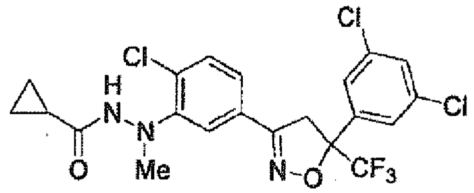

Melting point: 176°C

Production Example 30

In 2.0 mL of tetrahydrofuran were dissolved 200 mg of the N-methyl-N-{2-chloro-5-[5-(3,5-dichlorophenyl)-5-trifluorome thyl-4,5-dihydroisoxazol-3-yl]phenyl}hydrazine obtained by Reference Production Example 28 and 57 mg of triethylamine, and 81 mg of 3,3,3-trifluoropropionyl chloride was added dropwise thereto at room temperature. After stirring for 30 minutes at the same temperature, the reaction mixture was concentrated under reduced pressure. The resulting residue was subjected to silica gel column chromatography to obtain 241 mg of N'-methyl-N'-{2-chloro-5-[5-(3,5-dichlorophenyl)-5-trifluoro methyl-4,5-dihydroisoxazol-3-yl]phenyl}-3,3,3-trifluoropropi onohydrazide (herein referred to as the present hydrazide compound (30).).

Present hydrazide compound (30)

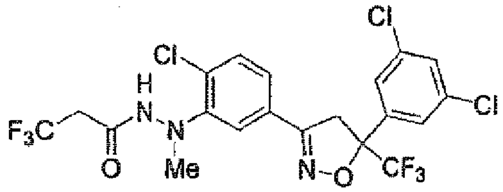

Melting point: 81°C

Production Example 31

In 2.0 mL of tetrahydrofuran were dissolved 200 mg of the N-methyl-N-{2-chloro-5-[5-(3,5-dichlorophenyl)-5-trifluorome thyl-4,5-dihydroisoxazol-3-yl]phenyl}hydrazine obtained by Reference Production Example 28 and 57 mg of triethylamine, and 88 mg of 4,4,4-trifluorobutyryl chloride was added dropwise thereto at room temperature. After stirring for 1 hour at the same temperature, the reaction mixture was concentrated under reduced pressure. The resulting residue was subjected to silica gel column chromatography to obtain 258 mg of N'-methyl-N'-{2-chloro-5-[5-(3,5-dichlorophenyl)-5-trifluoromethyl-4,5 -dihydroisoxazol-3-yl]phenyl}-4,4,4-trifluorobutyrohydrazide (herein referred to as the present hydrazide compound (31).).

Present hydrazide compound (31)

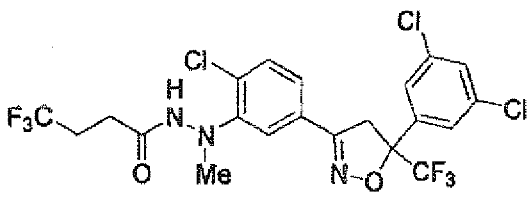

Melting point: 77°C

Production Example 32

In 2.0 mL of tetrahydrofuran were dissolved 200 mg of the N-methyl-N-{2-chloro-5-[5-(3,5-dichlorophenyl)-5-trifluorome thyl-4,5-dihydroisoxazol-3-yl]phenyl}hydrazine obtained by Reference Production Example 28 and 57 mg of triethylamine, and 43 mg of acetyl chloride was added dropwise thereto at room temperature. After stirring for 1 hour at the same temperature, the reaction mixture was concentrated under reduced pressure. The resulting residue was subjected to silica gel column chromatography to obtain 204 mg of N'-methyl-N'-{2-chloro-5-[5-(3,5-dichlorophenyl)-5-trifluoro methyl-4,5-dihydroisoxazol-3-yl]phenyl}acetohydrazide (herein referred to as the present hydrazide compound (32).).

Present hydrazide compound (32)

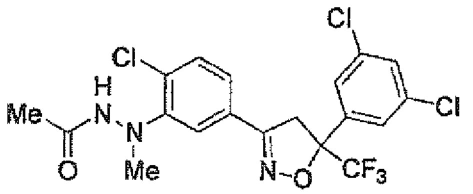

$^1$H-NMR ($CDCl_3$) δ: 7.88-7.24(7H, m),4.09-4.04(1H,m), 3.70-3.66(1H, m), 3.29-3.07(3H, m), 2.17-1.91(3H, m).

Production Example 33

In 4 mL of tetrahydrofuran were dissolved 525 mg of the tert-butyl N-{2-chloro-5-[5-(3,5-diohlorophenyl)-5-trifluoromethyl-4,5-dihydroisoxazol-3-yl]phenyl}carbazate obtained by Reference Production Example 23 and 121 mg of triethylamine, and 223 mg of 4-bromobutyryl chloride was added dropwise thereto at room temperature, and then the mixture was stirred for 1 hour. Water was added to the reaction mixture, and the mixture was extracted with ethyl acetate. The organic layer was dried over anhydrous sodium sulfate and then concentrated under reduced pressure to obtain 699 mg of crude tert-butyl N'-(4-bromobutyryl)-N-{2-chloro-5-[5-(3,5-dichlorophenyl)-5-trifluoromethyl-4,5-dihydroisoxazol-3-yl]phenyl}carbazate (herein referred to as the present hydrazide compound (33).).

Present hydrazide compound (33)

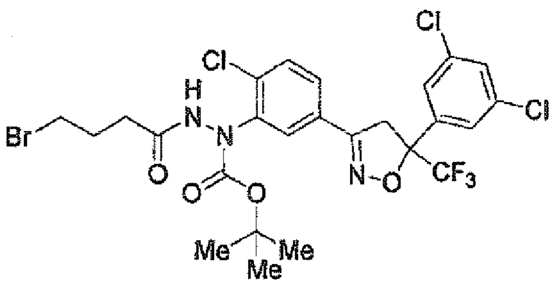

Production Example 34

To 309 mg of the tert-butyl N'-(4-bromobutyryl)-N-{2-chloro-5-[5-(3,5-dichlorophenyl)-5-trifluoromethyl-4,5-dihydroisoxazol-3-yl]phenyl}carbazate obtained by Production Example 33 was added 2 mL of trifluoroacetic acid, and the mixture was stirred at room temperature for 2 hours. The reaction mixture was concentrated under reduced pressure, and ethyl acetate was added to the residue. The organic layer was washed with a saturated sodium bicarbonate aqueous solution. The organic layer was dried over anhydrous sodium sulfate and then concentrated under reduced pressure. The resulting residue was subjected to silica gel column chromatography to obtain 573 mg of N'-{2-chloro-5-[5-(3,5-dichlorophenyl)-5-trifluoromethyl-4,5 -dihydroisoxazol-3-yl]phenyl}-4-bromobutyrohydrazide (herein referred to as the present hydrazide compound (34).).

Present hydrazide compound (34)

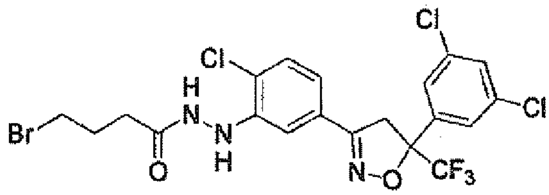

Melting point: 59°C

Production Example 35

In 2 mL of N,N-dimethylformamide were dissolved 350 mg of the tert-butyl N-{2-chloro-5-[5-(3,5-dichlorophenyl)-5-trifluoromethyl-4,5-dihydroisoxazol-3-yl]phenyl}carbazate obtained by Reference Production Example 23 and 187 mg of 5,5,5-trifluoropentanoic acid, and 153 mg of 1-ethyl-3-(3-dimethylaminopropyl)carbodiimide hydrochloride was added thereto at room temperature, and then the mixture was stirred for 3 hours. Water was added to the reaction mixture, and the mixture was extracted with ethyl acetate. The organic layer was dried over anhydrous sodium sulfate and then concentrated under reduced pressure to obtain 350 mg of crude tert-butyl N'-(5,5,5-trifluoropentanoyl)-N-{2-chloro-5-[5-(3,5-dichlorophenyl)-5-trifluoromethyl-4,5-di hydroisoxazol-3-yl]phenyl}carbazate (herein referred to as the present hydrazide compound (35).).

Present hydrazide compound (35)

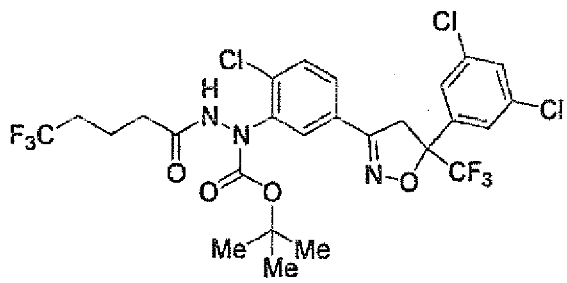

Production Example 36

To 350 mg of the crude tert-butyl N'-(5,5,5-trifluoropentanoyl)-N-{2-chloro-5-[5-(3,5-dichloro phenyl)-5-trifluoromethyl-4,5-dihydroisoxazol-3-yl]phenyl}ca rbazate obtained by Production Example 35 was added 2 mL of trifluoroacetic acid at room temperature, and the mixture was stirred at the same temperature for 2 hours. The reaction mixture was concentrated under reduced pressure, and ethyl acetate was added to the residue. The organic layer was washed with a saturated sodium bicarbonate aqueous solution. The organic layer was dried over anhydrous sodium sulfate and then concentrated under reduced pressure. The resulting residue was subjected to silica gel column chromatography to obtain 269 mg of N'-{2-chloro-5-[5-(3,5-dichlorophenyl)-5-trifluoromethyl-4,5 -dihydroisoxazol-3-yl]phenyl}-5,5,5-trifluoropentanohydrazid e (herein referred to as the present hydrazide compound (36).).

Present hydrazide compound (36)

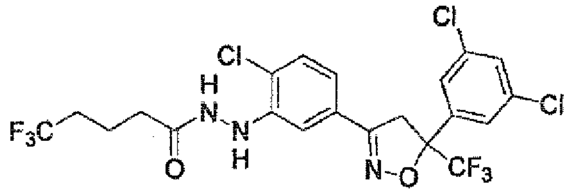

Melting point: 163°C

Production Example 37

In 2 mL of tetrahydrofuran were dissolved 367 mg of the tert-butyl N-{2-chloro-5-[5-(3,5-dichlorophenyl)-5-trifluoromethyl-4,5-dihydroisoxazol-3-yl]phenyl}carbazate obtained by Reference Production Example 23 and 84 mg of triethylamine, and 91 mg of methoxyacetyl chloride was added dropwise thereto at room temperature, and then the mixture was stirred for 1 hour. Ethyl acetate was added to the reaction mixture, and the organic layer was washed with water. The organic layer was dried over anhydrous sodium sulfate and then concentrated under reduced pressure to obtain 377 mg of crude tert-butyl N'-methoxyacetyl-N-{2-chloro-5-[5-(3,5-dichlorophenyl)-5-tri fluoromethyl-4,5-dihydroisoxazol-3-yl]phenyl}carbazate (herein referred to as the present hydrazide compound (37).).

Present hydrazide compound (37)

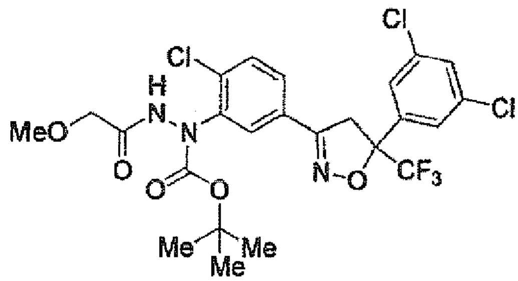

Production Example 38

To 309 mg of the crude tert-butyl N'-methoxyacetyl-N-{2-chloro-5-[5-(3,5-dichlorophenyl)-5-tri fluoromethyl-4,5-dihydroisoxazol-3-yl]phenyl}carbazate obtained by Production Example 37 was added 2 mL of trifluoroacetic acid at room temperature, and the mixture was stirred at the same temperature for 2 hours. The reaction mixture was concentrated under reduced pressure, and ethyl acetate was added to the residue. The organic layer was washed with a saturated sodium bicarbonate aqueous solution. The organic layer was dried over anhydrous sodium sulfate and then concentrated under reduced pressure. The resulting residue was subjected to silica gel column chromatography to obtain 276 mg of N'-{2-chloro-5-[5-(3,5-dichlorophenyl)-5-trifluoromethyl-4,5 -dihydroisoxazol-3-yl]phenyl}methoxyacetohydrazide (herein referred to as the present hydrazide compound (38).).

Present hydrazide compound (38)

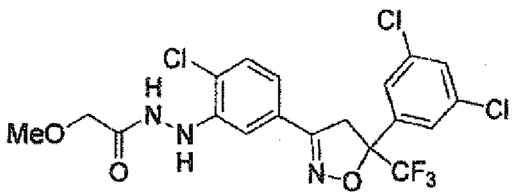

Melting point: 178°C

Production Example 39

In 4 mL of tetrahydrofuran were dissolved 524 mg of the tert-butyl N-{2-chloro-5-[5-(3,5-dichlorophenyl)-5-trifluoromethyl-4,5-dihydroisoxazol-3-yl]phenyl}carbazate obtained by Reference Production Example 23 and 121 mg of triethylamine, and 186 mg of 3-chloropivaloyl chloride was added dropwise thereto at room temperature, and then the mixture was stirred at the same temperature for 1 hour. Ethyl acetate was added to the reaction mixture, and the organic layer was washed with water. The organic layer was dried over anhydrous sodium sulfate and then concentrated under reduced pressure to obtain 643 mg of crude tert-butyl N'-(3-chloropivaloyl)-N-{2-chloro-5-[5-(3,5-dichlorophenyl)-5-trifluoromethyl-4,5-dihydroisoxazol-3-yl]phenyl}carbazate (herein referred to as the present hydrazide compound (39).).

Present hydrazide compound (39)

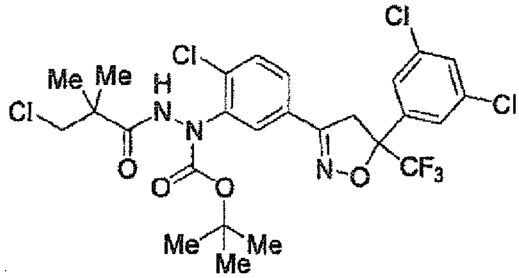

Production Example 40

To 643 mg of the crude tert-butyl N'-(3-chloropivaloyl)-N-{2-chloro-5-[5-(3,5-dichlorophenyl)-5-trifluoromethyl-4,5-dihydroisoxazol-3-yl]phenyl}carbazate obtained by Production Example 39 was added 5 mL of trifluoroacetic acid, and the mixture was stirred at room temperature for 2 hours. The reaction mixture was concentrated under reduced pressure, and ethyl acetate was added to the residue. The organic layer was washed with a saturated sodium bicarbonate aqueous solution. The organic layer was dried over anhydrous sodium sulfate and then concentrated under reduced pressure to obtain 520 mg of N'-{2-chloro-5-[5-(3,5-dichlorophenyl)-5-trifluoromethyl-4,5 -dihydroisoxazol-3-yl]phenyl}-3-chloropivalohydrazide (herein referred to as the present hydrazide compound (40).).

Present hydrazide compound (40)

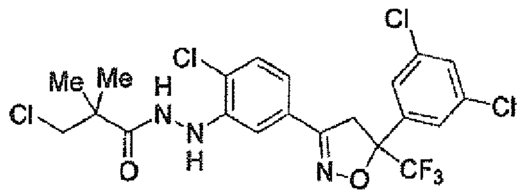

$^1$H-NMR($CDCl_3$)δ: 7.68(1H, d, J=3.2 Hz), 7.47(2H, d, J=1.7 Hz), 7.42(1H, t, J=2.0 Hz), 7.32(1H, d, J=8.3 Hz), 7.26(1H, d, J=1.5 Hz), 7.06(1H, dd, J=8.2, 2.1 Hz), 6.45(1H, d, J=3.2 Hz), 4.00(1H, d, J=17.6 Hz), 3.68(2H, s), 3.63(1H, d, J=17.6 Hz), 1.42(3H, s), 1.41(3H, s).

Production Example 41

In 4 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 56 mg of triethylamine, and 103 mg of 4-nitrobenzoyl chloride was added thereto at 0 °C, and then the mixture was stirred at room temperature for 10 minutes. The reaction mixture was concentrated under reduced pressure, and the resulting residue was subjected to silica gel column chromatography to obtain 166 mg of N'-{5-[5-[5-(3,5-dichlorophenyl)-5-trifluoromethyl-4,5-dihydroi soxazol-3-yl]-2-chlorophenyl}-4-nitrobenzohydrazide (herein referred to as the present hydrazide compound (41).).

Present hydrazide compound (41)

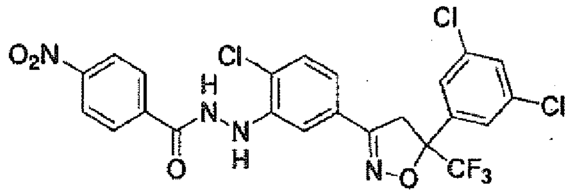

Melting point: 231°C

Production Example 42

In 4 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 57 mg of triethylamine, and 50 mg of butyryl chloride was added thereto at 0°C, and then the mixture was stirred at room temperature for 20 minutes. The reaction mixture was concentrated under reduced pressure, and the resulting residue was subjected to silica gel column chromatography to obtain 121 mg of N'-{5-[5-(3,5-dichlorophenyl)-5-trifluoromethyl-4,5-dihydroi soxazol-3-yl]-2-chlorophenyl}butyrohydrazide (herein referred to as the present hydrazide compound (42).).

Present hydrazide compound (42)

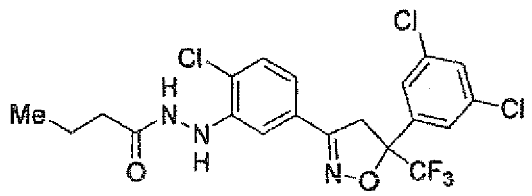

Melting point: 82°C

Production Example 43

In 4 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 57 mg of triethylamine, and 57 mg of pentanoyl chloride was added thereto at 0°C, and then the mixture was stirred at room temperature for 2 hours. The reaction mixture was concentrated under reduced pressure, and the resulting residue was subjected to silica gel column chromatography to obtain 164 mg of N'-{5-[5-(3,5-dichlorophenyl)-5-trifluoromethyl-4,5-dihydroi soxazol-3-yl]-2-chlorophenyl}pentanohydrazide (herein referred to as the present hydrazide compound (43).).

Present hydrazide compound (43)

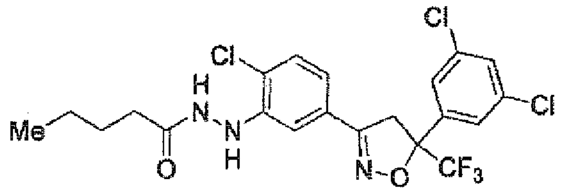

Melting point: 89°C

Production Example 44

In 4 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 57 mg of triethylamine, and 63 mg of hexanoyl chloride was added thereto at 0°C, and then the mixture was stirred at room temperature for 20 minutes. The reaction mixture was concentrated under reduced pressure, and the resulting residue was subjected to silica gel column
chromatography to obtain 134 mg of N'-{5-[5-(3,5-dichlorophenyl)-5-trifluoromethyl-4,5-dihydroi soxazol-3-yl]-2-chlorophenyl}hexanohydrazide (herein referred to as the present hydrazide compound (44).).

Present hydrazide compound (44)

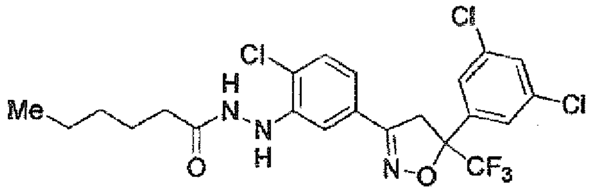

Melting point: 80°C

Production Example 45

In 4 mL of tetrahydrofuran were dissolved 200 mg of the crude 5-[5-(3,5-dichlorophenyl)-5-trifluoromethyl-4,5-dihydroisoxazol-3-yl]-2-chlorophenylhydrazine obtained by Reference Production Example 21 and 57 mg of triethylamine, and 78 mg of cinnamoyl chloride was added thereto at 0°C, and then the mixture was stirred at room temperature for 20 minutes. The reaction mixture was concentrated under reduced pressure, and the resulting residue was subjected to silica gel column chromatography to obtain 161 mg of N'-{5-[5-(3,5-dichlorophenyl)-5-trifluoromethyl-4,5-dihydroi soxazol-3-yl]-2-chlorophenyl}-3-phenylacrylohydrazide (herein referred to as the present hydrazide compound (45).).

Present hydrazide compound (45)

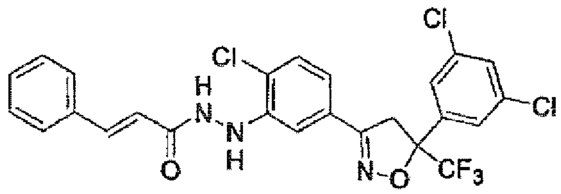

Melting point: 205°C

Production Example 46

In 2 mL of tetrahydrofuran were dissolved 247 mg of the tert-butyl N-{2-chloro-5-[5-(3,5-dichlorophenyl)-5-trifluoromethyl-4,5-dihydroisoxazol-3-yl]phenyl}carbazate obtained by Reference Production Example 23 and 56 mg of triethylamine, and 95 mg of 4-methoxybenzoyl chloride was added thereto at room temperature, and then the mixture was stirred for 1 hour. Ethyl acetate was added to the reaction mixture, and the organic layer was washed with water. The organic layer was dried over anhydrous sodium sulfate and then concentrated under reduced pressure to obtain 240 mg of crude tert-butyl N'-(4-methoxybenzoyl)-N-{2-chloro-5-[5-(3,5-dichlorophenyl)-5-trifluoromethyl-4,5-dihydroisoxazol-3-yl]phenyl}carbazate (herein referred to as the present hydrazide compound (46).).

Present hydrazide compound (46)

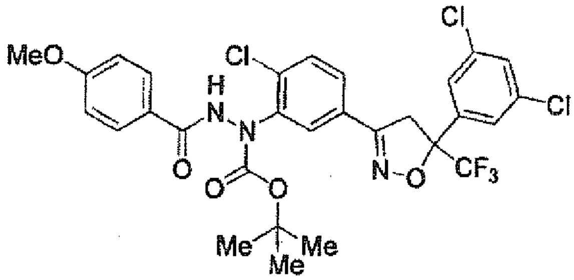

Production Example 47

To 240 mg of the crude tert-butyl N'-(4-methoxybenzoyl)-N-{2-chloro-5-[5-(3,5-dichlorophenyl)-5-trifluoromethyl-4,5-dihydroisoxazol-3-yl]phenyl}carbazate obtained by Production Example 46 was added 2 mL of trifluoroacetic acid, and the mixture was stirred at room temperature for 1 hour. The reaction mixture was concentrated under reduced pressure, and ethyl acetate was added to the residue. The organic layer was washed with a saturated sodium bicarbonate aqueous solution. The organic layer was dried over anhydrous sodium sulfate and then concentrated under reduced pressure to obtain 221 mg of N'-{2-chloro-5'-[5-(3,5-dichlorophenyl)-5-trifluoromethyl-4,5 -dihydroisoxazol-3-yl]phenyl}-4-methoxybenzohydrazide (herein referred to as the present hydrazide compound (47).).

Present hydrazide compound (47)

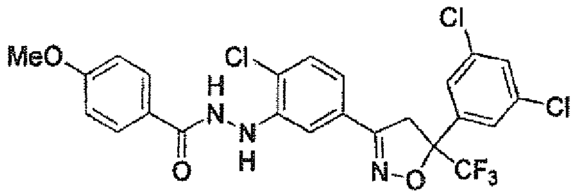

Melting point: 131°C

Production Example 48

In 4 mL of tetrahydrofuran were dissolved 1.31 g of the 5-[5-(3,5-dichlorophenyl)-5-trifluoromethyl-4,5-dihydroisoxa zol-3-yl]-2-ethylphenylhydrazine obtained by Reference Production Example 31 and 200 mg of triethylamine, and 290 mg of 3, 3, 3-trifluoropropionyl chloride was added dropwise thereto at room temperature. After stirring at the same temperature for 1 hour, the reaction mixture was concentrated under reduced pressure. The resulting residue was subjected to silica gel column chromatography to obtain 426 mg of N'-{5-[5-(3,5-dichlorophenyl)-5-trifluoromethyl-4,5-dihydroi soxazol-3-yl]-2-ethylphenyl}-3,3,3-propionohydrazide (herein referred to as the present hydrazide compound (48).).

Present hydrazide compound (48)

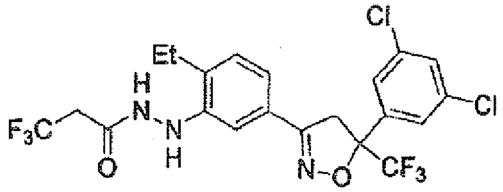

Melting point: 138.7°C

Production Example 49

In 2 mL of tetrahydrofuran, 200 mg of the compound obtained by Reference Production Example 33 and 65 mg of triethylamine were dissolved, and 54 mg of cyclopropanecarbonyl chloride was added dropwise thereto at room temperature, and then the mixture was stirred at the same temperature for 1 hour. Ethyl acetate was added to the reaction mixture, and the organic layer was washed with water. The organic layer was dried over anhydrous sodium sulfate and then concentrated under reduced pressure. The resulting residue was subjected to silica gel column chromatography to obtain 216 mg of a compound shown by the following formula (herein referred to as the present hydrazide compound (49).).

Present hydrazide compound (49)

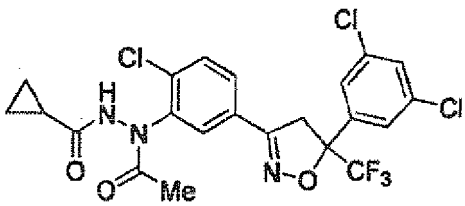

Melting point: 160°C

Production Example 50

In 2 mL of tetrahydrofuran, 200 mg of the compound obtained by Reference Production Example 33 and 65 mg of triethylamine were dissolved, and 55 mg of methoxyacetyl chloride was added dropwise thereto at room temperature, and then the mixture was stirred at the same temperature for 1 hour. Ethyl acetate was added to the reaction mixture, and the organic layer was washed with water. The organic layer was dried over anhydrous sodium sulfate and then concentrated under reduced pressure. The resulting residue was subjected to silica gel column chromatography to obtain 130 mg of a compound shown by the following formula (herein referred to as the present hydrazide compound (50).).

Present hydrazide compound (50)

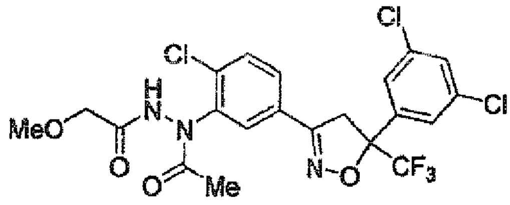

Melting point: 164°C

Production Example 51

In 2 mL of tetrahydrofuran, 200 mg of the compound obtained by Reference Production Example 33 and 65 mg of triethylamine were dissolved, and 40 mg of acetyl chloride was added dropwise thereto at room temperature, and then the mixture was stirred at the same temperature for 1 hour. Ethyl acetate was added to the reaction mixture, and the organic layer was washed with water. The organic layer was dried over anhydrous sodium sulfate and then concentrated under reduced pressure. The resulting residue was subjected to silica gel column chromatography to obtain 140 mg of a compound shown by the following formula (herein referred to as the present hydrazide compound (51).).

Present hydrazide compound (51)

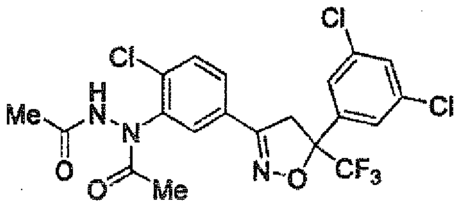

Melting point: 108°C

Production Example 52

In 2 mL of tetrahydrofuran, 200 mg of the compound obtained by Reference Production Example 33 and 65 mg of triethylamine were dissolved, and 72 mg of benzoyl chloride was added dropwise thereto at room temperature, and then the mixture was stirred at the same temperature for 1 hour. Ethyl acetate was added to the reaction mixture, and the organic layer was washed with water. The organic layer was dried over anhydrous sodium sulfate and then concentrated under reduced pressure. The resulting residue was subjected to silica gel column chromatography to obtain 281 mg of a compound shown by the following formula (herein referred to as the present hydrazide compound (52).).

Present hydrazide compound (52)

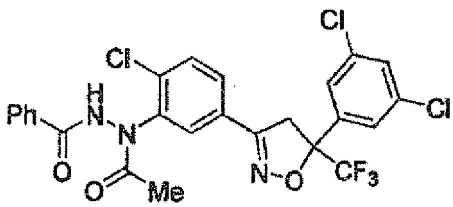

Melting point: 107°C

Production Example 53

In 2 mL of tetrahydrofuran, 200 mg of the compound obtained by Reference Production Example 33 and 65 mg of triethylamine were dissolved, and 90 mg of 4,4,4-trifluorobutyryl chloride was added dropwise thereto at room temperature, and then the mixture was stirred at the same temperature for 1 hour. Ethyl acetate was added to the reaction mixture, and the organic layer was washed with water. The organic layer was dried over anhydrous sodium sulfate and then concentrated under reduced pressure. The resulting residue was subjected to silica gel column chromatography to obtain 207 mg of a compound shown by the following formula (herein referred to as the present hydrazide compound (53).).

Present Hydrazide compound (53)

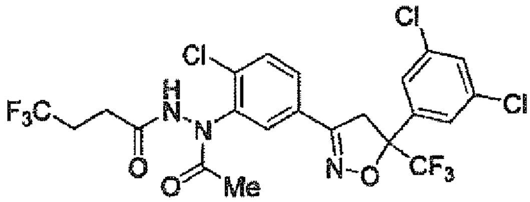

Melting point: 117°C

Production Example 54

In 2 mL of tetrahydrofuran, 200 mg of the compound obtained by Reference Production Example 35 and 58 mg of triethylamine were dissolved, and 38 mg of acetyl chloride was added dropwise thereto at room temperature, and then the mixture was stirred at the same temperature for 1 hour. Ethyl acetate was added to the reaction mixture, and the organic layer was washed with water. The organic layer was dried over anhydrous sodium sulfate and then concentrated under reduced pressure. The resulting residue was subjected to silica gel column chromatography to obtain 156 mg of a compound shown by the following formula (herein referred to as the present hydrazide compound (54).).

Present hydrazide compound (54)

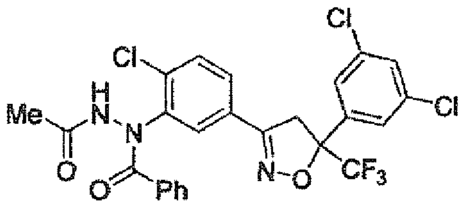

Melting point: 142°C

Production Example 55

In 2 mL of tetrahydrofuran, 200 mg of the compound obtained by Reference Production Example 35 and 51 mg of triethylamine were dissolved and 54 mg of cyclopropanecarbonyl chloride was added dropwise thereto at room temperature, and then the mixture was stirred at the same temperature for 1 hour. Ethyl acetate was added to the reaction mixture, and the organic layer was washed with water. The organic layer was dried over anhydrous sodium sulfate and then concentrated under reduced pressure. The resulting residue was subjected to silica gel column chromatography to obtain 103 mg of a compound shown by the following formula (herein referred to as the present hydrazide compound (55).).

Present hydrazide compound (55)

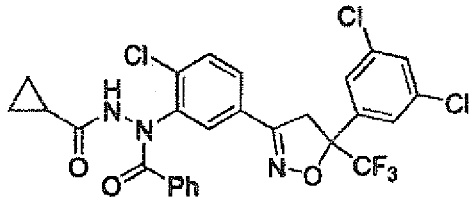

Melting point: 172°C

Production Example 56

In 2 mL of tetrahydrofuran, 200 mg of the compound obtained by Reference Production Example 35 and 65 mg of triethylamine were dissolved, and 78 mg of 4,4,4-trifluorobutyryl chloride was added dropwise thereto at room temperature, and then the mixture was stirred at the same temperature for 1 hour. Ethyl acetate was added to the reaction mixture, and the organic layer was washed with water. The organic layer was dried over anhydrous sodium sulfate and then concentrated under reduced pressure. The resulting residue was subjected to silica gel column chromatography to obtain 211 mg of a compound shown by the following formula (herein referred to as the present hydrazide compound (56).).

Present hydrazide compound (56)

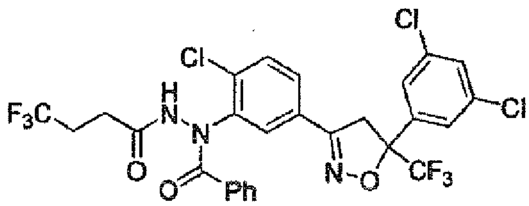

Melting point: 157°C

Production Example 57

In 2 mL of tetrahydrofuran, 300 mg of the compound obtained by Reference Production Example 35 and 85 mg of triethylamine were dissolved, and 79 mg of methoxyacetyl chloride was added dropwise thereto at room temperature, and then the mixture was stirred at the same temperature for 1 hour. Ethyl acetate was added to the reaction mixture, and the organic layer was washed with water. The organic layer was dried over anhydrous sodium sulfate and then concentrated under reduced pressure. The resulting residue was subjected to silica gel column chromatography to obtain 297 mg of a compound shown by the following formula (herein referred to as the present hydrazide compound (57).).

Present hydrazide compound (57)

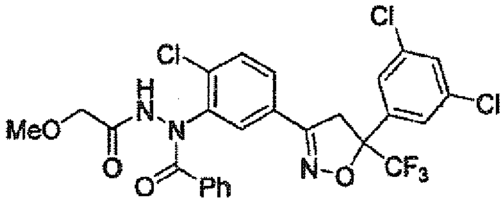

Melting point: 92°C

Production Example 58

In 2 mL of tetrahydrofuran, 241 mg of the crude compound obtained by Reference Production Example 21 was dissolved, and 82 mg of phenyl isocyanate was added dropwise thereto at room temperature, and then the mixture was stirred at room temperature for 3 hours. The reaction mixture was concentrated under reduced pressure, and the resulting residue was subjected to silica gel column chromatography to obtain 187 mg of a compound shown by the following formula (herein referred to as the present hydrazide compound (58).).

Present hydrazide compound (58)

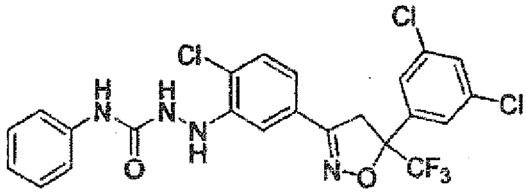

Melting point: 131°C

Production Example 59

In 6 mL of tetrahydrofuran, 200 of the crude compound obtained by Reference Production Example 21 was dissolved, and 85 mg of 4-methoxyphenyl isocyanate was added dropwise thereto at room temperature, and then the mixture was stirred at room temperature for 3 hours. The reaction mixture was concentrated under reduced pressure, and the resulting residue was subjected to silica gel column chromatography to obtain 191 mg of a compound shown by the following formula (herein referred to as the present hydrazide compound (59).).

Present hydrazide compound (59)

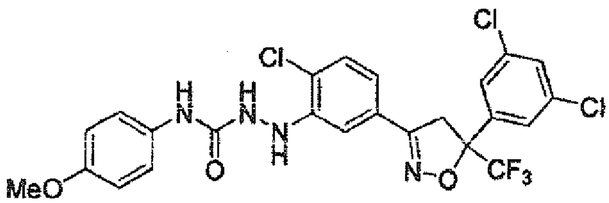

Melting point: 131°C

Production Example 60

In 5 mL of tetrahydrofuran, 196 mg of the crude compound obtained by Reference Production Example 21 was dissolved, and 136 mg of 4-nitrophenyl isocyanate was added dropwise thereto at room temperature, and then the

mixture was stirred at room temperature for 3 hours. The reaction mixture was concentrated under reduced pressure, and the resulting residue was subjected to silica gel column chromatography to obtain 110 mg of a compound shown by the following formula (herein referred to as the present hydrazide compound (60).).

Present hydrazide compound (60)

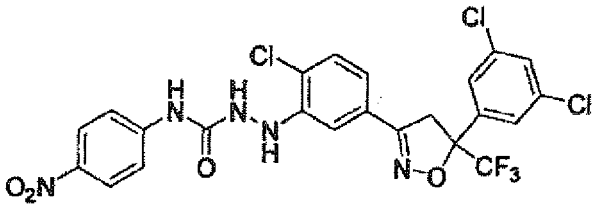

Melting point: 201°C

Production Example 61

In 6 mL of tetrahydrofuran, 220 mg of the crude compound obtained by Reference Production Example 21 was dissolved, and 79 mg of cyclohexyl isocyanate was added dropwise thereto at room temperature, and then the mixture was stirred at room temperature for 3 hours. The reaction mixture was concentrated under reduced pressure, and the resulting residue was subjected to silica gel column chromatography to obtain 100 mg of a compound shown by the following formula (herein referred to as the present hydrazide compound (61).).

Present hydrazide compound (61)

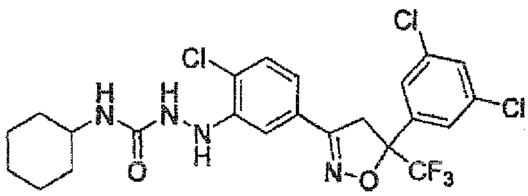

Melting point: 101°C

Production Example 62

In 6 mL of tetrahydrofuran, 340 mg of the crude compound obtained by Reference Production Example 21 was dissolved, and 173 mg of cyclohexyl isothiocyanate was added thereto at room temperature, and then the mixture was stirred at room temperature for 3 hours. The reaction mixture was concentrated under reduced pressure, and the resulting residue was subjected to silica gel column chromatograph to obtain 135 mg of a compound shown by the following formula (herein referred to as the present hydrazide compound (62).).

Present hydrazide compound (62)

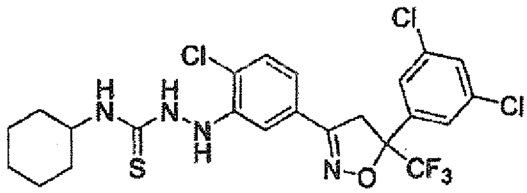

Melting point: 126°C

Production Example 63

In 6 mL of tetrahydrofuran, 240 mg of the crude compound obtained by Reference Production Example 21 was dissolved, and 114 mg of phenyl isothiocyanate was added dropwise thereto at room temperature, and then the mixture was stirred at room temperature for 3 hours. The reaction mixture was concentrated under reduced pressure, and the resulting residue was subjected to silica gel column chromatography to obtain 200 mg of a compound shown by the following formula (herein referred to as the present hydrazide compound (63).).

Present hydrazide compound (63)

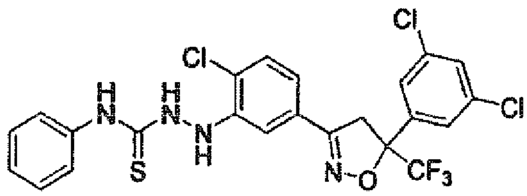

Melting point: 213°C

Next, reference production examples concerning the production of production intermediates will be given below.

Reference Production Example 1

In 15 mL of N,N-dimethylformamide, 249 mg of 3-nitrobenzaldoxime was dissolved, and 201 mg of N-chlorosuccinimide was added thereto at room temperature, and then the mixture was stirred at 60°C for 1 hour. To the reaction solution that had been cooled to room temperature, 362 mg of 2-(3,5-dichlorophenyl)-3,3,3-trifluoro-1-propene, followed by 152 mg of triethylamine, was added and the mixture was stirred at room temperature for 6 hours. Water was added to the reaction mixture, and the mixture was extracted with ethyl acetate. The organic layer was dried over anhydrous sodium sulfate and then concentrated under reduced pressure. The resulting residue was subjected to silica gel column chromatography to obtain 232 mg of 3-[5-(3,5-dichlorophenyl)-5-trifluoromethyl-4,5-dihydroisoxazol-3-yl]nitrobenzene.

3-[5-(3,5-dichlorophenyl)-5-trifluoromethyl-4,5-dihydr oi-soxazol-3-yl]nitrobenzene

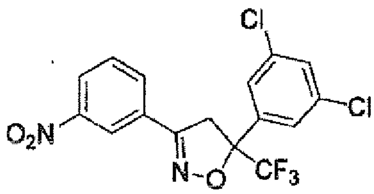

$^1$H-NMR($CBCl_3$) $\delta$: 8.43(1H, brs), 8.34-8.32(1H, m), 8.10(1H, d, J=8.0 Hz), 7.67-7.65(1H, m), 7.52(2H, s), 7.45-7.45(1H, m), 4.14(1H, d, J=17.3 Hz), 3.76(1H, d, J=17.3 Hz).

Reference Production Example 2

To 1 mL of a 2.5% acetic acid aqueous solution, 192 mg of iron powder (10 to 20 mesh) was added, and 1.5 mL of an ethanol suspension of 232 mg of the 3-[5-(3,5-dichlorophenyl)-5-trifluoromethyl-4,5-dihydroisoxazol-3-yl]nitrobenzene produced by Reference Production Example 1 was added thereto at 75°C. After stirring at the same temperature for 15 minutes, 300 mg of iron powder was further added, and the mixture was stirred at room temperature for 1 hour. The reaction mixture that had been cooled to room temperature was filtered and washed with ethyl acetate. Water and ethyl acetate were added to the filtrate, and the mixture was extracted. The organic layer was dried over anhydrous sodium sulfate and then concentrated under reduced pressure. The resulting residue was subjected to silica gel column chromatography to obtain 198 mg of 3-[5-(3,5-dichlorophenyl)-5-trifluoromethyl-4,5-dihydroisoxa zol-3-yl] aniline.

3-[5-(3,5-dichlorophenyl)-5-trifluoromethyl-4,5-dihydr oisoxazol-3-yl]aniline

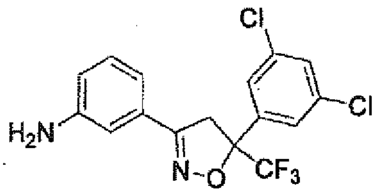

$^1$H-NMR ($CDCl_3$) δ: 7.51(2H, br s), 7.42(1H, t, J=1.9 Hz), 7.20(1H, t, J=7.8 Hz), 7.04(1H, t, J=1.9 Hz), 6.95-6.93(1H, m), 6.77-6.75(1H, m), 4.05(1H, d, J=17.1 Hz), 3.77(2H, brs), 3.66(1H, d, J=17.1 Hz).

Reference Production Example 3

In 4 mL of 1,4-dioxane, 1.0 g of 3-[5-(3,5-dichlorophenyl)-5-trifluoromethyl-4,5-dihydroisoxa zol-3-yl]aniline was dissolved, and 7 mL of concentrated hydrochloric acid was added thereto at room temperature. After stirring at the same temperature for 20 minutes, this solution was cooled to 0°C, and a solution of 184 mg of sodium nitrite dissolved in 4 mL of water was added dropwise. After stirring at the same temperature for 15 minutes, a solution of 1.11 g of tin (II) chloride dissolved in 2 mL of concentrated hydrochloric acid was added dropwise to this reaction solution. 2N sodium hydroxide was added to the reaction mixture solution, and the mixture was neutralized, and then the water layer was extracted with ethyl acetate. The organic layer was dried over anhydrous sodium sulfate and then concentrated under reduced pressure to obtain 1.0 g of crude 3-[5-(3,5-dichlorophenyl)-5-trifluoromethyl-4,5-dihydroisoxazol-3-yl]phenylhydrazine.

3-[5-(3,5-dichlorophenyl)-5-trifluoromethyl-4,5-dihydr oisoxazol-3-yl]phenylhydrazine

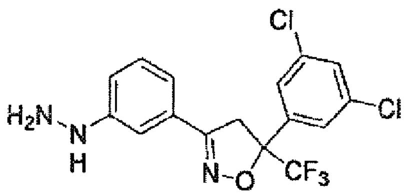

Reference Production Example 4

In 20 mL of tetrahydrofuran were dissolved 3.0 g of the 3-[5-(3,5-dichlorophenyl)-5-trifluoromethyl-4,5-dihydroisoxa zol-3-yl]aniline produced by Reference Production Example 2 and 971 mg of triethylamine, and 1.85 g of trifluoroacetic anhydride was added dropwise thereto at 0°C. After stirring at the same temperature for 15 minutes, a saturated sodium bicarbonate aqueous solution was added, and the mixture was extracted with ethyl acetate. The organic layer was dried over anhydrous sodium sulfate and then concentrated under reduced pressure to obtain 3.84 g of N-{3-[5-(3,5-dichlorophenyl)-5-trifluoromethyl-4,5-dihydroisoxazol-3-yl]phenyl}2,2,2-trifluoroacetamide.

N-{3-[5-(3,5-dichlorophenyl)-5-trifluoromethyl-4,5-dih ydroisoxazol-3-yl]phenyl}2,2,2-trifluoroacetamide

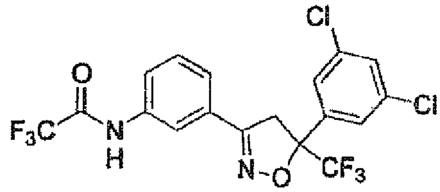

$^1$H-NMR ($CDCl_3$) δ: 8.02(1H, br s), 7.94(1H, t, J=1.8 Hz), 7.66-7.64(1H, m), 7.57-7.55(1H, m), 7.49-7.43(4H, m), 4.09 (1H, d, J=17.3 Hz), 3.71(1H, d, J=17.3 Hz).

Reference Production Example 5

In 20 mL of tetrahydrofuran were dissolved 3.84 g of the N-{3-[5-(3,5-dichlorophenyl)-5-trifluoromethyl-4,5-dihydrois oxazol-3-yl]phenyl}2,2,2-trifluoroacetamide produced by Reference Production Example 4 and 8.21 g of di-tert-butyl dicarbonate, and 195 mg of 4-dimethylpyridine was added thereto at room temperature. After stirring at the same temperature for 1.5 hours, ethyl acetate was added to the reaction mixture, and the organic layer was washed with 2 N hydrochloric acid and then with a saturated sodium bicarbonate aqueous solution. The residue obtained by drying the organic layer over anhydrous sodium sulfate and then concentrating under reduced pressure was dissolved in 20 mL of methanol, and 10 mL of water and 2.76 g of sodium carbonate were added at room temperature. After stirring at the same temperature for 2 hours, water was added, and the mixture was extracted with ethyl acetate. The organic layer was dried over anhydrous sodium sulfate and then concentrated under reduced pressure to obtain 3. 5 g of tert-butyl {3-[5-(3,5-dichlorophenyl)-5-trifluoromethyl-4,5-dihydroisox azol-3-yl]phenyl}carbamate.

tert-butyl {3-[5-(3,5-dichlorophenyl)-5-trifluoromethyl-4,5-dihydroisoxazol-3-yl]phenyl}carbamate

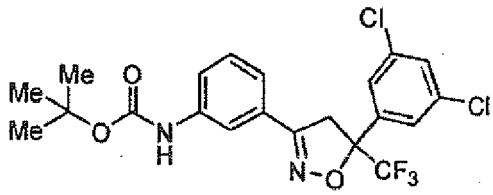

$^1$H-NMR($CDCl_3$) δ: 7.81(1H, s), 7.51(2H, s), 7.42-7.39 (2H, m), 7.35-7.31(2H, m), 6.55(1H, brs), 4.11(1H, d, J=16.9 Hz), 3.70(1H, d, J=16.9 Hz), 1.53(9H, s).

Reference Production Example 6

In 22 mL of tert-butyl methyl ether was dissolved 3.50 g of the tert-butyl {3-[5-(3,5-dichlorophenyl)-5-trifluoromethyl-4,5-dihydroisoxazol-3-yl]phenyl}carbamate produced by Reference Production Example 5, and 23 mL of a 28% sodium hydroxide aqueous solution, 7 mL of ammonia water, 2.56 g of ammonium chloride and 100 mg of trioctylmethyl ammonium chloride were added thereto. To this mixture solution, 53 mL of a 5% sodium hypochlorite aqueous solution was added dropwise at room temperature over 20 minutes. After stirring at the same temperature for 12 hours, the organic layer was separated. tert-Butyl methyl ether was added to the water layer, and the mixture was again extracted. The combined organic layers were dried over anhydrous sodium sulfate and then concentrated under reduced pressure. The resulting residue was subjected to silica gel column chromatography to obtain 1.52 g of tert-butyl N-{3-[5-(3,5-dichlorophenyl)-5-trifluoromethyl-4,5-dihydrois oxazol-3-yl]phenyl}carbazate.

tert-butyl N-{3-[5-(3,5-dichlorophenyl)-5-trifluoromethyl-4,5-dihydroisoxazol-3-yl]phenyl}carbazate

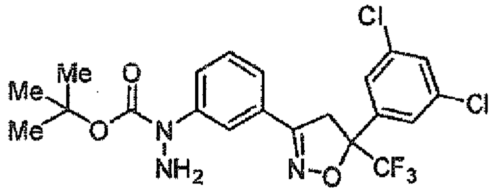

$^1$H-NMR($CDCl_3$) δ: 7.87(1H, s), 7.72-7.29(6H, m), 4.42(2H, br s), 4.09(1H, d, J=17.2 Hz), 3.69(1H, d, J=17.2 Hz), 1.53(9H, s).

Reference Production Example 7

In 20 mL of N,N-dimethylformamide were dissolved 3.71 g of the N-{3-[5-(3,5-dichlorophenyl)-5-trifluoromethyl-4,5-dihydroisoxazol-3-yl]phenyl}2,2,2-trifluoroacetamide produced by Reference Production Example 4 and 1.34 g of methyl iodide, and 1.31 g of sodium carbonate was added thereto at room temperature. Then, the mixture was stirred at the same temperature for 12 hours. Ethyl acetate was added to the reaction mixture, and the mixture was washed with 2N hydrochloric acid and then a saturated sodium bicarbonate aqueous solution. The organic layer was dried over anhydrous sodium sulfate and then concentrated under reduced pressure to obtain 3.70 g of N-methyl-N-{3-[5-(3,5-dichlorophenyl)-5-trifluoromethyl-4,5-dihydroisoxazol-3-yl]phenyl}2,2,2-trifluoroacetamide.

N-methyl-N-{3-[5-(3,5-dichlorophenyl)-5-trifluoromethy l-4,5-dihydroisoxazol-3-yl]phenyl}2,2,2-trifluoroacetamide

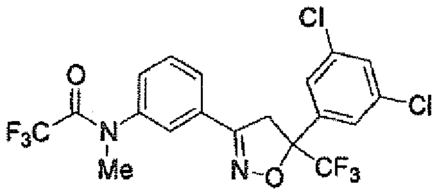

$^1$H-NMR($CDCl_3$) δ: 7.69-7.35(7H, m), 4.09(1H, d, J=17.3 Hz), 3.71(1H, d, J=17.3 Hz), 3.46-3.43(3H, m).

Reference Production Example 8

In 15 mL of methanol, 3.52 g of the N-methyl-N-{3-[5-(3,5-dichlorophenyl)-5-trifluoromethyl-4,5-dihydroisnxazol-3-yl]phenyl}2,2,2-trifluoroacetamide produced by Reference Production Example 7 was dissolved, and 2.00 g of sodium carbonate and 5 mL of water were added thereto at room temperature. After stirring at the same temperature for 2 hours, ethyl acetate was added, and the water layer was extracted with ethyl acetate. The organic layer was washed with 2% hydrochloric acid and then a saturated sodium bicarbonate aqueous solution. The organic layer was dried over anhydrous sodium sulfate and then concentrated under reduced pressure to obtain 2.80 g of N-methyl-3-[5-(3,5-dichloropheny)-5-trifluoromethyl-4,5-dih ydroisoxazol-3-yl]aniline.

N-methyl-3-[5-(3,5-dichlorophenyl)-5-trifiuoromethyl-4 ,5-dihydroisoxazol-3-yl]aniline

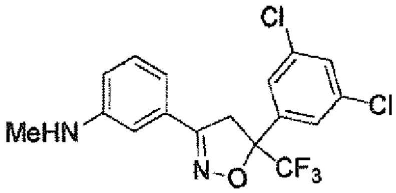

$^1$H-NMP ($CDCl_3$) δ: 7.51(2H, d, J=1.2 Hz), 7.41 (1H, t, J=1.8 Hz), 7.21(1H, t, J=7.9 Hz), 6.95(1H, t, J=2.0 Hz), 6.87(1H, ddd, J=7.6, 1.5, 1.0 Hz), 6.69(1H, ddd, J=8.3, 2.4, 0.7 Hz), 4.07(1H, d, J=17.3 Hz), 3.85(1H, br s), 3.67(1H, d, J=17.3Hz), 2.86(3H, s).

Reference Production Example 9

In 4 mL of tetrahydrofuran, 2.6 g of the N-methyl-3-[5-(3,5-dichlorophenyl)-5-trifluoromethyl-4,5-dih ydroisoxazol-3-yl]aniline produced by Reference Production Example 8 was dissolved, and 1.5 mL of concentrated hydrochloric acid and 3.0 mL of water were added thereto at room temperature After stirring at room temperature for 15 minutes, a solution of 581 mg of sodium nitrite dissolved in 5 mL of water was added dropwise to this mixture solution at 0°C. After stirring at the same temperature for 1 hour, a saturated sodium bicarbonate aqueous solution was added, and the water layer was extracted with ethyl acetate. The organic layer was dried over anhydrous sodium sulfate and then concentrated under reduced pressure to obtain 2.78 g of N-methyl-N-nitroso-3-[5-(3,5-dichlorophenyl)-5-trifiuorornethyl-4,5-dibydroisoxazol-3-yl]aniline.

N-methyl-N-nitroso-3-[5-(3,5-dichlorophenyl)-5-trifluo romethyl-4,5-dihydroisoxazol-3-yl]aniline

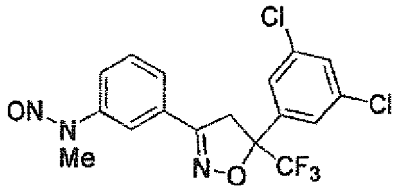

$^1$H-NMR($CDCl_3$) δ: 7.89-7.88(1H, m), 7.67-7.64(2H, m), 7.56-7.53(3H, m), 7.43-7.43(1H, m), 4.14(1H, d, J=17.4 Hz), 3.76(1H, d, J=17.4 Hz), 3.47(3H, s).

Reference Production Example 10

To a mixture of 1.00 g of the N-methyl-N-nitroso-3-[5-(3,5-dichlorophenyl)-5-trifluorometh yl-4,5-dihydroisoxazol-3-yl]aniline produced by Reference Production Example 9, 2 mL of ethanol, 2 mL of water and 2 mL of acetic acid was added 695 mg of zinc at room temperature. After stirring at room temperature for 5 hours, this mixture solution was filtered, and anhydrous sodium bicarbonate was added to the filtrate, then the mixture was extracted with chloroform. The organic layer was dried over anhydrous sodium sulfate and then concentrated under reduced pressure. The resulting residue was subjected to silica gel column chromatography to obtain 1.1 g of crude N-methyl-N-{3-[5-(3,5-dichlorophenyl)-5-trifluoromethyl-4,5-dihydroisoxazol-3-yl]phenyl}hydrazine.

N-methyl-N-{3-[5-(3,5-dichlorophenyl)-5-trifluoromethy l-4,5-dihydroisoxazol-3-yl]phenyl}hydrazine

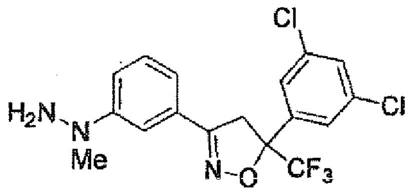

Reference Production Example 11

To a mixture of 3.00 g of 4-methyl-3-nitrobenzaldehyde, 1.64 g of hydroxylamine hydrochloride, 30 mL of ethanol and 15 mL of water was added 2. 24 g of sodium acetate at room temperature, and the mixture was stirred at room temperature for 1 hour. The reaction mixture was added to water, and the mixture was extracted with ethyl acetate. The organic layer was dried over anhydrous sodium sulfate and then concentrated under reduced pressure to obtain 2.77 g of 5-hydroxyiminomethyl-2-methylnitrobenzene.

5-hydroxyiminomethyl-2-methylnitrobenzene

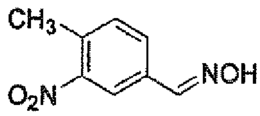

$^1$H-NMR(DMSO-$d_6$) δ :11.53(1H, s), 8.24(1H, s), 8.17 (1H, d, J=1.7 Hz), 7.84(1H, dd, J-8.0, 1.7 Hz), 7.54(1H, d, J=8.0 Hz), 2.52(3H, s).

Reference Production Example 12

In 30 mL of N,N-dimethylformamide were dissolved 2.77 g of the 5-hydroxyiminomethyl-2-methylnitrobenzene obtained by Reference Production Example 11 and 2.06 g of N-chlorosuccinimide, and the solution was stirred at 50°C for 1 hour. To the reaction solution that had been cooled to room temperature, 3.71 g of 2-(3,5-dichlorophenyl)-3,3,3-trifluoro-1-propene, followed by 1.56 g of triethylamine, was added and the mixture was stirred at room temperature for 6 hours. Water was added to the reaction mixture, and the mixture was extracted with ethyl acetate. The organic layer was dried over anhydrous sodium sulfate and then concentrated under reduced pressure. The resulting residue was subjected to silica gel column chromatography to obtain 4.99 g of 5-[5-(3,5-dichlorophenyl)-5-trifluoromethyl-4,5-dihydroisoxazol-3-yl]-2-methylnitrobenzene.

5-[5-(3,5-dichlorophenyl)-5-trifluoromethyl-4,5-dihydr oisoxazol-3-yl]-2-methylnitrobenzene

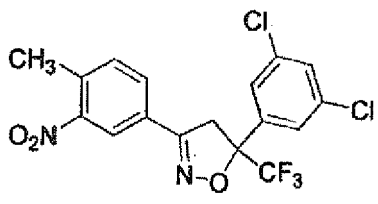

$^1$H-NMR ($ClDl_3$) δ: 8.16(1H, d, J=1.9 Hz), 7.90(1H, dd, J=8.1, 1.9 Hz), 7.51(2H, d, J=1.2 Hz), 7.45-7.43(2H, m), 4.11 (1H, d, J=17.1 Hz), 3.73(1H, d, J=17.1 Hz), 2.66(3H, s).

Reference Production Example 13

To 19 mL of a 2.5% acetic acid aqueous solution, 1.89 g of iron powder (10 to 20 mesh) was added, and 29 mL of an ethanol suspension of 4.62 g of the 5-[5-(3,5-dichlorophenyl)-5-trifluoromethyl-4,5-dihydroisoxa zol-3-yl]-2-methylnitrobenzene produced by Reference Production Example 12 was added thereto at 75°C. After stirring at the same temperature for 15 minutes, 1.80 g of iron powder was further added at the same temperature, and the mixture was stirred at the same temperature for 1 hour. The reaction mixture that had been cooled to room temperature was filtered and washed with ethyl acetate. A saturated sodium bicarbonate aqueous solution and ethyl acetate were added to the filtrate, and the mixture was extracted. The organic layer was dried over anhydrous sodium sulfate and then concentrated under reduced pressure. The resulting residue was subjected to silica gel column chromatography to obtain 3.18 g of 5-[5-(3,5-dichlorophenyl)-5-trifluoromethyl-4,5-dihydroisoxa zol-3-yl]-2-methylaniline.

5-[5-(3,5-dichlorophenyl)-5-tifluoromethyl-4,5-dihydr oisoxazol-3-yl]-2-methylaniline

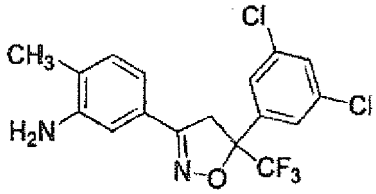

$^1$H-NMR ($CDCl_3$) $\delta$: 7.51(2H, d, J=1.4 Hz), 7.41(1H, t, J=1.8 Hz), 7.08(1H, d, J=7.8 Hz), 7.05(1H, d, J=1.8 Hz), 6.88(1H, dd, J=7.8, 1.7 Hz), 4.04(1H, d, J=17.1 Hz), 3.71(2H, brs), 3.65(1H, d, J=17.1 Hz), 2.19(3H, s).

Reference Production Example 14

To 420 mg of the 5-[5-(3,5-dichlorophenyl)-5-trifluorome-4,5-dihydroisoxazol-3-yl]-2-methylaniline produced by Reference Production Example 13 was added 2 mL of concentrated hydrochloric acid at room temperature. After stirring at the same temperature for 20 minutes, this solution was cooled to 0°C, and a solution of 82 mg of sodium nitrite dissolved in 0.5 mL of water was added dropwise. After stirring at the same temperature for 10 minutes, a solution of 451 mg of tin (II) chloride dissolved in 0.5 mL of concentrated hydrochloric acid was added dropwise to this reaction solution. Subsequently, a saturated sodium bicarbonate aqueous solution was added to the reaction mixture solution, and the mixture was neutralized. The water layer was extracted with ethyl acetate. The organic layer was dried over anhydrous sodium sulfate and then concentrated under reduced pressure to obtain 420 mg of crude 5-[5-(3,5-dichlorophenyl)-5-trifluoromethyl-4,5-dihydroisoxa zol-3-yl]-2-methylphenylhydrazine.

5-[5-(3,5-dichlorophenyl)-5-trifluoromethyl-4,5-dihydr oisoxazol-3-yl]-2-methylphenylhydrazine

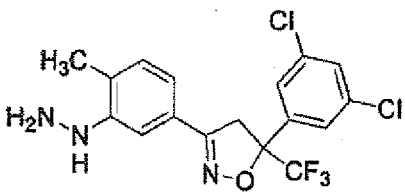

Reference Production Example 15

In a mixture solution of 20 mL of ethanol and 10 mL of water, 2 g of 4-fluoro-3-nitrobenzaldehyde, 1.07 g of hydroxylamine hydrochloride, and 1.45 g of sodium acetate were dissolved, and then the solution was stirred at room temperature for 6 hours. The reaction mixture was added to water and extracted with ethyl acetate. The organic layer was dried over anhydrous sodium sulfate and then concentrated under reduced pressure to obtain 1.95 g of 2-fluoro-5-hydroxyiminomethylnitrobenzene.

2-fluoro-5-hydroxyiminomethylnitrobenzene

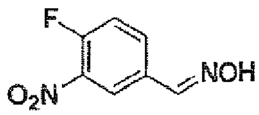

$^1$H-NMR (DMSO-$D_6$) δ: 11.63(1H, s), 8.34(1H, dd, J=7.2, 2.2 Hz), 8.28(1H, s), 8.04-8.02(1H, m), 7.65-7.62(1H, m).

Reference Production Example 16

In 20 mL of dimethylformamide were dissolved 1.95 g of the 2-fluoro-5-hydroxyiminomethylnitrobenzene obtained by Reference Production Example 15 and 1.42 g of N-chlorosuccinimide, and the solution was stirred at 60°C for 1 hour. To the reaction mixture solution that had been cooled to room temperature, 2.55 g of 2-(3,5-dichlorophenyl)-3,3,3-trifluoro-1-propene, followed by 1.07 g of triethylamine, was added, and the mixture was stirred at the same temperature for 6 hours. Water was added to the reaction mixture, and the mixture was extracted with ethyl acetate. The organic layer was dried over anhydrous sodium sulfate and then concentrated under reduced pressure. The resulting residue was subjected to silica gel column chromatography to obtain 1.40 g of 5-[5-(3,5-dichlorophenyl)-5-trifluoromethyl-4,5-dihydroisoxa zol-3-yl]-2-fluoronitrobenzene.

5-[5-(3,5-dichlorophenyl)-5-trifluoromethyl-4,5-dihydr oisoxazol-3-yl]-2-fluoronitrobenzene

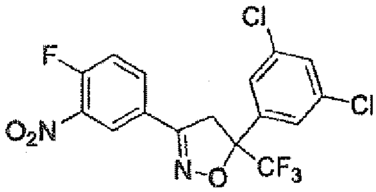

$^1$H-NMR($CDCl_3$) δ: 8.26(1H, dd, J=6.9, 2.3 Hz), 8.08-8.05(1H, m), 7.51(2H, d, J=1.2 Hz), 7.45(1H, t, J=1.9 Hz), 7.41(1H, dd, J=10.1, 8.7 Hz), 4.11(1H, d, J=17.4 Hz), 3.73(1H, d, J=17.9 Hz).

Reference Production Example 17

To a mixture solution of 0.2 g of acetic acid and 7 mL of water, 1.85 g of iron powder was added at room temperature, and 15 mL of an ethanol suspension of 1.40 g of the 5-[5-(3,5-dichlorophenyl)-5-trifluoromethyl-4,5-dihydroisoxa zol-3-yl]-2-fluoronitrobenzene obtained by Reference Production Example 16 was added thereto at 75°C, and then the mixture was stirred at 75°C for 20 minutes. The reaction mixture was filtered, and the filtrate was concentrated under reduced pressure. The resulting residue was subjected to silica gel column chromatography to obtain 806 mg of 5-[5-(3,5-dichlorophenyl)-5-trifluoromethyl-4,5-dihydroisoxa zol-3-yl]-2-fluoronitroaniline.

5-[5-(3,5-dichlorophenyl)-5-trifluoromethyl-4,5-dihydr oisoxazol-3-yl]-2-fluoronitroaniline

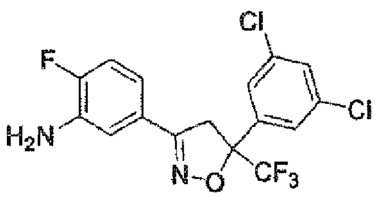

$^1$H-NMR ($CDCl_3$) δ: 7.50(2H, d, J=1.4 Hz), 7.42(1H, t, J=1.8 Hz), 7.18(1H, dd, J=8.5, 2.2 Hz), 7.01(1H, dd, J=10.6, 8.5 Hz), 6.90-6.87(1H, m), 4.03(1H, d, J=17.0 Hz), 3.85(2H, brs), 3.64(1H, d, J=17.0 Hz).

Reference Production Example 18

In 2 mL of 1,4-dioxane was dissolved 500 mg of the 5-[5-(3,5-dichlorophenyl)-5-trifluoromethyl-4,5-dihydroisoxa zol-3-yl]-2-fluoronitroaniline produced by Reference Production Example 17, and 4 mL of concentrated hydrochloric acid was added thereto at room temperature. After stirring at the same temperature for 20 minutes, this solution was cooled to 0°C, and a solution of 96 mg of sodium nitrite dissolved in 5 mL of water was added dropwise. After stirring at the same temperature for 15 minutes, a solution of 528 mg of tin (II) chloride dissolved in 1 mL of concentrated hydrochloric acid was added dropwise to this reaction solution. Subsequently, 2N sodium hydroxide was added to the reaction mixture solution, and the mixture was neutralized, and then the water layer was extracted with ethyl acetate. The organic layer was dried over anhydrous sodium sulfate and then concentrated under reduced pressure to obtain 412 mg of crude 5-[5-(3,5-dichlorophenyl)-5-trifluoromethyl-4,5-dihydroisoxazol-3-yl]-2-fluorophenylhydrazine.

5-[5-(3,5-dichlorophenyl)-5-trifluoromethyl-4,5-dihydr oisoxazol-3-yl]-2-fluorophenylhydrazine

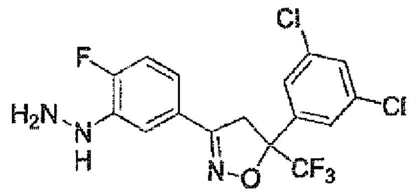

Reference Production Example 19

In 30 mL of dimethylformamide, 2.92 g of 2-chloro-5-hydroxyiminomethylnitrobenzene and 1.94 g of N-chlorosuccinimide were dissolved, and the solution was stirred at 60°C for 1 hour. To the mixture solution that had been cooled to room temperature, 3.50 g of 2-(3,5-dichlorophenyl)-3,3,3-trifluoro-1-propene, followed by 1.46 g of triethylamine, was added, and the mixture was stirred for 6 hours. Water was added to the reaction mixture, and the mixture was extracted with ethyl acetate. The organic layer was dried over anhydrous sodium sulfate and then concentrated under reduced pressure. The resulting residue was subjected to silica gel column chromatography to obtain 4.42 g of 5-[5-(3,5-dichlorophenyl)-5-trifluoromethyl-4,5-dihydroisoxa zol-3-yl]-2-chloronitrobenzene.

5-[5-(3,5-dichlorophenyl)-5-trifluoromethyl-4,5-dihydr oisoxazol-3-yl]-2-chloronitrobenzene

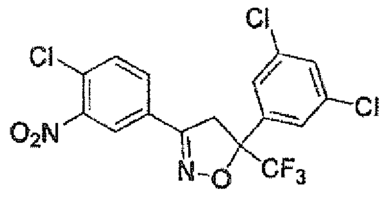

$^1$H-NMR($CDCl_3$) δ: 8.09(1H, d, J=2.1 Hz), 7.89(1H, dd, J=8.5, 2.1 Hz), 7.65(1H, d, J=8.5 Hz), 7.50(2H, d, J=1.6 Hz), 7.45(1H, t, J=1.6 Hz), 4.09(1H, d, J=17.3 Hz), 3.71(1H, d, J=17.3 Hz).

Reference Production Example 20

To a mixture solution of 0.38 g of acetic acid, 15 mL of water and 30 mL of ethanol, 3.46 g of iron powder was added at room temperature, and 2.73 g of the 5-[5-(3,5-dichlorophenyl)-trifluoromethyl-4,5-dihydroisoxaz ol-3-yl]-2-chlnronitrobenzene obtained by Reference Production Example 19 was added thereto at 75°C, and then the mixture was stirred at 75°C for 50 minutes. The reaction mixture was filtered, and the filtrate was concentrated under reduced pressure. The resulting residue was subjected to silica gel column chromatography to obtain 1.65 g of 5- [5- (3,5-dichlorophenyl)-5-trifluoromethyl-4,5-dihydroisoxazol-3-yl]-2-chloroaniline.

5-[5-(3,5-dichlorophenyl)-5-trifluoromethyl-4,5-dihydr oisoxazol-3-yl]-2-chloroaniline

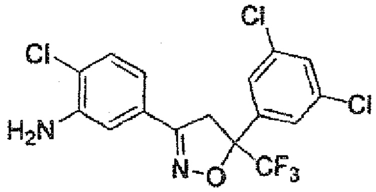

$^1$H-NMR ($CDCl_3$) δ: 7.49 (2H, d, J=1.7 Hz), 7.42 (1H, t, J=1.7 Hz), 7.29(1H, d, J=8.4 Hz), 7.13(1H, d, J=2.0 Hz), 6.89 (1H, dd, J=8.4, 2.0 Hz), 4.18(2H, br s), 4.03(1H, d, J=17.1 Hz), 3.64(1H, d, J=16.4 Hz).

Reference Production Example 21

In 2 mL of 1,4-dioxane was dissolved 500 mg of the 5-[5-(3,5-dichlorophenyl)-5-trifluoromethyl-4,5-dihydroisoxa zol-3-yl]-2-chloroaniline produced by Reference Production Example 20, and 6 mL of concentrated hydrochloric acid was added thereto at room temperature. After stirring at the same temperature for 20 minutes, this solution was cooled to 0°C, and a solution of 93 mg of sodium nitrite dissolved in 3 mL of water was added dropwise. After stirring at the same temperature for 15 minutes, a solution of 507 mg of tin (II) chloride dissolved in 4 mL of concentrated hydrochloric acid was added dropwise to this reaction solution. Subsequently, 2N sodium hydroxide was added to the reaction mixture solution, and the mixture was neutralized, and then the water layer was extracted with t-butyl methyl ether. The organic layer was dried over anhydrous sodium sulfate and then concentrated under reduced pressure to obtain 510 mg of crude 5-[5-(3,5-dichlorophenyl)-5-trifluoromethyl-4,5-dihydroisoxazol-3-yl]-2-chlorophenylhydrazine.

5-[5-(3,5-dichlorophenyl)-5-trifluoromethyl-4,5-dihydr oisoxazol-3-yl]-2-chlorophenylhydrazine

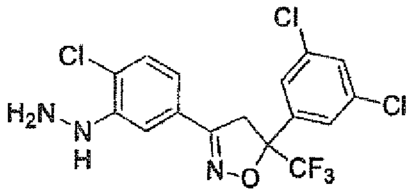

Reference Production Example 22

In 50 mL of toluene, 6.3 g of triphosgene was dissolved, and a solution of 8.7 g of the 5-[5-(3,5-dichlorophenyl)-5trifluoromethyl-4,5-dihydroisoxaz ol-3-yl]-2-chloroaniline obtained by Reference Production Example 20 dissolved in 50 mL of toluene and 10 mL of tetrahydrofuran was added dropwise thereto at room temperature. Another 50 mL of toluene was added to the reaction solution, and the mixture was stirred at 80°C for 1 hour. The reaction mixture that had been cooled to room temperature was concentrated under reduced pressure, and 100 mL of tert-butanol was added. To this solution, 14.6 g of triethylamine was added dropwise at room temperature, and the mixture was stirred at the same temperature for 16 hours. A saturated sodium bicarbonate aqueous solution was added to the reaction mixture, and the mixture was extracted with chloroform. The organic layer was dried over anhydrous sodium sulfate and then concentrated under reduced pressure. The resulting residue was subjected to silica gel column chromatography to obtain 9.81 g of tert-butyl {2-chloro-5-[5-(3,5-dichlorophenyl)-5-trifluoromethyl-4,5-di hydroisoxazol-3-yl]phenyl}carbamate.

tert-butyl {2-chloro-5-[5-(3,5-dichlorophenyl)-5-trifluoromethyl-4,5-dihydroisoxazol-3-yl]phenyl}carbamate

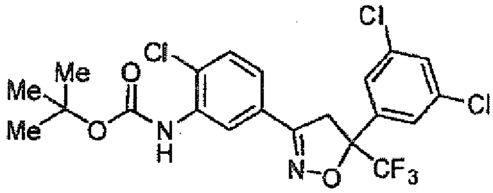

$^1$H-NMR ($CDCl_3$) δ: 8.39(1H, d, J=2.1 Hz), 7.52(2H, d, J=1.8 Hz), 7.47(1H, dd, J=8.5, 2.1 Hz), 7.42(1H, t, J=1.8 Hz), 7.39(1H, d, J=8.5 Hz), 7.09(1H, br s), 4.11(1H, d, J=17.3 Hz), 3.70(1H, d, J=17.3 Hz), 1.55(9H, s).

Reference Production Example 23

In 200 mL of tetrahydrofuran, 760 mg of sodium hydride (60% in oil) was suspended, and 50 mL of a tetrahydrofuran solution of 8.80 g of the tert-butyl {2-chloro-5-[5-(3,5-dichlorophenyl)-5tritluoromethyl-4,5-dih ydroisoxazol-3-yl]phenyl}carbamate produced by Reference Production Example 22 was added dropwise thereto at room temperature. After stirring at the same temperature for 20 minutes, 6.0 g of O- (diphenylphosphoryl) hydroxylamine was added at room temperature. After stirring at the same temperature for 15 hours, water and ethyl acetate were added, and the water layer was extracted with ethyl acetate. The organic layer was dried over anhydrous sodium sulfate and then concentrated under reduced pressure. The resulting residue was subjected to silica gel column chromatography to obtain 6.58 g of tert-butyl N-{2-chloro-5-[5-(3,5-dichlorophenyl)-5-trifluoromethyl-4,5-dihydroisoxazol-3-yl]phenyl}carbazate.

tert-butyl N-{2-chloro-5-[5-(3,5-dichlorophenyl)-5-trifluoromethyl-4,5-dihydroisoxazol-3-yl]phenyl}carbazate

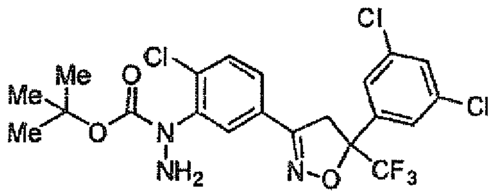

$^1$H-NMR ($CDCl_3$) δ: 7.55-7.44(6H, m), 4.07(1H, d, J=17.1 Hz), 3.68(1H, d, J=17.1 Hz), 1.41(9H, br s).

Reference Production Example 24

In 20 mL of tetrahydrofuran were dissolved 3.04 g of the 5-[5-(3,5-dichlorophenyl)-5trifluoromethyl-4,5-dihydroisoxaz ol-3-yl]-2-chlaroaniline obtained by Reference Production Example 20 and 772 mg of triethylamine, and 1.47 g of trifluoroacetic anhydride was added dropwise thereto at 0°C. After stirring at the same temperature for 30 minutes, the reaction mixture was diluted with tert-butyl methyl ether. Ethyl acetate was added to the reaction mixture, and the organic layer was washed with 2N hydrochloric acid and then a saturated sodium bicarbonate aqueous solution. The organic layer was dried over anhydrous sodium sulfate and then concentrated under reduced pressure to obtain 3.63 g of N-{2-chloro-5-[5-(3,5-dichlorophenyl)-5trifluoromethyl-4,5-d ihydroisoxazol-3-yl]phenyl}2,2,2-trifluoroacetamide.

N-{2-chloro-5-[5-(3,5-dichlorophenyl)-5trifluoromethyl -4,5-dihydroisoxazol-3-yl]phenyl}2,2,2-trifluoroacetamide

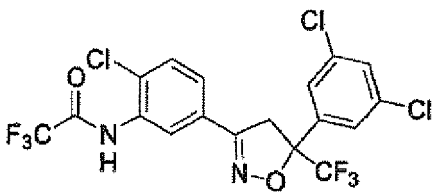

$^1$H-NMR($CDCl_3$) δ: 8.53(1H, d, J=2.2 Hz), 8.48(1H, br s), 7.67(1H, dd, J=8.5, 2.0 Hz), 7.52(1H, d, J=8.5 Hz), 7.50 (2H, s), 7.43-7.42(1H, m), 4.09(1H, d, J=17.3 Hz), 3.71(1H, d, J=17.3 Hz).

Reference Production Example 25

Sodium hydride (60% in oil) was suspended in 15 mL of N,N-dimethylformamide, and a solution of 3.6 g of the N-{2-chloro-5-[5-(3,5-dichlorophenyl)-5trifluoromethyl-4,5-d ihydroisoxazol-3-yl]-phenyl}-2,2,2-trifluoroacetamide obtained by Reference Production Example 24 dissolved in 15 mL of N,N-dimethylformamide was added dropwise thereto at room temperature. After stirring at the same temperature for 20 minutes, 1.52 g of methyl iodide was added, and the mixture was stirred for another hour. 2N hydrochloric acid was added to the reaction mixture solution, and the mixture was extracted with tert-butyl methyl ether. The organic layer was dried over anhydrous sodium sulfate and then concentrated under reduced pressure to obtain 3.73 g of N-methyl-N-{2-chloro-5-[5-(3,5-dichlorophenyl)-5-trifluoromethyl-4,5-dihydroisoxazol-3-yl]p henyl}-2,2,2-trifluoroacetamide.

N-methyl-N-{2-chloro-5-[5-(3,5-dichlorophenyl)-5-trifl uoromethyl-4,5-dihydroisoxazol-3-yl]phenyl}-2,2,2-trifluoroa cetamide

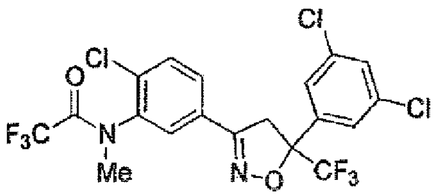

$^1$H-NMR ($CDCl_3$) δ: 7.70-7.40(6H, m), 4.06 (1H, dd, J=17.2, 13.0 Hz), 3.73-3.64(1H, m), 3.33-3.32(3H, m).

Reference Production Example 26

In 20 mL of methanol, 3.6 g of the N-methyl-N-{2-chloro-5-[5-(3,5-dichlorophenyl)-5-trifluorome thyl-4,5-dihydroisoxazol-3-yl]phenyl}2,2,2-trifluoroacetamid e obtained by Reference Production Example 25 was dissolved, and 1.97 g of potassium carbonate was added thereto at room temperature, and then the mixture was stirred at the same temperature for 3 hours. The precipitate was filtered out, water was added to the filtrate, and the mixture was extracted with t-butyl methyl ether. The organic layer was dried over anhydrous sodium sulfate and then concentrated under reduced pressure to obtain 2.20 g of N-methyl-2-chloro-5-[5-(3,5-dichlorophenyl)-5-trifluoromethyl-4,5-dihydroisoxazol-3-yl]aniline.

N-methyl-2-chloro-5-[5-(3,5-dichlorophenyl)-5-trifluor omethyl-4,5-dihydroisoxazol-3-yl]aniline

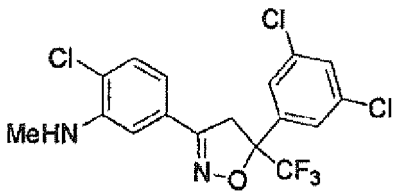

$^1$H-NMR ($CDCl_3$) δ: 7.51(2H, d, J=1.8 Hz), 7.42(1H, t, J=1.8 Hz), 7.28(1H, d, J=8.0 Hz), 6.99 (1H, d, J=2.0 Hz), 6.78 (1H, dd, J=8.0, 2.0 Hz), 4.48(1H, d, J=5.1 Hz), 4.07 (1H, d, J=17.2 Hz), 3.67(1H, d, J=17.2 Hz), 2.94(3H, d, J=5.1 Hz).

Reference Production Example 27

In 4 mL of tetrahydrofuran, 2.55 g of the N-methyl-2-chloro-5-[5-(3,5-dichlorophenyl)-5-trifluoromethy l-4,5-dihydroisoxazol-3-yl]aniline produced by Reference Production Example 26 was dissolved, and 4.5 mL of concentrated

hydrochloric acid was added, followed by 5.0 mL of water. After stirring at room temperature for 10 minutes, this mixture solution was cooled to 0°C, and a solution of 539 mg of sodium nitrite dissolved in 5 mL of water was added dropwise. After stirring at the same temperature for 1 hour, a saturated sodium bicarbonate aqueous solution was added to neutralize the mixture, and then the mixture was extracted with ethyl acetate. The organic layer was dried over anhydrous sodium sulfate and then concentrated under reduced pressure to obtain 2.69 g of N-methyl-N-nitroso-2-chloro-5-[5-(3,5-dichlorophenyl)-5-trif luoromethyl-4,5-dihydroisoxazol-3-yl]aniline.

N-methyl-N-nitroso-2-chloro-5-[5-(3,5-dichlorophenyl)-5-trifluoromethyl-4,5-dihydroisoxazol-3-yl]aniline

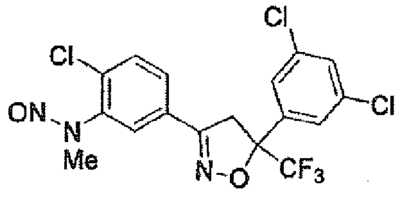

$^1$H-NMR ($CDCl_3$) $\delta$: 7.84-7.31(6H, m), 4.09(1H, d, J=17.3 Hz), 3.71(1H, d, J=17.3 Hz), 3.41(3H, s).

Reference Production Example 28

In 4 mL of tetrahydrofuran, 2.61 g of the N-methyl-N-nitroso-2-chloro-5-[5-(3,5-dichlorophenyl)-5-trif luoromethy2-4,5-dihydroisoxazol-3-yl]aniline produced by Reference Production Example 27 was dissolved, and 8 mL of ethanol, 8 mL of water and 8 mL of acetic acid were added thereto at room temperature, and then 695 mg of zinc was added to this mixture at room temperature. After stirring at room temperature for 3 hours, this mixture solution was filtered, and anhydrous sodium bicarbonate was added to the filtrate, and then the mixture was extracted with ethyl acetate. The organic layer was dried over anhydrous sodium sulfate and then concentrated under reduced pressure. The resulting residue was subjected to silica gel column chromatography to obtain 764 mg of N-methyl-N-{2-chloro-5-[5-(3,5-dichlorophenyl)-5-trifluorome thyl-4,5-dihydroisoxazol-3-yl]phenyl}hydrazine.

N-methyl-N-{2-chloro-5-[5-(3,5-dichlorophenyl)-5-trifl uoromethyl-4,5-dihydroisoxazol-3-yl]phenyl}hydrazine

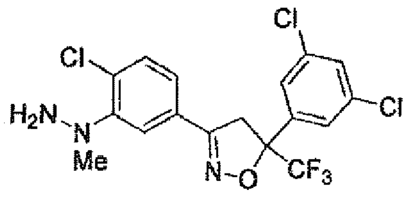

$^1$H-NMR ($CDCl_3$) $\delta$: 7.68-7.67(1H, m), 7.51(2H, s), 7.42-7.40(2H, m), 7.23-7.20(1H, m), 4.08(1H, d, J=17.2 Hz), 3.85(2H, br s), 3.69(1H, d, J=17.2 Hz), 3.06(3H, s).

Reference Production Example 29

In 40 mL of dimethylformamide were dissolved 4.30 g of 2-ethyl-5-hydroxyiminomethylnitrobenzene and 2.97 g of N-chlorosuccinimide, and the solution was stirred at 60°C for 1 hour. To the reaction solution that had been cooled to room temperature, 3.5 g of 2-(3,5-dichlorophenyl)-3,3,3-trifluoro-1-propene, followed by 1.7 g of triethylamine, was added, and the mixture was stirred for 6 hours. Water was added to the reaction mixture, and the mixture was extracted with ethyl acetate. The organic layer was dried over anhydrous sodium sulfate and then concentrated under reduced pressure. The resulting residue was subjected to silica gel column chromatography to obtain 5.74 g of 5-[5-(3,5-dichlorophenyl)-5-trifluoromethyl-4,5-dihydroisoxa zol-3-yl]-2-ethylnitrobenzene.

5-[5-(3,5-dichlorophenyl)-5-trifluoromethyl-4,5-dihydr oisoxazol-3-yl]-2-ethylnitrobenzene

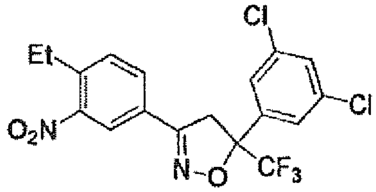

$^1H$-NMR ($CDCl_3$) δ: 8.07(1H, d, J=1.9 Hz), 7.91(1H, dd, J=8.0, 1.9 Hz), 7.51(2H, d, J=1.6 Hz), 7.46(1H, d, J=8.0 Hz), 7.44(1H, t, J=1.6 Hz), 4.10(1H, d, J=17.3 Hz), 3.72(1H, d, J=17.3 Hz), 2.96(2H, q, J=7.5 Hz), 1.30(3H, t, J=7.5 Hz).

Reference Production Example 30

To a mixture solution of 0.5 mL of acetic acid, 22 mL of water and 34 mL of ethanol, 4.33 g of iron power was added at room temperature, and 5.60 g of the 5-[5-(3,5-dichlorophenyl)-5-trifluoromethyl-4,5-dihydroisoxa zol-3-yl]-2-ethylnitrobenzene obtained by Reference production Example 29 was added thereto at 75°C, and then the mixture was stirred at the same temperature for 3 hours. The reaction mixture that had been cooled to room temperature was filtered and washed with ethyl acetate. A saturated sodium bicarbonate aqueous solution was added to the filtrate, and the mixture was extracted with ethyl acetate. The organic layer was dried over anhydrous sodium sulfate and then concentrated under reduced pressure to obtain 5.20 g of 5-[5-(3,5-dichlorophenyl)-5-trifluoromethyl-4,5-dihydroisoxazol-3-yl]-2-ethylaniline.

5-[5-(3,5-dichlorophenyl)-5-trifluoromethyl-4,5-dihydr oisoxazol-3-yl]-2-ethylaniline

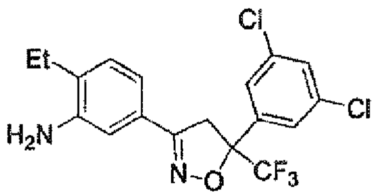

$^1H$-NMP ($CDCl_3$) δ: 7.51(2H, d, J=1.7 Hz), 7.41(1H, t, J=1.7 Hz), 7.10(1H, d, J=7.8 Hz), 7.04(1H, d, J=1.7 Hz), 6.93(1H, dd, J=7.8.1.7 Hz), 4.05(1H, d, J=17.1 Hz), 3.74(2H, br s), 3.65(1H, d, J=17.1 Hz), 2.52(2H, q, J=7.5 Hz), 1.25(3H, t, J=7.5 Hz).

Reference Production Example 31

In 2 mL of 1,4-dioxane, 1.00 g of the 5-[5-(3,5-dichlorophenyl)-5-trifluoromethyl-4,5-dihydroisoxa zol-3-yl]-2-ethylaniline produced by Reference Production Example 30 was dissolved, and 4 mL of concentrated hydrochloric acid was added thereto at room temperature. After stirring at the same temperature for 20 minutes, this solution was cooled to 0°C, and a solution of 188 mg of sodium nitrite dissolved in 1 mL of water was added dropwise. After stirring at the same temperature for 15 minutes, a solution of 1.03 g of tin (II) chloride dissolved in 2 mL of concentrated hydrochloric acid was added dropwise to this reaction solution. 2N sodium hydroxide was added to the reaction mixture, and the mixture solution was neutralized, then the water layer was extracted with chloroform. The organic layer was dried over anhydrous sodium sulfate and then concentrated under reduced pressure to obtain 1. 31 g of crude 5-[5-(3,5-dichlorophenyl)-5-trifluoromethyl-4,5-dihydroisoxa zol-3-yl]-2-ethylphenylhydrazine.

5-[5-(3,5-dichlorophenyl)-5-trifluoromethyl-4,5-dihydr oisoxazol-3-yl]-2-ethylphenylhydrazine

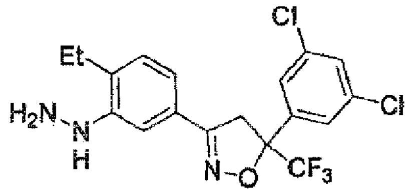

Reference Production Example 32

In 10 mL of acetic anhydride, 2.0 g of the compound obtained by Reference Production Example 20 was dissolved, and the mixture was stirred at room temperature for 0.5 hour. The reaction mixture was concentrated under reduced pressure, and the concentrate was dissolved in ethyl acetate and washed with a saturated sodium bicarbonate aqueous solution and then with saturated saline. The organic layer was dried over anhydrous sodium sulfate and then concentrated under reduced pressure to obtain 3.0 g of a crude compound shown by the following formula.

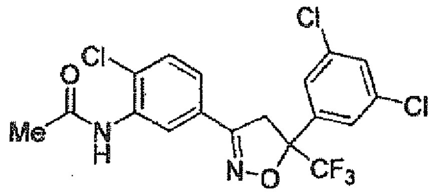

$^1$H-NMR ($CDCl_3$) $\delta$: 8.59(1H, br s), 7.67(1H, br s), 7.57(1H, dd, J=8.4, 2.1 Hz), 7.50(2H, d, J=1.2 Hz), 7.43-7.42(2H, m), 4.09(1H, d, J=17.4 Hz), 3.70(1H, d, J=17.4 Hz), 2.28(3H, s).

Reference Production Example 33

In 30 mL of tert-butyl methyl ether, 2.2 g of the compound obtained by Reference Production Example 32 was dissolved, and 14 mL of a 28% sodium hydroxide aqueous solution, 4.5 mL of ammonia water, 1.56 g of ammonium chloride, and 274 mg of trioctylmethyl ammonium chloride were added. To this mixture solution, 32 mL of a 5% sodium hypochlorite aqueous solution was added dropwise at room temperature over 20 minutes. After stirring at the same temperature for 2 hours, the organic layer was separated. tert-Butyl methyl ether was added to the water layer, and the mixture was extracted. The combined organic layers were dried over anhydrous sodium sulfate and then concentrated under reduced pressure. The resulting residue was subjected to silica gel column chromatography to obtain 2.0 g of a compound shown by the following formula.

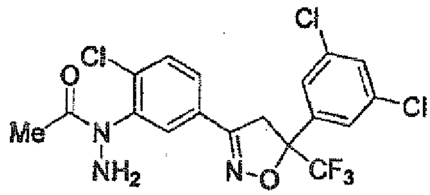

Melting point: 92°C

Reference Production Example 34

In 5 ml of pyridine, 2.0 g of the compound obtained by Reference Production Example 20 was dissolved, and 1.4 g of benzoyl chloride was added at room temperature. After stirring at the same temperature for 0.5 hour, ethyl acetate was added to the reaction mixture, and the mixture was washed with 3% hydrochloric acid. The organic layer was dried over anhydrous sodium sulfate and then concentrated under reduced pressure to obtain 2.4 g of a crude compound shown by the following formula.

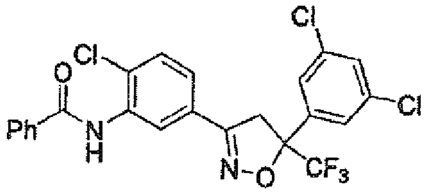

$^1$H-NMR($CDCl_3$) δ: 7.69-7.44(1H, m), 7.68(1H, dd, J=4.1, 2.0 Hz), 7.63(1H, dd, J=5.7, 2.1 Hz), 7.58(1H, d, J=7.8 Hz), 7.49(2H, d, J=7.6 Hz), 7.44(1H, t, J=2.0 Hz), 4.74(2H, s), 4.37(0H, s), 4.07(1H, dd, J=17.2, 6.7 Hz), 2.42(1H, s), 1.92 (2H, d, J=2.9 Hz).

Reference Production Example 35

In 20 mL of tetrahydrofuran, 2.4 g of the compound obtained by Reference Production Example 34 was dissolved, and 14 mL of a 28% sodium hydroxide aqueous solution, 4.5 mL of ammonia water, 2.56 g of ammonium chloride, and 274 mg of trioctylmethyl ammonium chloride were added. To this mixture solution, 48 mL of a 5% sodium hypochlorite aqueous solution was added dropwise at room temperature over 1 hour. After stirring at the same temperature for 2 hours, the organic layer was separated. tert-Butyl methyl ether was added to the water layer, and the mixture was extracted. The combined organic layers were dried over anhydrous sodium sulfate and then concentrated under reduced pressure. The resulting residue was subjected to silica gel column chromatography to obtain 1.8 g of a compound shown by the following formula.

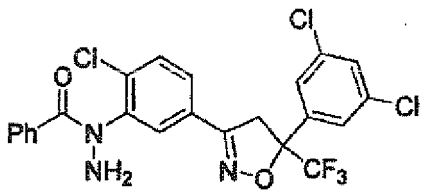

Melting point: 87°C

Next, formulation examples of the pest control composition of the present invention will be described. Herein, a part means a part by weight.

Formulation Example 1

Two parts of one hydrazide compound selected from the group consisting of the present hydrazide compounds (1) to (63) and 7 parts of thiamethoxam are dissolved in 37.5 parts of xylene and 37.5 parts of N,N-dimethylformamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 2

Five parts of one hydrazide compound selected from the group consisting of the present hydrazide compounds (1) to (63) and 4 parts of thiamethoxam are dissolved in 37.5 parts of xylene and 37.5 parts of N,N-dimethylformamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 3

Five parts of Sorpol 5060 (registered trade name of Toho Chemical Co., Ltd.) is added to a mixture of 20 parts of one hydrazide compound selected from the group consisting of the present hydrazide compounds (1) to (63) and 20 parts of thiamethoxam and mixed well, and 32 parts of Carplex #80 (registered trade name of Shionogi & Co. , Ltd. , synthetic hydrous silicon oxide fine powder) and 23 parts of 300 mesh diatomaceous earth are added thereto and mixed with a juice mixer to obtain wettable powders.

Formulation Example 4

One part of one Hydrazide compound selected from the group consisting of the present hydrazide compounds (1) to (63), 2 parts of thiamethoxam, 5 parts of synthetic hydrous silicon oxide fine powder, 5 parts of sodium dodecyl benzenesulfonate, 30 parts of bentonite, and 57 parts of clay are added and well stirred and mixed. Then, an appropriate amount of water is added to the mixture, and the mixture is further stirred, made into granules by a granulator, and subjected to ventilation drying to obtain granules.

Formulation Example 5

One and a half parts of one hydrazide compound selected from the group consisting of the present hydrazide compounds (1) to (63), 3 parts of thiamethoxam, 1 part of synthetic hydrous silicon oxide fine powder, 1 part of Driless B (manufactured by Sankyo Co., Ltd.) as flocculant, and 7 parts of clay are mixed well with a mortar, then stirred and mixed with a juice mixer. A quantity of 86.5 parts of cut clay is added to the resulting mixture, and the mixture is sufficiently stirred and mixed, to obtain dusts.

Formulation Example 6

Five parts of one hydrazide compound selected from the group consisting of the present hydrazide compounds (1) to (63) ; 5 parts of thiamethoxam; 35 parts of white carbon containing 50 parts of polyoxyethylene alkyl ether sulfate ammonium salt; and 55 parts of water are mixed, and the mixture is finely ground by a wet grinding method to obtain a formulation.

Formulation Example 7

A mixture of 0.2 part of one hydrazide compound selected from the group consisting of the present hydrazide compounds (1) to (63) and 0.3 part of thiamethoxam is dissolved in 10 parts of dichloromethane, and the solution is mixed with 89.5 parts of Isopar M (isoparaffin: registered trade name of Exxon Chemical Company) to obtain an oil solution.

Formulation Example 8

One twentieth (0.05) part of one hydrazide compound selected from the group consisting of the present hydrazide compounds (1) to (63), 0.05 part of thiamethoxam, and 49. 9 parts of Neochiozol (Chuo Kasei Co., Ltd.) are placed in an aerosol container. After attaching an aerosol valve, the container is charged with 25 parts of dimethyl ether and 25 parts of LPG, and an actuator is attached to the container, to obtain an oil-based aerosol.

Formulation Example 9

An aerosol container is charged with a dissolved mixture of 0.2 part of one hydrazide compound selected from the group consisting of the present hydrazide compounds (1) to (63), 0.4 part of thiamethoxam, 0.01 part of BHT, 5 parts of xylene, 3.39 parts of deodorized kerosene, and 1 part of an emulsifier {Atmos 300 (registered trade name of Atmos Chemical Co., Ltd.)} and 50 parts of distilled water. A valve part is attached, and 40 parts of a propellant (LPG) is pressurized and charged thereinto via the valve to obtain a water-based aerosol.

Formulation Example 10

Two parts of one hydrazide compound selected from the group consisting of the present hydrazide compounds (1) to (63) and 3 parts of thiamethoxam are dissolved in 80 parts of diethylene glycol monoethyl ether, and 15 parts of propylene carbonate is mixed with the solution, to obtain a spot-on liquid.

Formulation Example 11

Five parts of one hydrazide compound selected from the group consisting of the present hydrazide compounds (1) to (63) and 5 parts of thiamethoxam are dissolved in 70 parts of diethylene glycol monoethyl ether, and 20 parts of 2-octyldodecanol is mixed with the solution, to obtain a pour-on liquid.

Formulation Example 12

Sixty parts of Nikkol TEALS-42 (Nikko Chemicals Co. , Ltd., 42% aqueous solution of triethanolamine lauryl sulfate) and 20 parts of propylene glycol are added to a mixture of 0.25 part of one hydrazide compound selected from the group consisting of the present hydrazide compounds (1) to (63) and 0.25 part of thiamethoxam. After sufficiently stirring and mixing to form a homogeneous solution, 19.5 parts of water is added and further sufficiently stirred and mixed to obtain a shampoo formulation of the homogeneous solution.

Formulation Example 13

Eighty (80) mg of one hydrazide compound selected from the group consisting of the present hydrazide compounds (1) to (63), 20 mg of thiamethoxam, 68.75 mg of lactose, 237.5 mg of corn starch, 43.75 mg of microcrystalline cellulose, 18.75 mg of polyvinylpyrrolidone, 28.75 mg of sodium carboxymethyl starch, and 2.5 mg of magnesium stearate are mixed completely, and the mixture is compressed, to obtain a tablet of a suitable size.

Formulation Example 14

One hundred (100) mg of one hydrazide compound selected from the group consisting of the present hydrazide compounds (1) to (63), 100 mg of thiamethoxam, 148 mg of lactose, and 2 mg of magnesium stearate are mixed completely and packed into hard shell gelatin capsules or hydroxypropyl methylcellulose capsules, to obtain a capsule formulation.

Formulation Example 15

Two and a half parts of one hydrazide compound selected from the group consisting of the present hydrazide compounds (1) to (63) and 2.5 parts of thiamethoxam are dissolved in 5 parts of Polysorbate 85, 3 parts of benzyl alcohol, and 30 parts of propylene glycol, and the solution is adjusted to pH 6.0 to 6.5 using phosphate buffer and then brought to final volume by water, to obtain a liquid for oral administration.

Formulation Example 16

Three and six-tenth (3.6) parts of one hydrazide compound selected from the group consisting of the present hydrazide compounds (1) to (63), 3.6 parts of thiamethoxam, and 92.8 parts of Vosco S-55 (manufactured by Maruishi Pharmaceutical Co. , Ltd.) are dissolved and mixed at 100°C, poured into a suppository mold, and cooled and solidified to obtain a suppository.

Formulation Example 17

A mixture of 0.05 g of one hydrazide compound selected from the group consisting of the present hydrazide compounds (1) to (63) and 0.05 g of thiamethoxam is dissolved in 2 mL of propylene glycol, and the solution is impregnated into a 4.0 $\times$ 4.0 cm, 1.2 cm thick porous ceramic plate, to *obtain a heat-type smoking agent.

Formulation Example 18

Two and a half parts of one hydrazide compound selected from the group consisting of the present hydrazide compounds (1) to (63), 2.5 parts of thiamethoxam, and 95 parts of ethylene-methyl methacrylate copolymer (ratio of methyl methacrylate in the copolymer: 10% by weight, Acryft WD301, manufactured by Sumitomo Chemical Co., Ltd) are melt-kneaded by a closed-type pressure kneader (manufactured by Moriyama Company Ltd.). The resulting kneaded mixture is extruded from an extrusion molding machine through a molding die to obtain rod-shaped molded articles with 15 cm in length and 3 mm in diameter.

Formulation Example 19

Two and a half parts of one hydrazide compound selected from the group consisting of the present hydrazide compounds (1) to (63), 2.5 parts of thiamethoxam, and 95 parts of soft vinyl chloride resin are melt-kneaded by a closed-type pressure kneader (manufactured by Moriyama Company Ltd.). The resulting kneaded mixture is extruded from an extrusion molding machine through a molding die to obtain rod-shaped molded articles with 15 cm in length and 3 mm in diameter.

Formulation Example 20

Two parts of one hydrazide compound selected from the group consisting of the present hydrazide compounds (1) to (63) and 7 parts of clothianidin are dissolved in 37.5 parts of xylene and 37.5 parts of N,N-dimethylformamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 21

Five parts of one hydrazide compound selected from the group consisting of the present hydrazide compounds (1) to (63) and 4 parts of clothiartidin are dissolved in 37.5 parts of xylene and 37.5 parts of N,N-dimethylformamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 22

Five parts of Sorpol 5060 (registered trade name of Toho Chemical Co., Ltd.) is added to a mixture of 20 parts of one hydrazide compound selected from the group consisting of the present hydrazide compounds (1) to (63) and 20 parts of clothianidin and mixed well, and 32 parts of Carplex #80 (registered trade name of Shionogi & Co., Ltd)., synthetic hydrous silicon oxide fine powder) and 23 parts of 300 mesh diatomaceous earth are added thereto and mixed with a juice mixer to obtain wettable powders.

Formulation Example 23

One part of one hydrazide compound selected from the group consisting of the present hydrazide compounds (1) to (63), 2 parts of clothianidin, 5 parts of synthetic hydrous silicon oxide fine powder, 5 parts of sodium dodecyl benzenesulfonate, 30 parts of bentonite, and 57 parts of clay are added and well stirred and mixed. Then, an appropriate amount of water is added to the mixture, and the mixture is further stirred, made into granules by a granulator, and subjected to ventilation drying to obtain granules.

Formulation Example 24

One and a half parts of one hydrazide compound selected from the group consisting of the present hydrazide compounds (1) to (63), 3 parts of clothianidin, 1 part of synthetic hydrous silicon oxide fine powder, 1 part of Driless B (manufactured by Sankyo Co., Ltd.) as flocculant, and 7 parts of clay are mixed well with a mortar, then stirred and mixed with a juice mixer. A quantity of 86.5 parts of cut clay is added to the resulting mixture, and the mixture is sufficiently stirred and mixed, to obtain dusts.

Formulation Example 25

Five parts of one hydrazide compound selected from the group consisting of the present hydrazide compounds (1) to (63) ; 5 parts of clothianidin; 35 parts of white carbon containing 50 parts of polyoxyethylene alkyl ether sulfate ammonium salt; and 55 parts of water are mixed, and the mixture is finely ground by a wet grinding method to obtain a formulation.

Formulation Example 26

A mixture of 0.2 part of one hydrazide compound selected from the group consisting of the present hydrazide compounds (1) to (63) and 0.3 part of clothianidin is dissolved in 10 parts of dichloromethane, and the solution is mixed with 89.5 parts of Isopar M (isoparaffin: registered trade name of Exxon Chemical Company) to obtain an oil solution.

Formulation Example 27

One twentieth (0.05) part of one hydrazide compound selected from the group consisting of the present hydrazide compounds (1) to (63), 0.05 part of clothianidin, and 49.9 parts of Neochiozol (Chuo Kasei Co., Ltd.) are placed in an aerosol container. After attaching an aerosol valve, the container is charged with 25 parts of dimethyl ether and 25 parts of LPG, and an actuator is attached to the container, to obtain an oil-based aerosol.

Formulation Example 28

An aerosol container is charged with a dissolved mixture of 0.2 part of one hydrazide compound selected from the group consisting of the present hydrazide compounds (1) to (63), 0.4 part of clothianidin, 0.01 part of BHT, 5 parts of xylene, 3.39 parts of deodorized kerosene, and 1 part of an emulsifier {Atmos 300 (registered trade name of Atmos Chemical Co., Ltd.)} and 50 parts of distilled water. A valve part is attached, and 40 parts of a propellant (LPG) is pressurized and charged thereinto via the valve to obtain a water-based aerosol.

Formulation Example 29

Two parts of one hydrazide compound selected from the group consisting of the present hydrazide compounds (1) to (63) and 3 parts of clothianidin are dissolved in 80 parts of diethylene glycol monoethyl ether, and 15 parts of propylene carbonate is mixed with the solution, to obtain a spot-on liquid.

Formulation Example 30

Five parts of one hydrazide compound selected from the group consisting of the present hydrazide compounds (1) to (63) and 5 parts of clothianidin are dissolved in 70 parts of diethylene glycol monoethyl ether, and 20 parts of 2-octyldodecanol is mixed with the solution, to obtain a pour-on liquid.

Formulation Example 31

Sixty parts of Nikkol TEALS-42 (Nikko Chemicals Co., Ltd)., 42% aqueous solution of triethanolamine lauryl sulfate) and 20 parts of propylene glycol are added to a mixture of 0.25 part of one hydrazide compound selected from the group consisting of the present hydrazide compounds (1) to (63) and 0.25 part of clothianidin. After sufficiently stirring and mixing to form a homogeneous solution, 19.5 parts of water is added and further sufficiently stirred and mixed to obtain a shampoo formulation of the homogeneous solution.

Formulation Example 32

Eighty (80) mg of one hydrazide compound selected from the group consisting of the present hydrazide compounds (1) to (63), 20 mg of clothianidin, 68.75 mg of lactose, 237.5 mg of corn starch, 43.75 mg of microcrystalline cellulose, 18.75 mg of polyvinylpyrrolidone, 28.75 mg of sodium carboxymethyl starch, and 2.5 mg of magnesium stearate are mixed completely, and the mixture is compressed, to obtain a tablet of a suitable size.

Formulation Example 33

One hundred (100) mg of one hydrazide compound selected from the group consisting of the present hydrazide compounds (1) to (63), 100 mg of clothianidin, 148 mg of lactose, and 2 mg of magnesium stearate are mixed completely and packed into hard shell gelatin capsules or hydroxypropyl methylcellulose capsules, to obtain a capsule formulation.

Formulation Example 34

Two and a half parts of one hydrazide compound selected from the group consisting of the present hydrazide compounds (1) to (63) and 2.5 parts of clothianidin are dissolved in 5 parts of Polysorbate 85, 3 parts of benzyl alcohol, and 30 parts of propylene glycol, and the solution is adjusted to pH 6.0 to 6.5 using phosphate buffer and then brought to final volume by water, to obtain a liquid for oral administration.

Formulation Example 35

Three and six-tenth (3.6) parts of one hydrazide compound selected from the group consisting of the present hydrazide compounds (1) to (63), 3.6 part of clothianidin, and 92.8 parts of Vosco S-55 (manufactured by Maruishi Pharmaceutical Co. , Ltd.) are dissolved and mixed at 100 °C, poured into a suppository mold, and cooled and solidified to obtain a suppository.

Formulation Example 36

A mixture of 0. 05 g of one hydrazide compound selected from the group consisting of the present hydrazide

compounds (1) to (63) and 0.05 g of clothianidin is dissolved in 2 mL of propylene glycol, and the solution is impregnated into a 4.0 × 4.0 cm, 1.2 cm thick porous ceramic plate, to obtain a heat-type smoking agent.

Formulation Example 37

Two and a half parts of one hydrazide compound selected from the group consisting of the present Hydrazide compounds (1) to (63), 2.5 parts of clothianidin, and 95 parts of ethylene-methyl methacrylate copolymer (ratio of methyl methacrylate in the copolymer: 10% by weight, Acryft WD301, manufactured by Sumitomo Chemical Co., Ltd) are melt-kneaded by a closed-type pressure kneader (manufactured by Moriyama Company Ltd.). The resulting kneaded mixture is extruded from an extrusion molding machine through a molding die to obtain rod-shaped molded articles with 15 cm in length and 3 mm in diameter.

Formulation Example 38

Two and a half parts of one hydrazide compound selected from the group consisting of the present hydrazide compounds (1) to (63), 2.5 parts of clothianidin, and 95 parts of soft vinyl chloride resin are melt-kneaded by a closed-type pressure kneader (manufactured by Moriyama Company Ltd.). The resulting kneaded mixture is extruded from an extrusion molding machine through a molding die to obtain rod-shaped molded articles with 15 cm in length and 3 mm in diameter.

Formulation Example 39

Two parts of one hydrazide compound selected from the group consisting of the present hydrazide compounds (1) to (63), 2 parts of 2-(2,6-difluorophenyl)-4-[4-tert-butyl-2-ethoxyphenyl]-4,5-dihydrooxazole, and 5 parts of clothianidin are dissolved in 37.5 parts of xylene and 37.5 parts of N,N-dimethylformamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 40

Five parts of one hydrazide compound selected from the group consisting of the present hydrazide compounds (1) to (63), 2 parts of 2-(2,6-difluorophenyl)-4-[4-tert-butyl-2-ethoxyphenyl]-4,5-dihydrooxazole, and 2 parts of clothianidin are dissolved in 37.5 parts of xylene and 37.5 parts of N,N-dimethylformamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 41

Five parts of Sorpol 5060 (registered trade name of Toho Chemical Co., Ltd.) is added to a mixture of 20 parts of one hydrazide compound selected from the group consisting of the present hydrazide compounds (1) to (63), 10 parts of 2-(2,6-difluorophenyl)-4-[4-tert-butyl-2-ethoxyphenyl]-4,5-d ihydrooxazole, and 10 parts of clothianidin and mixed well, and 32 parts of Carplex #80 (registered trade name of Shionogi & Co. , Ltd., synthetic hydrous silicon oxide fine powder) and 23 parts of 300 mesh diatomaceous earth are added thereto and mixed with a juice mixer to obtain wettable powders.

Formulation Example 42

One part of one hydrazide compound selected from the group consisting of the present hydrazide compounds (1) to (63), 1 part of 2-(2,6-difluorophenyl)-4-[4-tert-butyl-2-ethoxyphenyl]-4, 5-dihydrooxazole, 1 part of clothianidin, 5 parts of synthetic hydrous silicon oxide fine powder, 5 parts of sodium dodecyl benzenesulfonate, 30 parts of bentonite, and 57 parts of clay are added and well stirred and mixed. Then, an appropriate amount of water is added to the mixture, and the mixture is further stirred, made into granules by a granulator, and subjected to ventilation drying to obtain granules.

Formulation Example 43

One and a half parts of one hydrazide compound selected from the group consisting of the present hydrazide compounds (1) to (63), 1 part of 2-(2,6-difluorophenyl)-4-[4-tert-butyl-2-ethoxyphenyl]-4,5-dihydrooxazole, 2 parts of clothianidin, 1 part of synthetic hydrous silicon oxide fine powder, 1 part of Driless B (manufactured by Sankyo Co. , Ltd.) as flocculant, and 7 parts of clay are mixed well with a mortar, then stirred and mixed with a juice mixer. A quantity

of 86.5 parts of cut clay is added to the resulting mixture, and the mixture is sufficiently stirred and mixed, to obtain dusts. Form

ulation Example 44

Five parts of one hydrazide compound selected from the group consisting of the present hydrazide compounds (1) to (63); 2 parts of 2-(2,6-difluorophenyl)-4-[4-tert-butyl-2-ethoxyphenyl]-4,5-dihydrooxazole; 3 parts of clothianidin; 35 parts of white carbon containing 50 parts of polyoxyethylene alkyl ether sulfate ammonium salt; and 55 parts of water are mixed, and the mixture is finely ground by a wet grinding method to obtain a formulation.

Formulation Example 45

A mixture of 0.2 part of one hydrazide compound selected from the group consisting of the present hydrazide compounds (1) to (63), 0.2 part of 2-(2,6-difluorophenyl)-4-[4-tert-butyl-2-ethoxyphenyl]-4,5-dihydrooxazole, and 0.1 part of clothianidin is dissolved in 10 parts of dichloromethane, and the solution is mixed with 89.5 parts of Isopar M (isoparaffin: registered trade name of Exxon Chemical Company) to obtain an oil solution.

Formulation Example 46

One twentieth (0.05) part of one hydrazide compound selected from the group consisting of the present hydrazide compounds (1) to (63), 0.05 part of cypermethrin, 0.02 part of 2-(2,6-difluorophenyl)-4-[4-tert-butyl-2-ethoxyphenyl]-4,5-d ihydrooxazole, 0.03 part of clothianidin, and 49.9 parts of Neochiozol (Chuo Kasei Co., Ltd.) are placed in an aerosol container. After attaching an aerosol valve, the container is charged with 25 parts of dimethyl ether and 25 parts of LPG, and an actuator is attached to the container, to obtain an oil-based aerosol.

Formulation Example 47

An aerosol container is charged with a dissolved mixture of 0.2 part of one hydrazide compound selected from the group consisting of the present hydrazide compounds (1) to (63), 0.2 part of 2-(2,6-difluorophenyl)-4-[4-tert-butyl-2-ethoxyphenyl]-4,5-dihydrooxazole, 0.2 part of clothianidin, 0.01 part of BHT, 5 parts of xylene, 3.39 parts of deodorized kerosene, and 1 part of an emulsifier {Atmos 300 (registered trade name of Atmos Chemical Co., Ltd.)} and 50 parts of distilled water. A valve part is attached, and 40 parts of a propellant (LPG) is pressurized and charged thereinto via the valve to obtain a water-based aerosol.

Formulation Example 48

Two parts of one hydrazide compound selected from the group consisting of the present hydrazide compounds (1) to (63), 2 parts of 2-(2,6-difluorophenyl)-4-[4-tert-butyl-2-ethoxyphenyl]-4, 5-dihydrooxazole, and 1 part of clothianidin are dissolved in 80 parts of diethylene glycol monoethyl ether, and 15 parts of propylene carbonate is mixed with the solution, to obtain a spot-on liquid.

Formulation Example 49

Five parts of one hydrazide compound selected from the group consisting of the present hydrazide compounds (1) to (63), 2 parts of 2-(2,6-difluorophenyl)-4-[4-tert-butyl-2-ethoxyphenyl]-4,5-dihydrooxazole, and 3 parts of clothianidin are dissolved in 70 parts of diethylene glycol monoethyl ether, and 20 parts of 2-octyldodecanol is mixed with the solution, to obtain a pour-on liquid.

Formulation Example 50

Sixty parts of Nikkol TEALS-42 (Nikko Chemicals Co., Ltd., 42% aqueous solution of triethanolamine lauryl sulfate) and 20 parts of propylene glycol are added to a mixture of 0.25 part of one hydrazide compound selected from the group consisting of the present hydrazide compounds (1) to (63), 0.1 part of 2-(2,6-difluorophenyl)-4-[4-tert-butyl-2-ethoxyphenyl]-4,5-d ihydrooxazole, and 0.15 part of clothianidin. After sufficiently stirring and mixing to form a homogeneous solution, 19.5 parts of water is added and further sufficiently stirred and mixed to obtain a shampoo formulation of the homogeneous solution.

Formulation Example 51

Eighty (80) mg of one hydrazide compound selected from the group consisting of the present hydrazide compounds (1) to (63), 10 mg of 2-(2,6-difluorophenyl)-4-[4-tert-butyl-2-ethoxyphenyl]-4,5-d ihydrooxazole, 10 mg of clothianidin, 68.75 mg of lactose, 237.5 mg of corn starch, 43.75 mg of microcrystalline cellulose, 18.75 mg of polyvinylpyrrolidone, 28.75 mg of sodium carboxymethyl starch, and 2.5 mg of magnesium stearate are mixed completely, and the mixture is compressed, to obtain a tablet of a suitable size.

Formulation Example 52

One hundred (100) mg of one hydrazide compound selected from the group consisting of the present Hydrazide compounds (1) to (63), 50 mg of 2-(2,6-difluorophenyl)-4-[4-tert-butyl-2-ethoxyphenyl]-4,5-dihydrooxazole, 50 mg of clothianidin, 148 mg of lactose, and 2 mg of magnesium stearate are mixed completely and packed into hard shell gelatin capsules or hydroxypropyl methylcellulose capsules, to obtain a capsule formulation.

Formulation Example 53

Two and a half parts of one hydrazide compound selected from the group consisting of the present hydrazide compounds (1) to (63), 1 part of 2-(2,6-difluorophenyl)-4-[4-tert-butyl-2-ethoxyphenyl]-4,5-dihydrooxazole, and 1.5 parts of clothianidin are dissolved in 5 parts of Polysorbate 85, 3 parts of benzyl alcohol, and 30 parts of propylene glycol, and the solution is adjusted to pH 6.0 to 6.5 using phosphate buffer and then brought to final volume by water, to obtain a liquid for oral administration.

Formulation Example 54

Three and six-tenth (3.6) parts of one hydrazide compound selected from the group consisting of the present hydrazide compounds (1) to (63), 1.6 parts of 2-(2,6-difluorophenyl)-4-[4-tert-butyl-2-ethoxyphenyl]-4,5-dihydrooxazole, 2 parts of clothianidin, and 92.8 parts of Vosco S-55 (manufactured by Maruishi Pharmaceutical Co., Ltd.) are dissolved and mixed at 100°C, poured into a suppository mold, and cooled and solidified to obtain a suppository.

Formulation Example 55

A mixture of 0.05 g of one hydrazide compound selected from the group consisting of the present hydrazide compounds (1) to (63), 0.02 g of 2-(2,6-difluorophenyl)-4-[4-tert-butyl-2-ethoxyphenyl]-4,5-dihydrooxazole, and 0.03 g of clothianidin is dissolved in 2 mL of propylene glycol, and the solution is impregnated into a 4.0 × 4.0 cm, 1.2 cm thick porous ceramic plate, to obtain a heat-type smoking agent.

Formulation Example 56

Two and a half parts of one hydrazide compound selected from the group consisting of the present hydrazide compounds (1) to (63), 1 part of 2-(2,6-difluorophenyl)-4-[4-tert-butyl-2-ethoxyphenyl]-4,5-dihydrooxazole, 1.5 parts of clothianidin, and 95 parts of ethylene-methyl methacrylate copolymer (ratio of methyl methacrylate in the copolymer: 10% by weight, Acryft WD301, manufactured by Sumitomo Chemical Co., Ltd) are melt-kneaded by a closed-type pressure kneader (manufactured by Moriyama Company Ltd.). The resulting kneaded mixture is extruded from an extrusion molding machine through a molding die to obtain rod-shaped molded articles with 15 cm in length and 3 mm in diameter.

Formulation Example 57

Two and a half parts of one hydrazide compound selected from the group consisting of the present hydrazide compounds (1) to (63), 1 part of 2-(2,6-difluorophenyl)-4-[4-tert-butyl-2-ethoxyphenyl] -4, 5-dihydrooxazole, 1. 5 parts of clothianidin, and 95 parts of soft vinyl chloride resin are melt-kneaded by a closed-type pressure kneader (manufactured by Moriyama Company Ltd.). The resulting kneaded mixture is extruded from an extrusion molding machine through a molding die to obtain rod-shaped molded articles with 15 cm in length and 3 mm in diameter.

Formulation Example 58

Two parts of one hydrazide compound selected from the group consisting of the present hydrazide compounds

(1) to (63) and 7 parts of imidacloprid are dissolved in 37.5 parts of xylene and 37.5 parts of N,N-dimethylformamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 59

Five parts of one hydrazide compound selected from the group consisting of the present Hydrazide compounds (1) to (63) and 4 parts of imidacloprid are dissolved in 37.5 parts of xylene and 37.5 parts of N,N-dimethylformamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 60

Five parts of Sorpol 5060 (registered trade name of Toho Chemical Co., Ltd.) is added to a mixture of 20 parts of one hydrazide compound selected from the group consisting of the present hydrazide compounds (1) to (63) and 20 parts of imidacloprid and mixed well, and 32 parts of Carplex #80 (registered trade name of Shionogi & Co., Ltd)., synthetic hydrous silicon oxide fine powder) and 23 parts of 300 mesh diatomaceous earth are added thereto and mixed with a juice mixer to obtain wettable powders.

Formulation Example 61

One part of one hydrazide compound selected from the group consisting of the present hydrazide compounds (1) to (63), 2 parts of imidacloprid, 5 parts of synthetic hydrous silicon oxide fine powder, 5 parts of sodium dodecyl benzenesulfonate, 30 parts of bentonite, and 57 parts of clay are added and well stirred and mixed. Then, an appropriate amount of water is added to the mixture, and the mixture is further stirred, made into granules by a granulator, and subjected to ventilation drying to obtain granules.

Formulation Example 62

One and a half parts of one hydrazide compound selected from the group consisting of the present hydrazide compounds (1) to (63), 3 parts of imidacloprid, 1 part of synthetic hydrous silicon oxide fine powder, 1 part of Driless B (manufactured by Sankyo Co., Ltd.) as flocculant, and 7 parts of clay are mixed well with a mortar, then stirred and mixed with a juice mixer. A quantity of 86.5 parts of cut clay is added to the resulting mixture, and the mixture is sufficiently stirred and mixed, to obtain dusts.

Formulation Example 63

Five parts of one hydrazide compound selected from the group consisting of the present hydrazide compounds (1) to (63) ; 5 parts of imidacloprid; 35 parts of white carbon containing 50 parts of polyoxyethylene alkyl ether sulfate ammonium salt; and 55 parts of water are mixed, and the mixture is finely ground by a wet grinding method to obtain a formulation.

Formulation Example 64

A mixture of 0.2 part of one hydrazide compound selected from the group consisting of the present hydrazide compounds (1) to (63) and 0.3 part of imidacloprid, is dissolved in 10 parts of dichloromethane, and the solution is mixed with 89.5 parts of Isopar M (isoparaffin: registered trade name of Exxon Chemical Company) to obtain an oil solution.

Formulation Example 65

One twentieth (0.05) part of one hydrazide compound selected from the group consisting of the present hydrazide compounds (1) to (63), 0.05 part of imidacloprid, and 49.9 parts of Neochiozol (Chuo Kasei Co., Ltd.) are placed in an aerosol container. After attaching an aerosol valve, the container is charged with 25 parts of dimethyl ether and 25 parts of LPG, and an actuator is attached to the container, to obtain an oil-based aerosol.

Formulation Example 6

An aerosol container is charged with a dissolved mixture of 0.2 part of one hydrazide compound selected from

the group consisting of the present hydrazide compounds (1) to (63), 0.4 part of imidacloprid, 0.01 part of BHT, 5 parts of xylene, 3.39 parts of deodorized kerosene, and 1 part of an emulsifier {Atmos 300 (registered trade name of Atmos Chemical Co., Ltd.)} and 50 parts of distilled water. A valve part is attached, and 40 parts of a propellant (LPG) is pressurized and charged thereinto via the valve to obtain a water-based aerosol.

Formulation Example 67

Two parts of one hydrazide compound selected from the group consisting of the present hydrazide compounds (1) to (63) and 3 parts of imidacloprid are dissolved in 80 parts of diethylene glycol monoethyl ether, and 15 parts of propylene carbonate is mixed with the solution, to obtain a spot-on liquid.

Formulation Example 68

Five parts of one hydrazide compound selected from the group consisting of the present hydrazide compounds (1) to (63) and 5 parts of imidacloprid are dissolved in 70 parts of diethylene glycol monoethyl ether, and 20 parts of 2-octyldodecanol is mixed with the solution, to obtain a pour-on liquid.

Formulation Example 69

Sixty parts of Nikkol TEALS-42 (Nikko Chemicals Co., Ltd., 42% aqueous solution of triethanolamine lauryl sulfate) and 20 parts of propylene glycol are added to a mixture of 0.25 part of one hydrazide compound selected from the group consisting of the present hydrazide compounds (1) to (63) and 0.25 part of imidacloprid. After sufficiently stirring and mixing to form a homogeneous solution, 19.5 parts of water is added and further sufficiently stirred and mixed to obtain a shampoo formulation of the homogeneous solution.

Formulation Example 70

Eighty (80) mg of one hydrazide compound selected from the group consisting of the present hydrazide compounds (1) to (63), 20 mg of imidacloprid, 68.75 mg of lactose, 237.5 mg of corn starch, 43.75 mg of microcrystalline cellulose, 18.75 mg of polyvinylpyrrolidone, 28.75 mg of sodium carboxymethyl starch, and 2.5 mg of magnesium stearate are mixed completely, and the mixture is compressed, to obtain a tablet of a suitable size.

Formulation Example 71

One hundred (100) mg of one hydrazide compound selected from the group consisting of the present hydrazide compounds (1) to (63), 100 mg of imidacloprid, 148 mg of lactose, and 2 mg of magnesium stearate are mixed completely and packed into hard shell gelatin capsules or hydroxypropyl methylcellulose capsules, to obtain a capsule formulation.

Formulation Example 72

Two and a half parts of one hydrazide compound selected from the group consisting of the present hydrazide compounds (1) to (63) and 2.5 parts of imidacloprid are dissolved in 5 parts of Polysorbate 85, 3 parts of benzyl alcohol, and 30 parts of propylene glycol, and the solution is adjusted to pH 6.0 to 6.5 using phosphate buffer and then brought to final volume by water, to obtain a liquid for oral administration.

Formulation Example 73

Three and six-tenth (3.6) parts of one hydrazide compound selected from the group consisting of the present hydrazide compounds (1) to (63), 3.6 parts of imidacloprid, and 92.8 parts of Vosco S-55 (manufactured by Maruishi Pharmaceutical Co., Ltd.) are dissolved and mixed at 100°C, poured into a suppository mold, and cooled and solidified to obtain a suppository.

Formulation Example 74

A mixture of 0.05 g of one hydrazide compound selected from the group consisting of the present Hydrazide compounds (1) to (63) and 0.05 g of imidacloprid is dissolved in 2 mL of propylene glycol, and the solution is impregnated into a 4.0 × 4.0 cm, 1.2 cm thick porous ceramic plate, to obtain a heat-type smoking agent.

Formulation Example 75

Two and a half parts of one hydrazide compound selected from the group consisting of the present hydrazide compounds (1) to (63), 2.5 parts of imidacloprid, and 95 parts of ethylene-methyl methacrylate copolymer (ratio of methyl methacrylate in the copolymer: 10% by weight, Acryft WD301, manufactured by Sumitomo Chemical Co., Ltd) are melt-kneaded by a closed-type pressure kneader (manufactured by Moriyama Company Ltd.). The resulting kneaded mixture is extruded from an extrusion molding machine through a molding die to obtain rod-shaped molded articles with 15 cm in length and 3 mm in diameter.

Formulation Example 76

Two and a half parts of one hydrazide compound selected from the group consisting of the present hydrazide compounds (1) to (63), 2.5 parts of imidacloprid, and 95 parts of soft vinyl chloride resin are melt-kneaded by a closed-type pressure kneader (manufactured by Moriyama Company Ltd.). The resulting kneaded mixture is extruded from an extrusion molding machine through a molding die to obtain rod-shaped molded articles with 15 cm in length and 3 mm in diameter.

Formulation Example 77

Two parts of one hydrazide compound selected from the group consisting of the present hydrazide compounds (1) to (63) and 7 parts of thiacloprid are dissolved in 37.5 parts of xylene and 37.5 parts of N,N-dimethylformamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 78

Five parts of one hydrazide compound selected from the group consisting of the present hydrazide compounds (1) to (63) and 4 parts of thiacloprid are dissolved in 37.5 parts of xylene and 37.5 parts of N,N-dimethylformamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 79

Five parts of Sorpol 5060 (registered trade name of Toho Chemical Co., Ltd.) is added to a mixture of 20 parts of one hydrazide compound selected from the group consisting of the present hydrazide compounds (1) to (63) and 20 parts of thiacloprid and mixed well, and 32 parts of Carplex #80 (registered trade name of Shionogi & Co., Ltd)., synthetic hydrous silicon oxide fine powder) and 23 parts of 300 mesh diatomaceous earth are added thereto and mixed with a juice mixer to obtain wettable powders.

Formulation Example 80

One part of one hydrazide compound selected from the group consisting of the present hydrazide compounds (1) to (63), 2 parts of thiacloprid, 5 parts of synthetic hydrous silicon oxide fine powder, 5 parts of sodium dodecyl benzenesulfonate, 30 parts of bentonite, and 57 parts of clay are added and well stirred and mixed. Then, an appropriate amount of water is added to the mixture, and the mixture is further stirred, made into granules by a granulator, and subjected to ventilation drying to obtain granules.

Formulation Example 81

One and a half parts of one hydrazide compound selected from the group consisting of the present hydrazide compounds (1) to (63), 3 parts of thiacloprid, 1 part of synthetic hydrous silicon oxide fine powder, 1 part of Driless B (manufactured by Sankyo Co., Ltd.) as flocculant, and 7 parts of clay are mixed well with a mortar, then stirred and mixed with a juice mixer. A quantity of 86.5 parts of cut clay is added to the resulting mixture, and the mixture is sufficiently stirred and mixed, to obtain dusts.

Formulation Example 82

Five parts of one hydrazide compound selected from the group consisting of the present Hydrazide compounds

(1) to (63); 5 parts of thiacloprid; 35 parts of white carbon containing 50 parts of polyoxyethylene alkyl ether sulfate ammonium salt; and 55 parts of water are mixed, and the mixture is finely ground by a wet grinding method to obtain a formulation.

Formulation Example 83

A mixture of 0.2 part of one hydrazide compound selected from the group consisting of the present hydrazide compounds (1) to (63) and 0.3 part of thiacloprid is dissolved in 10 parts of dichloromethane, and the solution is mixed with 89.5 parts of Isopar M (isoparaffin: registered trade name of Exxon Chemical Company) to obtain an oil solution.

Formulation Example 84

One twentieth (0.05) part of one Hydrazide compound selected from the group consisting of the present hydrazide compounds (1) to (63), 0.05 part of thiacloprid, and 49.9 parts of Neochiozol (Chuo Kasei Co., Ltd.) are placed in an aerosol container. After attaching an aerosol valve, the container is charged with 25 parts of dimethyl ether and 25 parts of LPG, and an actuator is attached to the container, to obtain an oil-based aerosol.

Formulation Example 85

An aerosol container is charged with a dissolved mixture of 0.2 part of one hydrazide compound selected from the group consisting of the present hydrazide compounds (1) to (63), 0.4 part of thiacloprid, 0.01 part of BHT, 5 parts of xylene, 3.39 parts of deodorized kerosene, and 1 part of an emulsifier {Atmos 300 (registered trade name of Atmos Chemical Co., Ltd.)} and 50 parts of distilled water. A valve part is attached, and 40 parts of a propellant (LPG) is pressurized and charged thereinto via the valve to obtain a water-based aerosol.

Formulation Example 86

Two parts of one hydrazide compound selected from the group consisting of the present hydrazide compounds (1) to (63) and 3 parts of thiacloprid are dissolved in 80 parts of diethylene glycol monoethyl ether, and 15 parts of propylene carbonate is mixed with the solution, to obtain a spot-on liquid.

Formulation Example 87

Five parts of one hydrazide compound selected from the group consisting of the present hydrazide compounds (1) to (63) and 5 parts of thiacloprid are dissolved in 70 parts of diethylene glycol monoethyl ether, and 20 parts of 2-octyldodecanol is mixed with the solution, to obtain a pour-on liquid.

Formulation Example 88

Sixty parts of Nikkol TEALS-42 (Nikko Chemicals Co., Ltd. , 42% aqueous solution of triethanolamine lauryl sulfate) and 20 parts of propylene glycol are added to a mixture of 0.25 part of one hydrazide compound selected from the group consisting of the present hydrazide compounds (1) to (63) and 0.25 part of thiacloprid. After sufficiently stirring and mixing to form a homogeneous solution, 19.5 parts of water is added and further sufficiently stirred and mixed to obtain a shampoo formulation of the homogeneous solution.

Formulation Example 89

Eighty (80) mg of one hydrazide compound selected from the group consisting of the present hydrazide compounds (1) to (63), 20 mg of thiacloprid, 68.75 mg of lactose, 237. 5 mg of corn starch, 43.75 mg of microcrystalline cellulose, 18.75 mg of polyvinyl pyrrolidone, 28.75 mg of sodium carboxymethyl starch, and 2.5 mg of magnesium stearate are mixed completely, and the mixture is compressed, to obtain tablets of a suitable size.

Formulation Example 90

One hundred (100) mg of one hydrazide compound selected from the group consisting of the present hydrazide compounds (1) to (63), 100 mg of thiacloprid, 148 mg of lactose, and 2 mg of magnesium stearate are mixed completely and packed into hard shell gelatin capsules or hydroxypropyl methylcellulose capsules, to obtain a capsule formulation.

Formulation Example 91

Two and a half parts of one hydrazide compound selected from the group consisting of the present hydrazide compounds (1) to (63) and 2.5 parts of thiacloprid are dissolved in 5 parts of Polysorbate 85, 3 parts of benzyl alcohol, and 30 parts of propylene glycol, and the solution is adjusted to pH 6.0 to 6.5 using phosphate buffer and then brought to final volume by water, to obtain a liquid for oral administration.

Formulation Example 92

Three and six-tenth (3.6) parts of one hydrazide compound selected from the group consisting of the present hydrazide compounds (1) to (63), 3.6 parts of thiacloprid, and 92.8 parts of Vosco S-55 (manufactured by Maruishi Pharmaceutical Co., Ltd.) are dissolved and mixed at 100°C, poured into a suppository mold, and cooled and solidified to obtain a suppository.

Formulation Example 93

A mixture of 0.05 g of one hydrazide compound selected from the group consisting of the present hydrazide compounds (1) to (63) and 0.05 g of thiacloprid is dissolved in 2 mL of propylene glycol, and the solution is impregnated into a 4.0 $\times$ 4.0 cm, 1.2 cm thick porous ceramic plate, to obtain a heat-type smoking agent.

Formulation Example 94

Two and a half parts of one hydrazide compound selected from the group consisting of the present hydrazide compounds (1) to (63), 2. 5 parts of thiacloprid, and 95 parts of ethylene-methyl methacrylate copolymer (ratio of methyl methacrylate in the copolymer: 10% by, Acryft WD301, manufactured by Sumitomo Chemical Co., Ltd) are melt-kneaded by a closed-type pressure kneader (manufactured by Moriyama Company Ltd.). The resulting kneaded mixture is extruded from an extrusion molding machine through a molding dine to obtain rod-shaped molded articles with 15 cm in length and 3 mm in diameter.

Formulation Example 95

Two and a half parts of one hydrazide compound selected from the group consisting of the present hydrazide compounds (1) to (63), 2.5 parts of thiacloprid, and 95 parts of soft vinyl chloride resin are melt-kneaded by a closed-type pressure kneader (manufactured by Moriyama Company Ltd.). The resulting kneaded mixture is extruded from an extrusion molding machine through a molding die to obtain rod-shaped molded articles with 15 cm in length and 3 mm in diameter.

Formulation Example 96

Two parts of one hydrazide compound selected from the group consisting of the present hydrazide compounds (1) to (63) and 7 parts of acetamiprid are dissolved in 37.5 parts of xylene and 37.5 parts of N,N-dimethylformamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 97

Five parts of one hydrazide compound selected from the group consisting of the present hydrazide compounds (1) to (63) and 4 parts of acetamiprid are dissolved in 37.5 parts of xylene and 37.5 parts of N,N-dimethylformamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 98

Five parts of Sorpol 5060 (registered trade name of Toho Chemical Co., Ltd.) is added to a mixture of 20 parts of one hydrazide compound selected from the group consisting of the present hydrazide compounds (1) to (63) and 20 parts of acetamiprid and mixed well, and 32 parts of Carplex #80 (registered trade name of Shionogi & Co. , Ltd., synthetic hydrous silicon oxide fine powder) and 23 parts of 300 mesh diatomaceous earth are added thereto and mixed with a juice mixer to obtain wettable powders.

Formulation Example 99

One part of one hydrazide compound selected from the group consisting of the present hydrazide compounds (1) to (63), 2 parts of acetamiprid, 5 parts of synthetic hydrous silicon oxide fine powder, 5 parts of sodium dodecyl benzenesulfonate, 30 parts of bentonite, and 57 parts of clay are added and well stirred and mixed. Then, an appropriate amount of water is added to the mixture, and the mixture is further stirred, made into granules by a granulator, and subjected to ventilation drying to obtain granules.

Formulation Example 100

One and a half parts of one hydrazide compound selected from the group consisting of the present hydrazide compounds (1) to (63), 3 parts of acetamiprid, 1 part of synthetic hydrous silicon oxide fine powder, 1 part of Driless B (manufactured by Sankyo Co., Ltd.) as flocculant, and 7 parts of clay are mixed well with a mortar, then stirred and mixed with a juice mixer. A quantity of 86.5 parts of cut clay is added to the resulting mixture, and the mixture is sufficiently stirred and mixed, to obtain dusts.

Formulation Example 101

Five parts of one hydrazide compound selected from the group consisting of the present hydrazide compounds (1) to (63); 5 parts of acetamiprid; 35 parts of white carbon containing 50 parts of polyoxyethylene alkyl ether sulfate ammonium salt; and 55 parts of water are mixed, and the mixture is finely ground by a wet grinding method to obtain a formulation.

Formulation Example 102

A mixture of 0.2 part of one hydrazide compound selected from the group consisting of the present hydrazide compounds (1) to (63) and 0.3 part of acetamiprid is dissolved in 10 parts of dichloromethane, and the solution is mixed with 89.5 parts of Isopar M (isoparaffin: registered trade name of Exxon Chemical Company) to obtain an oil solution.

Formulation Example 103

One twentieth (0.05) part of one hydrazide compound selected from the group consisting of the present hydrazide compounds (1) to (63), 0.05 part of acetamiprid, and 49.9 parts of Neochiozol (Chuo Kasei Co., Ltd.) are placed in an aerosol container. After attaching an aerosol valve, the container is charged with 25 parts of dimethyl ether and 25 parts of LPG, and an actuator is attached to the container, to obtain an oil -based aerosol.

Formulation Example 104

An aerosol container is charged with a dissolved mixture of 0.2 part of one hydrazide compound selected from the group consisting of the present hydrazide compounds (1) to (63), 0.4 part of acetamiprid, 0.01 part of BHT, 5 parts of xylene, 3.39 parts of deodorized kerosene, and 1 part of an emulsifier {Atmos 300 (registered trade name of Atmos Chemical Co., Ltd.)} and 50 parts of distilled water. A valve part is attached, and 40 parts of a propellant (LPG) is pressurized and charged thereinto via the valve to obtain a water-based aerosol.

Formulation Example 105

Two parts of one hydrazide compound selected from the group consisting of the present hydrazide compounds (1) to (63) and 3 parts of acetamiprid are dissolved in 80 parts of diethylene glycol monoethyl ether, and 15 parts of propylene carbonate is mixed with the solution, to obtain a spot-on liquid.

Formulation Example 106

Five parts of one hydrazide compound selected from the group consisting of the present hydrazide compounds (1) to (63) and 5 parts of acetamiprid are dissolved in 70 parts of diethylene glycol monoethyl ether, and 20 parts of 2-octyldodecanol is mixed with the solution, to obtain a pour-on liquid.

Formulation Example 107

Sixty parts of Nikkol TEALS-42 (Nikko Chemicals Co. , Ltd. , 42% aqueous solution of triethanolamine lauryl sulfate) and 20 parts of propylene glycol are added to a mixture of 0.25 part of one hydrazide compound selected from the group consisting of the present hydrazide compounds (1) to (63) and 0.25 part of acetamiprid. After sufficiently stirring and mixing to form a homogeneous solution, 19.5 parts of water is added and further sufficiently stirred and mixed to obtain a shampoo formulation of the homogeneous solution.

Formulation Example 108

Eighty (80) mg of one hydrazide compound selected from the group consisting of the present hydrazide compounds (1) to (63), 20 mg of acetamiprid, 68.75 mg of lactose, 237.5 mg of corn starch, 43.75 mg of microcrystalline cellulose, 18.75 mg of polyvinyl pyrrolidone, 28.75 mg of sodium carboxymethyl starch, and 2.5 mg of magnesium stearate are mixed completely, and the mixture is compressed, to obtain tablets of a suitable size.

Formulation Example 109

One hundred (100) mg of one hydrazide compound selected from the group consisting of the present hydrazide compounds (1) to (63), 100 mg of acetamiprid, 148 mg of lactose, and 2 mg of magnesium stearate are mixed completely and packed into hard shell gelatin capsules or hydroxypropyl methylcellulose capsules, to obtain a capsule formulation.

Formulation Example 110

Two and a half parts of one hydrazide compound selected from the group consisting of the present hydrazide compounds (1) to (63) and 2.5 parts of acetamiprid are dissolved in 5 parts of Polysorbate 85, 3 parts of benzyl alcohol, and 30 parts of propylene glycol, and the solution is adjusted to pH 6.0 to 6.5 using phosphate buffer and then brought to final volume by water, to obtain a liquid for oral administration.

Formulation Example 11

Three and six-tenth (3.6) parts of one hydrazide compound selected from the group consisting of the present hydrazide compounds (1) to (63), 3.6 parts of acetamiprid, and 92.8 parts of Vosco S-55 (manufactured by Maruishi Pharmaceutical Co. , Ltd.) are dissolved and mixed at 100°C, poured into a suppository mold, and cooled and solidified to obtain a suppository.

Formulation Example 112

A mixture of 0.05 g of one hydrazide compound selected from the group consisting of the present hydrazide compounds (1) to (63) and 0.05 g of acetamiprid is dissolved in 2 mL of propylene glycol, and the solution is impregnated into a 4.0 × 4.0 cm, 1.2 cm thick porous ceramic plate, to obtain a heat-type smoking agent.

Formulation Example 113

Two and a half parts of one hydrazide compound selected from the group consisting of the present hydrazide compounds (1) to (63), 2.5 parts of acetamiprid, and 95 parts of ethylene-methyl methacrylate copolymer (ratio of methyl methacrylate in the copolymer: 10% by, Acryft WD301, manufactured by Sumitomo Chemical Co., Ltd) are melt-kneaded by a closed-type pressure kneader (manufactured by Moriyama Company Ltd.). The resulting kneaded mixture is extruded from an extrusion molding machine through a molding die to obtain rod-shaped molded articles with 15 cm in length and 3 mm in diameter.

Formulation Example 114

Two and a half parts of one hydrazide compound selected from the group consisting of the present hydrazide compounds (1) to (63), 2.5 parts of acetamiprid, and 95 parts of soft vinyl chloride resin are melt-kneaded by a closed-type pressure kneader (manufactured by Moriyama Company Ltd.). The resulting kneaded mixture is extruded from an extrusion molding machine through a molding die to obtain rod-shaped molded articles with 15 cm in length and 3 mm in diameter.

Formulation Example 115

Two parts of one hydrazide compound selected from the group consisting of the present hydrazide compounds (1) to (63) and 7 parts of nitenpyram are dissolved in 37.5 parts of xylene and 37.5 parts of N, N-dimethylf ormamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 116

Five parts of one hydrazide compound selected from the group consisting of the present Hydrazide compounds (1) to (63) and 4 parts of nitenpyram are dissolved in 37.5 parts of xylene and 37.5 parts of N,N-dimethylformamide. To this solution are added 10 parts of polyoxyethylene styryl phenyl ether and 6 parts of calcium dodecyl benzenesulfonate, and the mixture is well stirred and mixed to obtain an emulsifiable concentrate.

Formulation Example 117

Five parts of Sorpol 5060 (registered trade name of Toho Chemical Co., Ltd.) is added to a mixture of 20 parts of one hydrazide compound selected from the group consisting of the present hydrazide compounds (1) to (63) and 20 parts of nitenpyram and mixed well, and 32 parts of Carplex #80 (registered trade name of Shionogi & Co., Ltd., synthetic hydrous silicon oxide fine powder) and 23 parts of 300 mesh diatomaceous earth are added thereto and mixed with a juice mixer to obtain wettable powders.

Formulation Example 118

One part of one hydrazide compound selected from the group consisting of the present hydrazide compounds (1) to (63), 2 parts of nitenpyram, 5 parts of synthetic hydrous silicon oxide fine powder, 5 parts of sodium dodecyl benzenesulfonate, 30 parts of bentonite, and 57 parts of clay are added and well stirred and mixed. Then, an appropriate amount of water is added to the mixture, and the mixture is further stirred, made into granules by a granulator, and subjected to ventilation drying to obtain granules.

Formulation Example 119

One and a half parts of one hydrazide compound selected from the group consisting of the present hydrazide compounds (1) to (63), 3 parts of nitenpyram, 1 part of synthetic hydrous silicon oxide fine powder, 1 part of Driless B (manufactured by Sankyo Co., Ltd.) as flocculant, and 7 parts of clay are mixed well with a mortar, then stirred and mixed with a juice mixer. A quantity of 86.5 parts of cut clay is added to the resulting mixture, and the mixture is sufficiently stirred and mixed, to obtain dusts.

Formulation Example 120

Five parts of one hydrazide compound selected from the group consisting of the present hydrazide compounds (1) to (63); 5 parts of nitenpyram; 35 parts of white carbon containing 50 parts of polyoxyethylene alkyl ether sulfate ammonium salt; and 55 parts of water are mixed, and the mixture is finely ground by a wet grinding method to obtain a formulation.

Formulation Example 121

A mixture of 0.2 part of one hydrazide compound selected from the group consisting of the present hydrazide compounds (1) to (63) and 0.3 part of nitenpyram is dissolved in 10 parts of dichloromethane, and the solution is mixed with 89.5 parts of Isopar M (isoparaffin: registered trade name of Exxon Chemical Company) to obtain an oil solution.

Formulation Example 122

One twentieth (0.05) part of one hydrazide compound selected from the group consisting of the present hydrazide compounds (1) to (63), 0.05 part of nitenpyram, and 49.9 parts of Neochiozol (Chuo Kasei Co., Ltd.) are placed in an aerosol container. After attaching an aerosol valve, the container is charged with 25 parts of dimethyl ether and 25 parts of LPG, and an actuator is attached to the container, to obtain an oil-based aerosol.

Formulation Example 123

An aerosol container is charged with a dissolved mixture of 0.2 part of one hydrazide compound selected from the group consisting of the present hydrazide compounds (1) to (63), 0.4 part of nitenpyram, 0.01 part of BHT, 5 parts of xylene, 3.39 parts of deodorized kerosene, and 1 part of an emulsifier {Atmos 300 (registered trade name of Atmos Chemical Co., Ltd.)} and 50 parts of distilled water. A valve part is attached, and 40 parts of a propellant (LPG) is pressurized and charged thereinto via the valve to obtain a water-based aerosol.

Formulation Example 124

Two parts of one hydrazide compound selected from the group consisting of the present hydrazide compounds (1) to (63) and 3 parts of nitenpyram are dissolved in 80 parts of diethylene glycol monoethyl ether, and 15 parts of propylene carbonate is mixed with the solution, to obtain a spot-on liquid.

Formulation Example 125

Five parts of one hydrazide compound selected from the group consisting of the present hydrazide compounds (1) to (63) and 5 parts of nitenpyram are dissolved in 70 parts of diethylene glycol monoethyl ether, and 20 parts of 2-octyldodecanol is mixed with the solution, to obtain a pour-on liquid.

Formulation Example 126

Sixty parts of Nikkol TEALS-42 (Nikko Chemicals Co. , Ltd)., 42% aqueous solution of triethanolamine lauryl sulfate) and 20 parts of propylene glycol are added to a mixture of 0.25 part of one hydrazide compound selected from the group consisting of the present hydrazide compounds (1) to (63) and 0.25 part of nitenpyram. After sufficiently stirring and mixing to form a homogeneous solution, 19.5 parts of water is added and further sufficiently stirred and mixed to obtain a shampoo formulation of the homogeneous solution.

Formulation Example 127

Eighty (80) mg of one hydrazide compound selected from the group consisting of the present hydrazide compounds (1) to (63), 20 mg of nitenpyram, 68.75 mg of lactose, 237.5 mg of corn starch, 43.75 mg of microcrystalline cellulose, 18.75 mg of polyvinyl pyrrolidone, 28.75 mg of sodium carboxymethyl starch, and 2.5 mg of magnesium stearate are mixed completely, and the mixture is compressed, to obtain tablets of a suitable size.

Formulation Example 128

One hundred (100) mg of one hydrazide compound selected from the group consisting of the present hydrazide compounds (1) to (63), 100 mg of nitenpyram, 148 mg of lactose, and 2 mg of magnesium stearate are mixed completely and packed into hard shell gelatin capsules or hydroxypropyl methylcellulose capsules, to obtain a capsule formulation.

Formulation Example 129

Two and a half parts of one hydrazide compound selected from the group consisting of the present hydrazide compounds (1) to (63) and 2.5 parts of nitenpyram are dissolved in 5 parts of Polysorbate 85, 3 parts of benzyl alcohol, and 30 parts of propylene glycol, and the solution is adjusted to pH 6.0 to 6.5 using phosphate buffer and then brought to final volume by water, to obtain a liquid for oral administration.

Formulation Example 130

Three and six-tenth (3.6) parts of one hydrazide compound selected from the group consisting of the present hydrazide compounds (1) to (63), 3.6 parts of nitenpyram, and 92.8 parts of Vosco S-55 (manufactured by Maruishi Pharmaceutical Co. , Ltd.) are dissolved and mixed at 100°C, poured into a suppository mold, and cooled and solidified to obtain a suppository.

Formulation Example 131

A mixture of 0.05 g of one hydrazide compound selected from the group consisting of the present hydrazide

compounds (1) to (63) and 0.05 g of nitenpyram is dissolved in 2 mL of propylene glycol, and the solution is impregnated into a 4.0 × 4.0 cm, 1.2 cm thick porous ceramic plate, to obtain a heat-type smoking agent.

Formulation Example 132

Two and a half parts of one hydrazide compound selected from the group consisting of the present hydrazide compounds (1) to (63), 2.5 part of nitenpyram, and 95 parts of ethylene-methyl methacrylate copolymer (ratio of methyl methacrylate in the copolymer: 10% by, Acryft MD301, manufactured by Sumitomo Chemical Co., Ltd) are melt-kneaded by a closed-type pressure kneader (manufactured by Moriyama Company Ltd).). The resulting kneaded mixture is extruded from an extrusion molding machine through a molding die to obtain rod-shaped molded articles with 15 cm in length and 3 mm in diameter.

Formulation Example 133

Two and a half parts of one hydrazide compound selected from the group consisting of the present hydrazide compounds (1) to (63), 2.5 parts of nitenpyram, and 95 parts of soft vinyl chloride resin are melt-kneaded by a closed-type pressure kneader (manufactured by Moriyama Company Ltd.). The resulting kneaded mixture is extruded from an extrusion molding machine through a molding die to obtain rod-shaped molded articles with 15 cm in length and 3 mm in diameter.

Next, the following test examples are given to show that the pest control composition of the present invention has an excellent controlling effect on pests.

Test Example 1

Acetone solutions were prepared so that the present hydrazide compounds and the present neonicotinoid compounds would have the concentrations (w/v) shown in Table 1. In the bottom of a polyethylene cup with a diameter of 5.5 cm, a filter paper with the same diameter was placed, and then 0.7 mL of the acetone solution for test was added dropwise on the filter paper. The filter paper was allowed to stand at room temperature for 1 hour in order to dry acetone, and then 30 mg of sucrose and 0.7 mL of water were added as a feed. Five male imagoes of German cockroach (*Blattalla germanica*) were released in the polyethylene cup, and the lid was closed. The life and death of the German cockroach was examined after 7 days, and the mortality rate was calculated.

The result is shown in the following Table 1.

[Table 1]

| Present hydrazide compound + Present neonicotinoid compound | Concentration (ppm w/v) | Mortality Rate (%) |
|---|---|---|
| Present Hydrazide compound (11) + clothianidin | 2000 + 2000 | 100 |
| Present hydroxide compound (13) + clothianidin | 2000 + 2000 | 100 |
| Present hydrazide compound (17) + clothianidin | 2000 + 2000 | 100 |
| Present hydrazide compound (30) + clothianidin | 2000 + 2000 | 100 |
| Present hydrazide compound (31) + clothianidin | 2000 + 2000 | 100 |
| Present hydrazide compound (38) + clothianidin | 2000 + 2000 | 100 |

As a result, the pest control composition of the present invention exhibited high pest control effects.

Test Example 2 Insecticidal test on *Haemaphysalis longicornis* by treating filter paper

Acetone solutions were prepared so that the present hydrazide compounds and the present neonicotinoid compounds would have the amount of drug (mg/$m^2$) shown in Table 2 when treated on a filter paper. To one surface of the filter paper (TOYO No. 2; 5 × 10 cm), 1 mL of the acetone solution was uniformly applied. After drying, the filter paper was folded into two, and then the sides were clipped to make a pouch. Into the pouch, test ticks (*Haemaphysalis longicornis,* non-blood sucking young ticks, 10 ticks per group) were released. The open part of the pouch was closed tightly with clips, and the number of dead ticks was examined after 2 days. The mortality rate was calculated by the following calculation formula.

Mortality Rate (%) = 100 × (Number of Dead Tick/Number of Test Tick)

The result is shown in the following Table 2.

[Table 2]

| Present hydrazide compound + Present neonicotinoid compound | Amount of drug (mg/$m^2$) | Mortality Rate (%) |
|---|---|---|
| Present hydrazide compound (11) + clothianidin | 500 + 2500 | 100 |
| Present hydrazide compound (13) + clothianidin | 500 + 2500 | 100 |
| Present hydrazine compound (17) + clothianidin | 500 + 2500 | 100 |
| Present hydrazide compound (30) + clothianidin | 500 + 2500 | 100 |
| Present hydrazide compound (31) + clothianidin | 500 + 2500 | 100 |
| Present hydrazide compound (38) + clothianidin | 500 + 2500 | 100 |

As a result, the pest control composition of the present invention exhibited high pest control effects.

Test Example 3 Insecticidal test on *Haemaphysalis longicornis* by treating glass surface

Acetone solutions were each prepared for the present hydrazide compounds and the present neonicotinoid compounds. To inner walls of a glass screw tube and lid (NO. 5, manufactured by Maruemu Corporation), 0.2 mL of the acetone solution was uniformly applied. After drying, test ticks (*Haemaphysalis longicornis*, non-blood sucking young ticks, 10 ticks per group) are released in the screw tube. The lid is closed tightly, and the number of dead ticks is examined after 2 days. The mortality rate was calculated by the following calculation formula.

Mortality Rate (%) = 100 × (Number of Dead Tick/Number of Test Tick)

The result is shown in the following Table 3.

[Table 3]

| Present hydrazide compound | | Present neonicotinoid compound | | Mortality Rate (%) |
|---|---|---|---|---|
| Compound | Dosage (mg/$m^2$) | Compound | Dosage (mg/$m^2$) | |
| (17) | 0.032 | -- | -- | 3.3 |
| -- | -- | Nitenpyram | 500 | 20 |
| (17) | 0.032 | Nitenpyram | 500 | 100 |

As a result, the pest control composition of the present invention exhibited high pest control effects.

INDUSTRIAL APPLICABILITY

The pest control composition of the present invention has an excellent control effect on pests and is useful.

## Claims

**1.** A pest control composition comprising:

a hydrazide compound shown by formula (1):

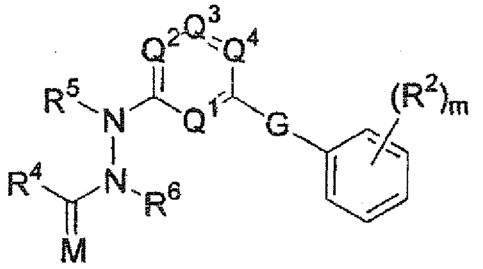

(1)

wherein
G represents a group shown by any of the following formulae G-1 to G-3:

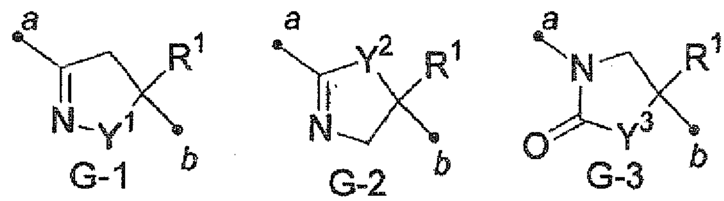

wherein each of a point (a) and point (b) represents a bond, and the point (b) represents a bond with a phenyl ring,
$R^1$ represents a C1 to C4 haloalkyl group,
$Y^1$ represents an oxygen atom, a sulfur atom, or an $NR^7$ group,
$Y^2$ represents an oxygen atom, a sulfur atom, an $NR^7$ group, or a methylene group, and
$Y^3$ represents, an oxygen atom, a sulfur atom, an $NR^7$ group, or a methylene group,
wherein $R^7$ represents a C1 to C6 alkyl group, a C2 to C6 alkenyl group, a C2 to C6 alkynyl group, a C3 to C6 cycloalkyl group, a C4 to C7 cycloalkylalkyl group, a C2 to C6 alkylcarbonyl group, a C2 to C6 alkoxycarbonyl group, a C2 to C6 alkylaminocarbonyl group, a C3 to C9 dialkylaminocarbonyl group, a phenyl group, a cyano group, a formyl group, or a hydrogen atom,
M represents an oxygen atom or a sulfur atom,
$Q^1$, $Q^2$, $Q^3$, and $Q^4$ independently represent a nitrogen atom or a $CR^3$ group,
wherein $R^3$ represents a C1 to C6 alkyl group optionally substituted by a halogen atom or atoms, a C1 to C6 alkoxy group optionally substituted by a halogen atom or atoms, a nitro group, a cyano group, a halogen atom, or a hydrogen atom,
m represents any integer of 0 to 5,
$R^2$ represents a C1 to C6 alkyl group optionally substituted by a halogen atom or atoms, a C1 to C6 alkoxy group optionally substituted by a halogen atom or atoms, a C1 to C6 alkylthio group, a C1 to C6 alkylsulfinyl group, a C1 to C6 alkylsulfonyl group, a nitro group, a cyano group, or a halogen atom, with the proviso that $R^2$ is the same or different when m is any integer of 2 to 5,
$R^5$ and $R^6$ are independently represent a C1 to C12 chain hydrocarbon group optionally substituted by a group or groups selected from Group E1, a benzoyl group optionally substituted by a group or groups selected from Group E2, a C2 to C6 alkylcarbonyl group, a C2 to C6 alkoxycarbonyl group, a C3 to C12 cycloalkyl group, a formyl group, or a hydrogen atom,
$R^4$ represents a C1 to C12 chain hydrocarbon group optionally substituted by a group or groups selected from the Group E1, a C3 to C12 cyclic hydrocarbon group optionally substituted by a group or groups selected from the Group E2, a 5- to 6-membered heterocyclic group optionally substituted by a group or groups selected from the Group E2, an $OR^8$ group, an $N(R^9)R^{10}$ group, or a hydrogen atom,
wherein $R^8$ represents a C1 to C12 chain hydrocarbon group optionally substituted by a group or groups selected from the Group E1, a C3 to C12 cyclic hydrocarbon group optionally substituted by a group or groups selected from the Group E2, or a 5- to 6-membered heterocyclic group optionally substituted by a group or groups selected from the Group E2,
$R^9$ represents a C1 to C12 chain hydrocarbon group optionally substituted by a group or groups selected from

the Group E1 or a C3 to C12 cyclic hydrocarbon group optionally substituted by a group or groups selected from the Group E2, and
$R^{10}$ represents a C1 to C12 chain hydrocarbon group optionally substituted by a group or groups selected from the Group E1, or a hydrogen atom, or
$R^9$ and $R^{10}$ are bonded at their ends to represent a C2 to C9 alkanediyl group,
the Group E1 represents a group consisting of a C3 to C12 cyclic hydrocarbon group optionally substituted by a group or groups selected from the Group E2, a 5- to 6-membered heterocyclic group optionally substituted by a group or groups selected from the Group E2, a phenoxy group optionally substituted by a group or groups selected from the Group E2, a phenylamino group optionally substituted by a group or groups selected from the Group E2, a C2 to C6 alkylcarbonyl group, a C2 to C6 alkoxycarbonyl group, a C1 to C6 alkoxy group, a C1 to C6 alkylamino group, a C2 to C12 dialkylamino group, a nitro group, a cyano group, a formyl group, and a halogen atom,
the Group E2 represents a group consisting of a C1 to C6 alkyl group optionally substituted by a halogen atom or atoms, a C1 to C6 alkoxy group optionally substituted by a halogen atom or atoms, a C2 to C6 alkylcarbonyl group, a C2 to C6 alkoxycarbonyl group, a C1 to C6 alkylamino group, a C2 to C12 dialkylamino group, a nitro group, a cyano group, a formyl group, and a halogen atom, and
a neonicotinoid compound selected from the group consisting of imidacloprid, thiacloprid, acetamiprid, nitenpyram, clothianidin, and thiamethoxam.

**2.** The pest control composition according to claim 1, wherein in the formula (1), G is a group shown by G-1, $Y^1$ is an oxygen atom, $R^1$ is a trifluoromethyl group, each of $Q^1$, $Q^3$, and $Q^4$ is a CH group, $Q^2$ is a $CR^3$ group, $R^3$ is the group defined above, $(R^2)_m$ is a substituent at 3- and 5-positions, m is 2, and $R^2$ is a chlorine atom.

**3.** The pest control composition according to claim 1 or 2, wherein in the formula (1), $R^6$ is a hydrogen atom.

**4.** The pest control composition according to any one of claims 1 to 3, wherein in the formula (1), $R^5$ is a hydrogen atom or a methyl group.

**5.** The pest control composition according to any one of claims 1 to 4, wherein in the formula (1), $R^3$ is a halogen atom.

**6.** The pest control composition according to any one of claims 1 to 5, wherein in the formula (1), $R^3$ is a chlorine atom.

**7.** The pest control composition according to any one of claims 1 to 6, wherein in the formula (1), $R^4$ is a C2 to C6 alkyl group, a C1 to C6 haloalkyl group, a C3 to C6 cycloalkyl group, or a (C1 to C6 alkoxy) C1 to C6 alkyl group.

**8.** The pest control composition according to any one of claims 1 to 7, wherein in the formula (1), $R^4$ is a C2 to C6 alkyl group.

**9.** The pest control composition according to any one of claims 1 to 7, wherein in the formula (1), $R^4$ is a C1 to C6 haloalkyl group.

**10.** The pest control composition according to any one of claims 1 to 7, wherein in the formula (1), $R^4$ is a C3 to C6 cycloalkyl group.

**11.** The pest control composition according to any one of claims 1 to 7, wherein in the formula (1), $R^4$ is a (C1 to C6 alkoxy) C1 to C6 alkyl group.

**12.** The pest control composition according to any one of claims 1 to 11, wherein the neonicotinoid compound is a compound selected from the group consisting of clothianidin, nitenpyram, and thiamethoxam.

**13.** The pest control composition according to any one of claims 1 to 11, wherein the neonicotinoid compound is clothianidin.

**14.** A method for controlling pests comprising applying effective amounts of the hydrazide compound shown by the formula (1) of claim 1 and a neonicotinoid compound selected from the group consisting of imidacloprid, thiacloprid, acetamiprid, nitenpyram, clothianidin, and thiamethoxam to pests or habitats of pests.

**15.** A combination use of the hydrazide compound shown by the formula (1) of claim 1 and a neonicotinoid compound

selected from the group consisting of imidacloprid, thiacloprid, acetamiprid, nitenpyram, clothianidin, and thiamethoxam for controlling pests.

**INTERNATIONAL SEARCH REPORT**

International application No.
PCT/JP2011/067565

A. CLASSIFICATION OF SUBJECT MATTER
*A01N43/80*(2006.01)i, *A01N43/40*(2006.01)i, *A01N47/40*(2006.01)i, *A01N51/00*(2006.01)i, *A01P7/02*(2006.01)i, *A01P7/04*(2006.01)i

According to International Patent Classification (IPC) or to both national classification and IPC

B. FIELDS SEARCHED

Minimum documentation searched (classification system followed by classification symbols)
A01N43/80, A01N43/40, A01N47/40, A01N51/00, A01P7/02, A01P7/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2008-266293 A (Sumitomo Chemical Co., Ltd.), 06 November 2008 (06.11.2008), claims; paragraphs [0101] to [0103], [0108] & WO 2008/126665 A2 & EP 2139874 A2 & US 2010/137372 A1 & AR 065867 A1 | 1-15 |
| P,X | WO 2010/090344 A1 (Sumitomo Chemical Co., Ltd.), 12 August 2010 (12.08.2010), claims; page 84, lines 11 to 23; page 86, lines 13 to 15 & JP 2010-202643 A & AR 075365 A1 | 1-15 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 September, 2011 (05.09.11) | 13 September, 2011 (13.09.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007518737 A
- WO 2007123855 A
- JP 2007091708 A
- WO 2007123853 A
- JP 2008110971 A
- JP 7278094 A
- JP 2209868 A
- JP 1070467 A
- JP 3218354 A
- JP 4273863 A

### Non-patent literature cited in the description

- *Org. Lett.,* 2001, vol. 3, 3803-3805